(19) Europäisches Patentamt
European Patent Office
Office européen des brevets

(11) Numéro de publication : **0 465 323 A1**

(12) # DEMANDE DE BREVET EUROPEEN

(21) Numéro de dépôt : **91401773.6**

(22) Date de dépôt : **28.06.91**

(51) Int. Cl.$^5$ : **C07D 239/30,** C07D 239/34, C07D 239/36, C07D 239/38, C07D 403/10, A61K 31/505

(30) Priorité : **02.07.90 FR 9008346**
**29.11.90 FR 9014963**

(43) Date de publication de la demande :
**08.01.92 Bulletin 92/02**

(84) Etats contractants désignés :
**AT BE CH DE DK ES FR GB GR IT LI LU NL SE**

(71) Demandeur : **LABORATOIRES UPSA**
**1 bis, rue du Docteur Camille Bru**
**F-47000 Agen (FR)**

(72) Inventeur : **Bru-Magniez, Nicole**
**24-26, avenue Raphel**
**F-75016 Paris (FR)**
Inventeur : **Teulon, Jean-Marie**
**13, Avenue Guibert**
**F-78170 LA Celle Saint Coud (FR)**
Inventeur : **Nicolai, Eric**
**52, rue St Quen, Résidence Le Hameau**
**F-14000 Caen (FR)**

(74) Mandataire : **Hubert, Philippe et al**
**Cabinet Beau de Loménie 55, rue d'Amsterdam**
**F-75008 Paris (FR)**

(54) **Dérivés de pyrimidine antagonistes des récepteurs à l'angiotensine II, leurs procédés de préparation et compositions pharmaceutiques les contenant.**

(57) La présente invention concerne les dérivés de formule :

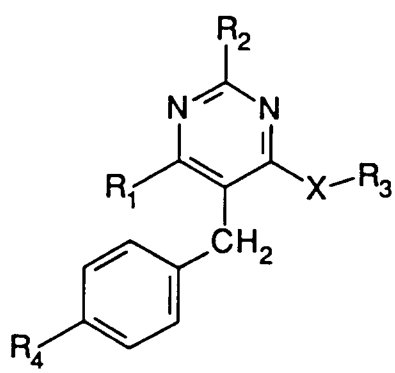

Formule (I)

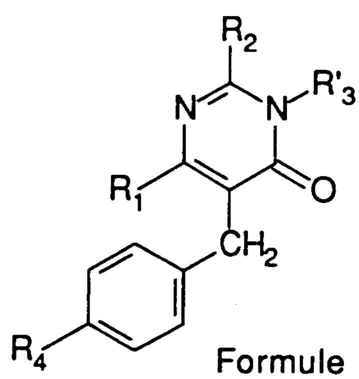

Formule (I')

dans lesquelles :

$R_1$ est un radical alkyle inférieur de 1 à 6 atomes de carbone ou un radical alkylène inférieur de 2 à 6 atomes de carbone.

$R_2$ est un radical alkyle inférieur ou un aromatique ou un groupement fonctionnel ou l'atome d'hydrogène ou un atome d'halogène.

X peut représenter une liaison, un atome d'oxygène, de soufre, un radical NH ou un halogène.

$R_3$ ou $R'_3$ peuvent être un groupement fonctionnel, un atome d'hydrogène, un radical alkyle inférieur, un noyau aromatique ou un hétérocycle.

$R_4$ peut représenter un groupement acide sulfonique-2 benzoyl amino, carboxy-2 dichloro-3,6 benzoyl amino, carboxy-2 phényl, sulfoxy-2 phényl ou (tétrazol-yl-5)-2 phényl.

Ainsi que leurs sels d'addition et leur utilisation en thérapeutique notamment pour le traitement des maladies cardiovasculaires en particulier pour le traitement de l'hypertension et de l'insuffisance cardiaque.

EP 0 465 323 A1

La présente invention concerne en tant que produits nouveaux, les dérivés de pyrimidine de formules générales (I) et (I′) ci-dessous et éventuellement leurs sels d'addition en particulier les sels d'addition pharmaceutiquement acceptables.

Les composés en question présentent un profil pharmacologique très intéressant dans la mesure où ils sont doués de propriétés antagonistes des récepteurs à l'angiotensine II. Ils sont donc particulièrement indiqués pour le traitement des maladies cardiovasculaires, en particulier pour le traitement de l'hypertension et pour le traitement de l'insuffisance cardiaque.

La présente invention concerne également le procédé de préparation des dits produits et leurs applications en thérapeutique.

Ces dérivés de pyrimidine sont caractérisés en ce qu'ils répondent aux formules générales (I) et (I′) :

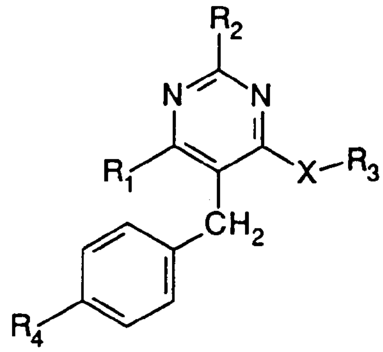

Formule (I)

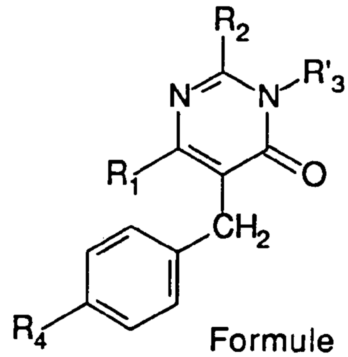

Formule (I')

Dans la formule (I),

$R^1$ est un radical alkyle inférieur de 1 à 6 atomes de carbone ou un radical alkényle inférieur de 2 à 6 atomes de carbone ;

$R^2$ est l'atome d'hydrogène, un atome d'halogène, un radical alkyle inférieur de 1 à 6 atomes de carbone, un radical halogéno alkyle inférieur de 1 à 6 atomes de carbone, un radical cyclo alkyle en $C_3$-$C_7$, un groupement OH, SH ou $NH_2$, un groupement $OR_5$, $SR_5$ ou $NHR_5$, $R_5$ étant un radical alkyle inférieur de 1 à 6 atomes de carbone, un radical halogéno alkyle inférieur de 1 à 6 atomes de carbone ou un radical cycloalkyle en $C_3$-$C_7$, NH $COR_6$, $R_6$ ayant la même signification que $R_5$ mais pouvant être également un noyau aromatique, un méthane biphényle substitué ou non ou un hétérocycle ; $R_2$ peut encore représenter un noyau aromatique ou un hétérocycle ;

X peut représenter une liaison, un atome d'oxygène, de soufre, un radical NH ou un halogène,

$R_3$ sera absent quand X est un halogène ou peut représenter: un atome d'hydrogène, un radical alkyle inférieur de 1 à 6 atomes de carbone, un radical cycloalkyle en $C_3$-$C_7$, ou encore : un groupement $-(CH_2)_n-CN$, un groupement $-(CH_2)_n-COOR_7$, un groupement $-(CH_2)_n-OR_7$, un groupement

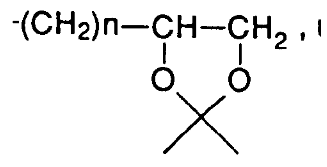

un groupement $^-(CH_2)n-O-COR_7$, un groupement

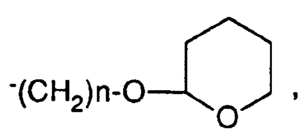

un groupement $^-(CH_2)n-SR_7$, n étant un nombre entier de 0 à 5, $R_7$ étant un atome d'hydrogène ou un radical alkyle inférieur de 1 à 6 atomes de carbone ; $R_3$ peut encore représenter un groupement $-(CH_2)_p-CONR_8R_9$, $-(CH_2)_p-$ $NR_5R_9$, p étant un nombre entier de 0 à 5, $R_8$ et $R_9$ représentant indépendamment un atome d'hydrogène ou un radical alkyle inférieur de 1 à 6 atomes de carbone ou pouvant former ensemble avec l'atome d'azote auquel ils sont rattachés un hétérocycle choisi parmi la pyrrolidine, la pipéridine, la morpholine, la thiomorpholine, un phtalimide ou une pipérazine qui peut être substituée par un alkyle inférieur, un noyau aromatique ou un hétérocycle ; $R_3$ peut encore représenter un groupement $-(CH_2)_q-NH-(CH_2)_r-COOR_{10}$ ou $-(CH_2)_q-NH-CO-NHR_{11}$ ou $-(CH_2)_q-NH-CS-NH-R_{11}$, q et r étant des nombres entiers de 0 à 5, $R_{10}$ et $R_{11}$ représentant indépendamment un radical alkyle inférieur de 1 à 6 atomes de carbone, $R_{11}$ pouvant en outre être un noyau aromatique ou un hétérocycle ou encore un groupement $-(CH_2)_n-COOR_7$, n et $R_7$ étant définis comme ci-dessus ; $R_3$ peut encore représenter le groupement $SO_3H$ ou un de ses esters ou un de ses amides; $R_3$ peut encore représenter un groupement amino acide

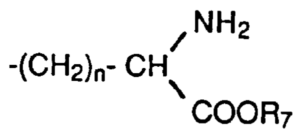

ou un de ses amides

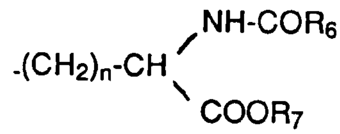 ,

n, $R_6$ et $R_7$ étant définis comme ci-dessus ; $R_3$ peut enfin représenter un radical $-(CH_2)_n-$ noyau aromatique ou $-(CH_2)_n-$ hétérocycle, n étant défini comme ci-dessus ;

$R_4$ peut représenter un groupement nitro, amino, -COO $R_{12}$, $R_{12}$ étant un atome d'hydrogène ou un radical alkyle inférieur de 1 à 6 atomes de carbone ou un benzyle ; $R_4$ peut également représenter les radicaux suivants :

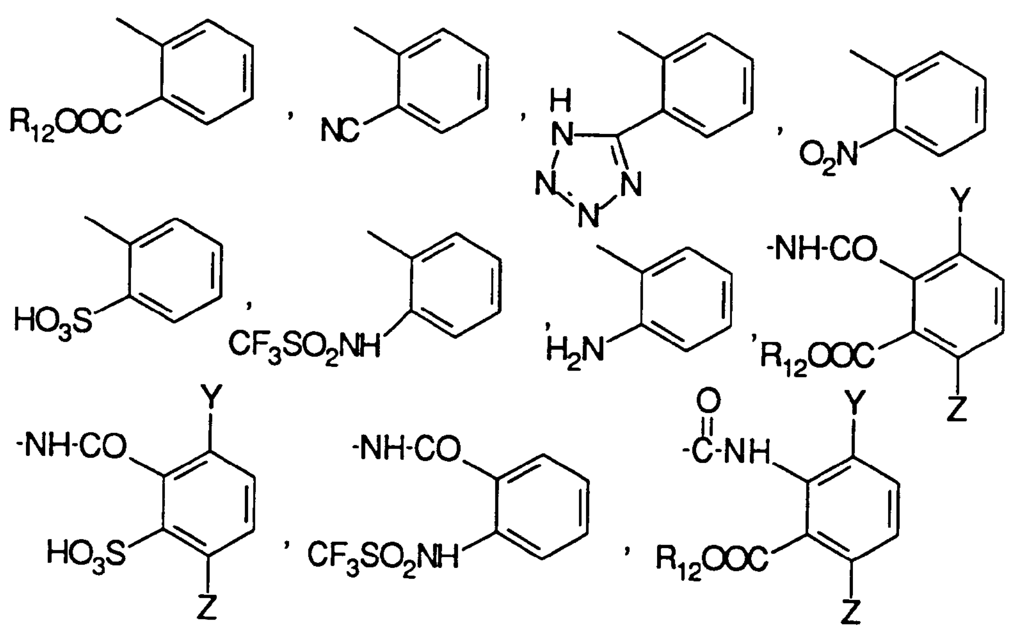
, , , , , , , ,

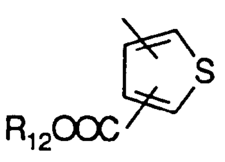 , 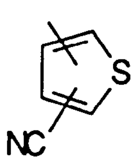 , 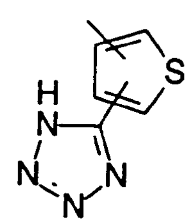

dans lesquels $R_{12}$ a la même signification que ci-dessus et Y et Z peuvent représenter indépendamment un atome d'hydrogène, un radical alkyle inférieur, un atome d'halogène, un radical alkoxy inférieur ou un radical trifluorométhyle ;

dans la formule (I'), $R_1$, $R_2$ et $R_4$ ont la même signification que dans la formule (I), $R'_3$ a la même signification que $R_3$ à la différence que contrairement à ce dernier, il ne pourra représenter un groupement $SO_3H$ ou un de ses esters ou un de ses amides et que de plus dans le cas de $R'_3$ le nombre q ne pourra être inférieur à 2, le nombre p égal à 0 et le nombre n égal à 0 sauf dans le cas d'un groupement $-(CH_2)_n-COOR_7$ ou d'un noyau aromatique ou d'un hétérocycle ;

Les dérivés précités pouvant se présenter sous la forme de sels d'addition en particulier de sels d'addition pharmaceutiquement acceptables.

Dans la description et les revendications, on entend par alkyle inférieur une chaîne hydrocarbonée ayant de 1 à 6 atomes de carbone, linéaire ou ramifiée. Un radical alkyle inférieur est par exemple un radical méthyle, éthyle, propyle, isopropyle, butyle, isobutyle, tertiobutyle, pentyle, isopentyle, hexyle, isohexyle.

On entend par alkényle inférieur, une chaîne hydrocarbonée ayant de 2 à 6 atomes de carbone linéaire ou ramifiée et présentant une insaturation. Un radical alkényle inférieur est par exemple un radical éthène, propène, isopropène, butène, isobutène, pentène, isopentène, hexène, isohexène.

Par radical halogéno alkyle inférieur, on entend un radical alkyle de 1 à 6 atomes de carbone dont 1 à 7 atomes d'hydrogène ont été substitués par 1 à 7 atomes d'halogène. Un radical halogéno alkyle inférieur est par exemple un radical trifluorométhyl, un radical trifluoro-2,2,2 éthyl, un radical pentafluoro éthyl, un radical difluoro-2,2 trifluoro-3,3,3 propyl, un radical heptafluoro propyl.

Par radical cycloalkyle en $C_3$-$C_7$, on entend un radical hydrocarboné cyclique saturé, il s'agit de préférence d'un radical cyclopropane, cyclobutane, cyclohexane ou cycloheptane.

On entend par alkoxy inférieur, un groupement 0-alkyle inférieur, alkyle inférieur étant défini comme ci-dessus.

On entend par thioalkyle inférieur un groupement S-alkyle inférieur, alkyle inférieur étant défini comme ci-dessus.

On entend par halogène un atome de chlore, de brome, d'iode ou de fluor.

Par noyau aromatique on entend un noyau phényle ou naphtyle, le phényle ou le naphtyle pouvant être éventuellement substitués par un groupement alkyle inférieur, un halogène, un groupement halogéno alkyle inférieur, un alkoxy inférieur, un thioalkyle inférieur ou un nitro.

Par hétérocycle on entend un cycle aromatique de 5 à 7 atomes comportant au moins un hétéroatome tel que azote, oxygène ou soufre, l'hétérocycle pouvant être éventuellement substitué par un radical alkyle inférieur, un halogène, un groupement halogéno alkyle inférieur, un alkoxy inférieur, un thio alkyle inférieur, un nitro ou un noyau aromatique.

Un hétérocycle est par exemple la pyridine, le thiophène, le furane, la pyrimidine, la pipérazine, la pyridazine, une diazépine, un thiazole, un imidazole, un oxazole, une thiazépine une oxazépine, un triazole, un tétrazole.

Selon une variante de réalisation, $R_1$ est un groupement n-propyl ;
selon une autre variante de réalisation $R_1$ est un groupement n-butyl ;
selon une variante de réalisation, $R_2$ est un groupement méthyl ;
selon une autre variante de réalisation, $R_2$ est l'atome d'hydrogène ;
selon une autre variante de réalisation, $R_2$ est un groupement méthyl thio ;
selon une variante de réalisation, X est l'atome d'oxygène ;
selon une autre variante de réalisation, X est l'atome de soufre ;
selon une variante de réalisation, $R_3$ est un groupement éthoxy carbonyl méthyl ;
selon une autre variante de réalisation, $R'_3$ est un groupement éthoxy carbonyl méthyl ;
selon une autre variante de réalisation, $R_3$ est un groupement hydroxy-2 éthyl ;
selon une autre variante de réalisation, $R'_3$ est un groupement hydroxy-2 éthyl ;
selon une autre variante de réalisation, $R_3$ est un radical méthyl ;
selon une autre variante de réalisation, $R_3$ ou $R'_3$ est l'atome d'hydrogène ;
selon une variante de réalisation, $R_4$ est un groupement sulfoxy-2 benzoyl amino ;
selon une autre variante de réalisation, $R_4$ est un groupement carboxy-2 phényl ;
selon une autre variante de réalisation, $R_4$ est un groupement (tétrazol-yl-5)-2 phényl.

Les composés de l'invention particulièrement préférés sont ceux qui sont choisis parmi les produits de formule :

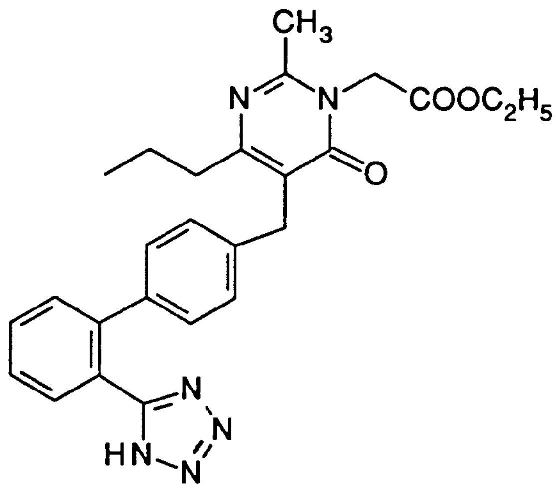

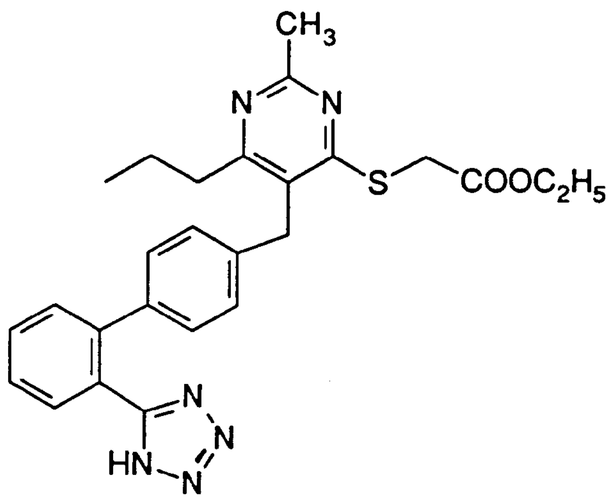

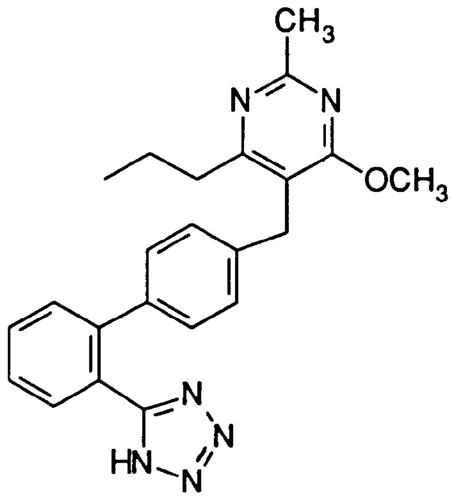

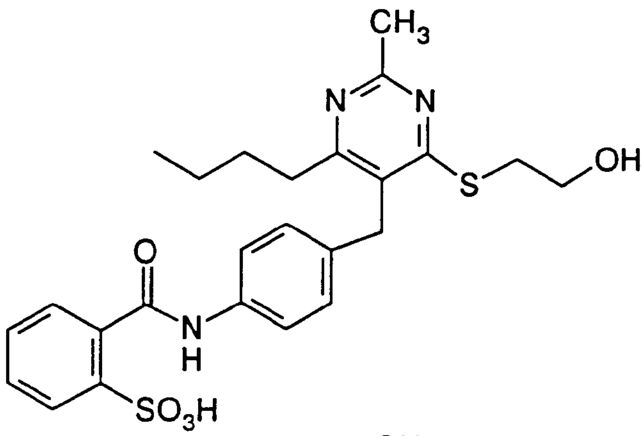

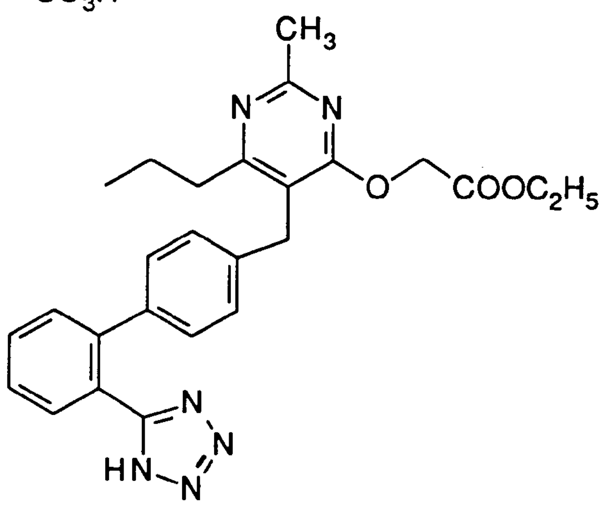

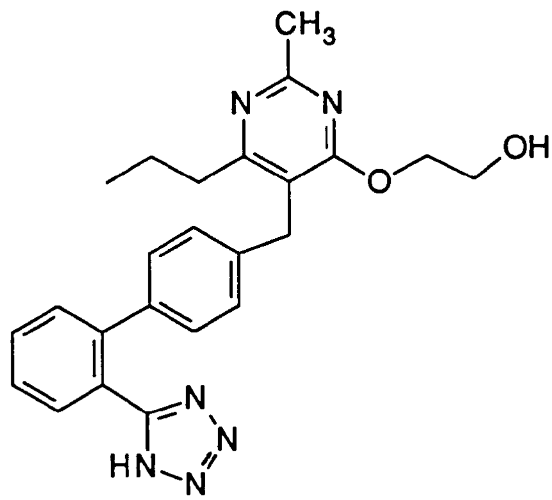

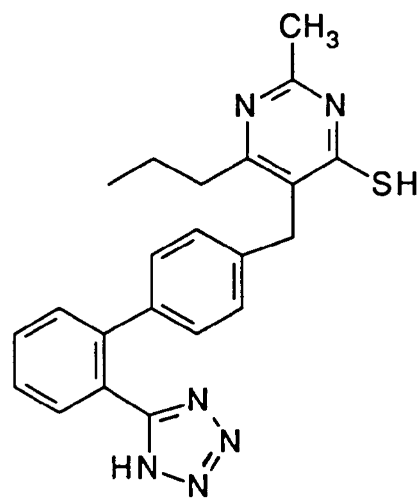

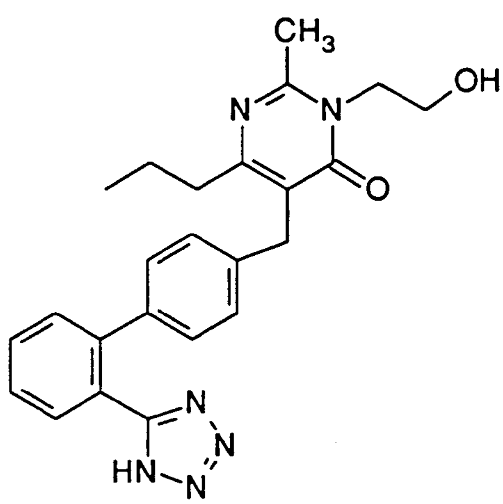

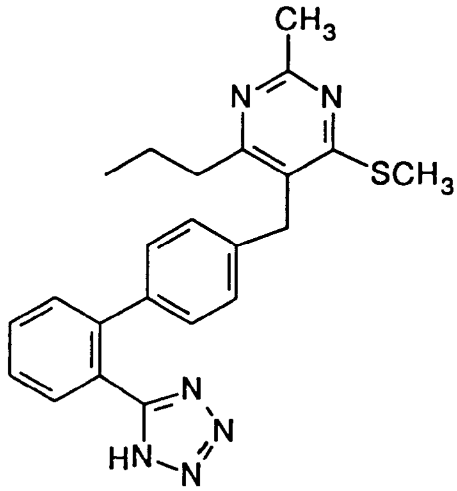

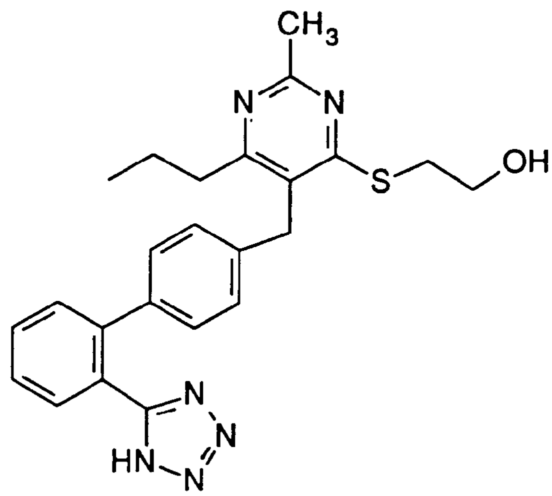

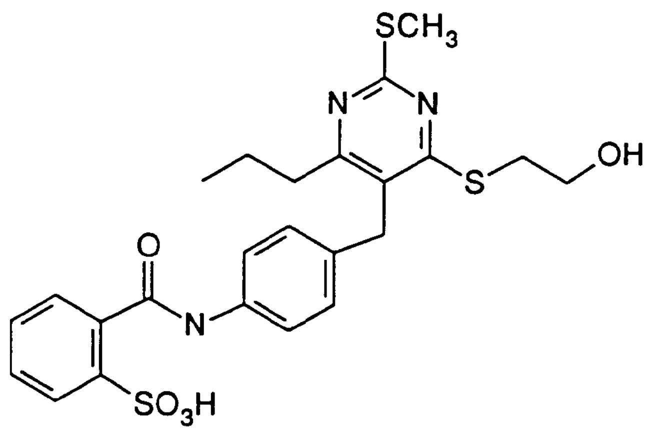

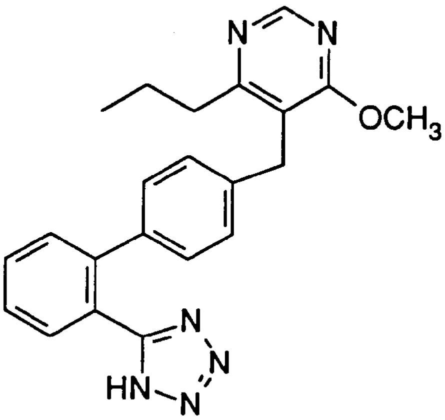

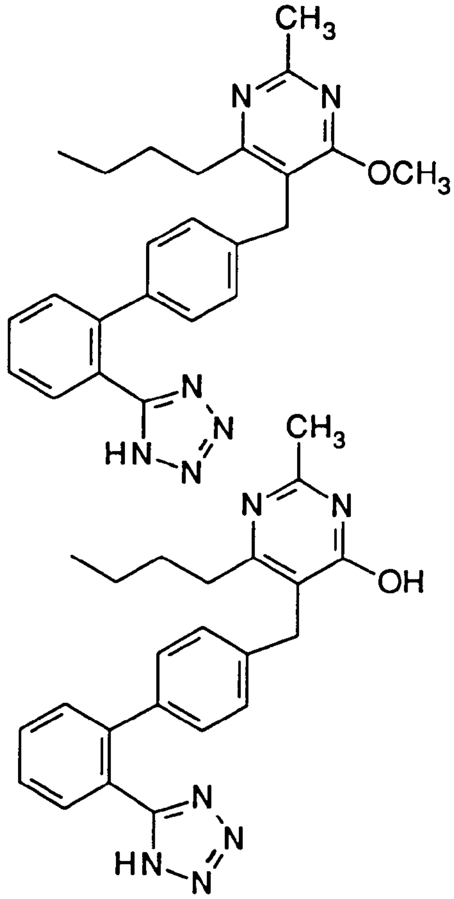

Selon l'invention, les composés de formule (I) ou (I') pourront être synthétisés selon la suite de réactions suivantes :

On préparera :

– les oxo-3 alkanoates d'alkyle de formule (II) :

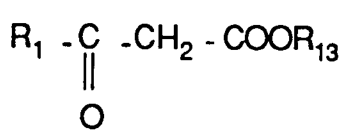

Formule (II)

dans laquelle $R_1$ est défini comme ci-dessus et $R_{13}$ représente un radical alkyle inférieur, de préférence méthyle ou éthyle

– les oxo-3 nitriles de formule (III) :

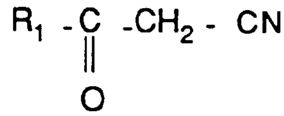

Formule (III)

dans laquelle $R_1$ est défini comme ci-dessus

– les dicétones -1,3 de formule (IV) :

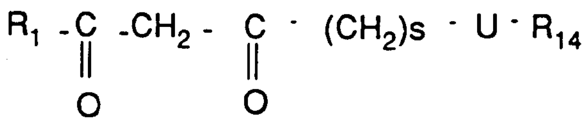

Formule (IV)

dans laquelle $R_1$ est défini comme ci-dessus, s est un nombre entier de 1 à 5, U peut être un atome d'oxygène, de soufre ou un méthylène, $R_{14}$ est un atome d'hydrogène ou un alkyle inférieur de préférence méthyle ou éthyle, par des méthodes connues en soi, telles que par exemple la réaction de Claisen ou la méthode à l'acide de Meldrum, certaines de ces méthodes peuvent être trouvées dans la littérature aux références :

– OIKAWA.Y; SUGANO.K; YONEMITSU.O; J.Org.Chem., 1978, 43(10), 2087-88.
– WIERENGA.W; SKULNICK.H.I. ; J.Org.Chem., 1979, 44, 310.
– HOUGHTON.R; LAPHAM.D; SYNTHESIS, 1982, 6, 451-2.
– BRAM.G ; VILKAS.M; Bull.Soc.Chim.France, 1964(5), 945-51.
– BALYAKINA M.V.; ZHDANOVICH E.S.; PREOBRAZHENSKII N.A.; Tr. Vses. Nauchn. Issled. Vitam in. Inst., 1961,7 8-16.
– RENARD M. ; MAQUINAY A. ; Bull. Soc. Chim. Belg., 1946, 55, 98-105.
– BRUCE F.W. ; COOVER H.W. ; J. Am. Chem. Soc., 1944, 66, 2092-94.
– EBY C.J. et HAUSER C.R.; J. Am. Chem. Soc. 1957, 79, 723-5

Par benzylation des composés de formule (II), (III), (IV) par des composés de formule (V)

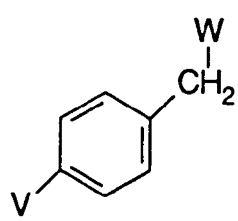

Formule (V)

en présence d'une base telle qu'un carbonate de sodium ou de potassium dans l'acétone, un alcoolate de sodium ou de potassium dans un alcool, un hydrure de sodium ou de lithium dans des solvants comme le tétrahydrofurane, le dioxane ou le diméthyl formamide par exemple, à une température comprise entre 50 et 100°C ou encore, en présence d'un équivalent de chlorure ou de bromure de lithium et de deux équivalents de diisopropyl éthyl amine au reflux du tétrahydrofurane selon la référence : SUNG-EUN YOO; KYU YANG YI; Bull. Korean. Chem. Soc. 1989, 10 (1), 112

On obtiendra les composés de formule :

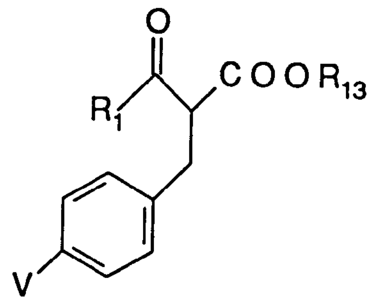

Formule (VI)

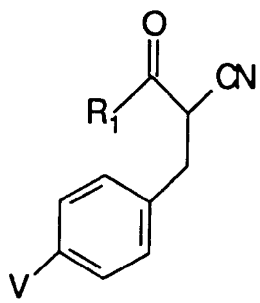

Formule (VII)

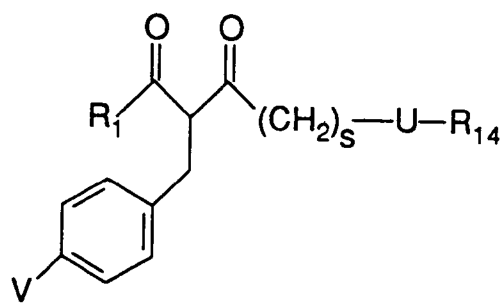

Formule (VIII)

Ces composés de formule (VI), (VII) et (VIII) peuvent être également obtenus par condensation d'un aldéhyde de formule (IX)

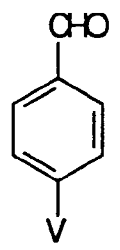

Formule (IX)

sur les composés de formule (II), (III) et (IV) suivie d'une hydrogénation en présence d'un catalyseur comme le Nickel de Raney, le palladium sur charbon ou l'oxyde de platine dans un solvant comme un alcool ou le tétrahydrofurane sous pression ou à pression ordinaire lorsque les substitutions présentes le permettent.

D'une manière plus générale, on trouvera des méthodes de préparation des composés de formule (VI), (VII) et (VIII) dans les références suivantes :

- DURGESHWARI.P ; CHAUDHURY.N.D; J.Ind.Chem.Soc, 1962,39, 735-6
- HEINZ.P ; KREGLEWSKI.A; J.Prakt.Chem, 1963, 21 (3-4), 186-197
- ZAUGG.H.E ; DUNNIGAN.D.A; MICHAELS.R.J; SWETT.LR; J.Org.Chem., 1961, 26, 644-51 .
- KAGAN.H.B ; HENG SUEN.Y; Bull.Soc.Chim.France, 1966 (6), 1819-22.
- RATHKE.M.W. ; DEITCH.J ; Tetrahedron Lett, 1971 (31 ), 2953-6.
- BORRIES KUBEL; Liebigs.Ann.Chem, 1980, 1392-1401,.
- MARQUET. J; MORENO-MANAS. M; Chem. Lett, 1981,2, 173-6.
- IOFFE. T; POPOV.E.M; VATSURO.K.V. ; TULIKOVA.E.K.; KABACHNIK.M.I. ; Tetrahedron, 1962, 18, 923-940.
- SHEPHERD.T.M; Chem. Ind. (London), 1970, 17, 567.

Dans la formule (V), W représente un atome d'halogène préférentiellement le chlore ou le brome.

Dans la même formule :

V peut être un groupement nitro, le dérivé de formule (V) est alors commercial.

V peut être un groupement $COOR_{15}$, $R_{15}$ étant un radical alkyle inférieur ou benzyle, le dérivé de formule (V) sera alors préparé par chloration ou bromation à l'aide du N-chloro succinimide ou du N-bromosuccinimide dans un solvant comme le tétrachlorure de carbone ou le dibromoéthane d'un ester de l'acide p-méthyl benzoïque qui est commercial selon la référence :

- JULIA.M; CHASTRETTE.F; Bull.Soc.Chim.France, 1962(2) , 2247.

V peut être un groupement

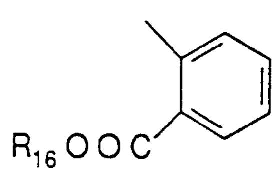

$R_{16}$ étant un radical alkyle inférieur ou benzyle ; les composés de formule (V) sont alors préparés par réaction d'un magnésien du p-bromo toluène sur un composé de formule :

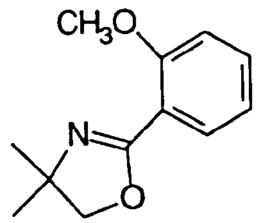

pour obtenir un composé de formule :

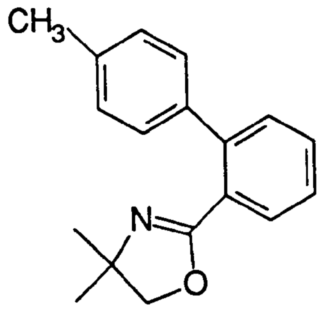

qui est ensuite hydrolysé pour conduire au composé de formule :

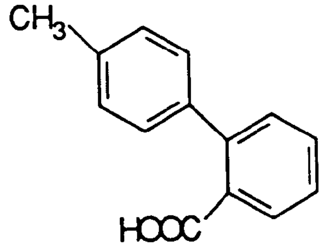

On trouvera des modes opératoires pour les trois étapes décrites ci-dessus dans la référence :
- MEYERS.A.I. ; MIHELICH E.D., J.Am.Chem.Soc., 1975, 97, 7383.

L'acide est ensuite estérifié par un alcool de formule $R_{16}OH$, $R_{16}$ étant défini comme ci-dessus.
Ces dérivés sont alors bromés ou chlorés par exemple par le N-bromo succinimide, le N-chloro succinimide ou le brome dans un solvant comme le tétrachlorure de carbone ou le dibromoéthane ou le dichloroéthane pour conduire aux composés de formule (V) où V est le groupement

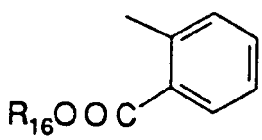

V peut être le groupement

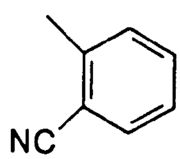

dans ce cas le composé :

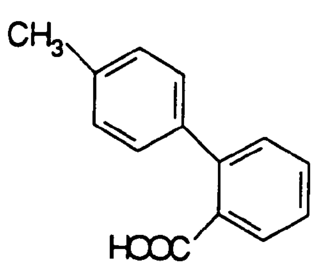

précédemment préparé sera transformé en amide primaire par action du chlorure d'acide, obtenu avec le chlorure de thionyle ou l'oxychlorure de phosphore, sur l'ammoniaque et cet amide sera transformé en nitrile par

action de l'oxychlorure de phosphore dans le diméthylformamide ou du chlorure de thionyle. Le nitrile obtenu :

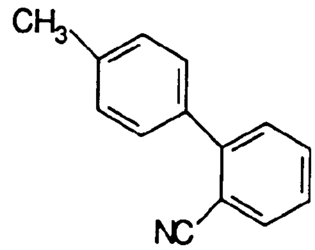

sera ensuite bromé ou chloré dans les mêmes conditions que l'ester précédent pour conduire aux composés de formule (V) où V est le groupement

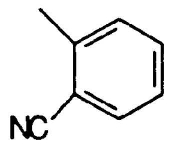

V peut être le groupement

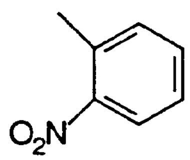

dans ce cas, le composé

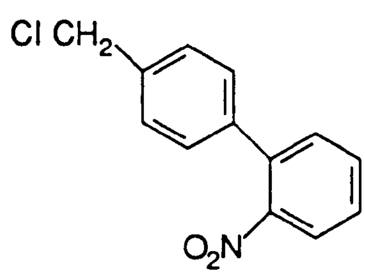

sera préparé par chlorométhylation du 2-nitro biphényl commercial selon les réf. :

CA : 70 (25) : 114837 d

CA : 69 (2) : 3704

V peut être un groupement

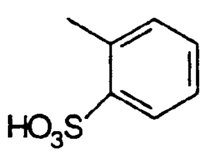

,

les composés de formule (V) sont alors préparés de la façon suivante : par une réaction de Wurtz entre le para iodo toluène et l'orthonitro iodo benzène en présence de cuivre et chauffage entre 180 et 210°C.

On obtiendra :

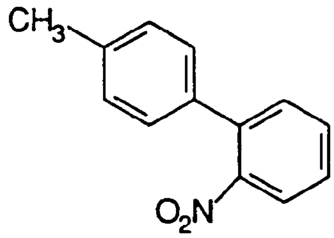

après hydrogénation du groupement nitro en amine, diazotation avec Na $NO_2$ dans l'acide chlorhydrique concentré, suivie d'un traitement au $SO_2$ en présence de Cu $Cl_2$ dans l'acide acétique on obtiendra le composé

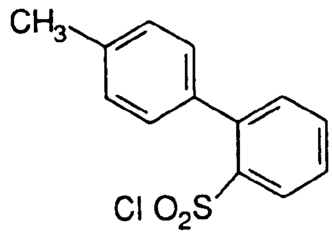

qui sera traité au méthanol en présence de pyridine pour conduire à l'ester :

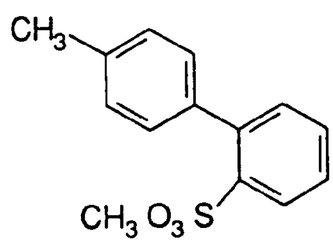

Ce dérivé est alors bromé ou chloré par exemple par le N-bromo succinimide ou le N-chloro succinimide dans un solvant comme le tétrachlorure de carbone ou le dibromo éthane pour permettre d'accéder aux composés de formule (V) où V est le groupement

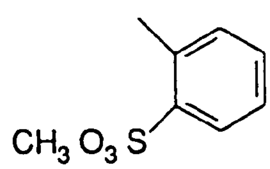

et par hydrolyse aux composés de formule (V) où V est le groupement

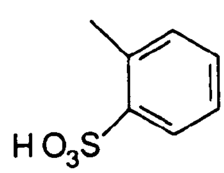

La bromation ou la chloration pourra être également effectuée sur le composé

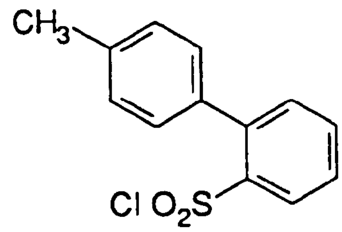

le passage à l'acide sulfonique se faisant alors par hydrolyse de la fonction chlorure de l'acide sulfonique.

V peut-être un groupement

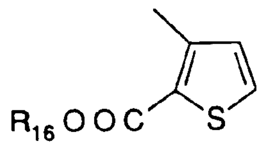

, I

$R_{16}$ étant un radical alkyl inférieur ou benzyle, les composés de formule (IV) correspondants sont obtenus de la façon suivante :

A partir du composé :

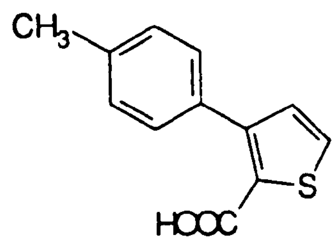

dont on peut trouver la préparation dans la référence :

-FISSELMANN. H; HABITCH.H; Ger. Offen.: 1,092,929 (1960); CA: 57:

5894 g on obtiendra les composés de formule :

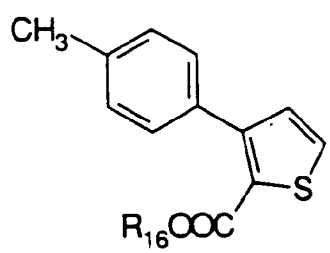

par estérification à l'aide d'un alcool de formule $R_{16}OH$, $R_{16}$ étant défini comme ci-dessus par des méthodes classiques connues de l'homme de l'art.

Ces composés sont ensuite traités par le N-chloro succinimide ou le N-bromo succinimide dans un solvant comme le trétrachlorure de carbone ou le dibromoéthane par exemple, pour donner les composés de formule (IV) dans lesquels V représente le groupement

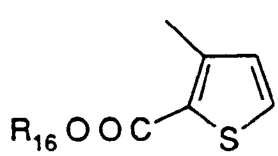

,

$R_{16}$ étant défini comme ci-dessus

- V peut-être le groupement

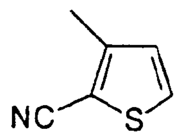

dans ce cas les composés de formule (IV) correspondants seront préparés de la façon suivante :

à partir du composé acide (p-méthyl phényl)-3 thiophène-2 carboxylique dont la préparation est donnée ci-dessus, par traitement avec le chlorure de thionyle puis l'ammoniac on obtient le composé amide qui est ensuite déshydraté par le chlorure de thionyle ou l'oxy chlorure de phosphore sans solvant ou dans le diméthyl formamide pour conduire au composé nitrile :

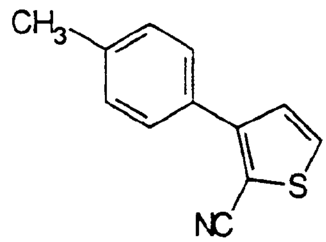

Ce composé nitrile est ensuite halogéné par le N-chloro succinimide ou le N-bromo succinimide dans un solvant comme le tétrachlorure de carbone ou le dibromoéthane pour donner les composés de formule (IV) dans lesquels V représente le groupement

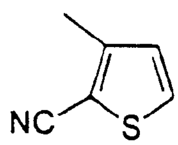

V peut-être le groupement

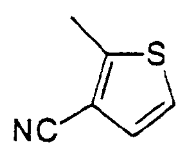

dans ce cas les composés de formule (IV) correspondants sont synthétisés de la façon suivante :

à partir de la chloro-4 méthyl-4'butyrophénone de formule :

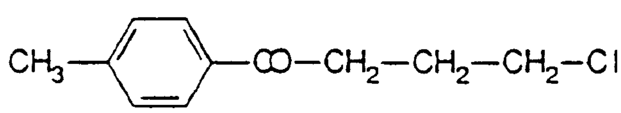

dont la préparation peut être trouvée dans le brevet BE : 577,977 du 15 mai 1959, CA : 54, 4629 c,

par traitement par l'oxychlorure de phosphore et le diméthyl formamide selon les conditions décrites dans la référence :

- VOLODINA. M.A; TENENT'EV A. P.; KUDRYASHOVA. V.A. ;

KABOSHINA. L.N ; Khim. Geterosikl. Soedim ; 1967, 5-8;

on obtiendra le composé de formule :

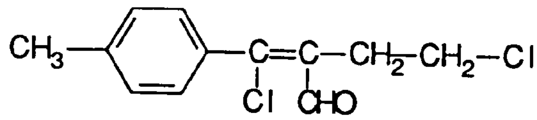

Ce composé est ensuite traité par le sulfure de sodium dans un solvant comme le tétrahydrofurane au reflux pour donner le dérivé

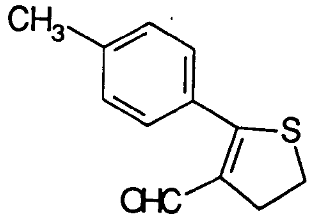

qui est ensuite transformé en deux étapes en dérivé nitrile par déshydratation de l'oxime formée à partir de l'aldéhyde et de l'hydroxylamine. Cette déshydratation pourra être effectuée par exemple à l'aide de l'anhydride acétique pour donner le composé nitrile :

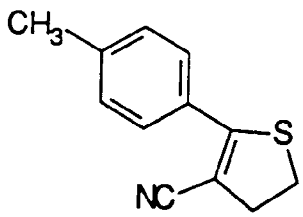

qui pourra être ensuite aromatisé par traitement avec du brome dans le tétrachlorure de carbone puis avec le tertiobutylate de potassium dans le tétrahydrofurane pour donner le composé :

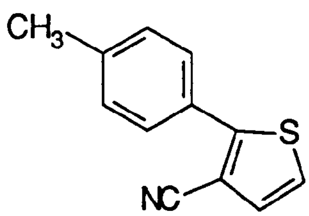

Ce composé peut être ensuite chloré ou bromé par des agents d'halogénation tels que le N-chloro succinimide ou le N-bromo succinimide dans un solvant comme le tétrachlorure de carbone ou le dibromoéthane pour donner les composés de formule (IV) dans lesquels V représente le groupement

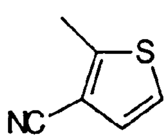

V peut être le groupement

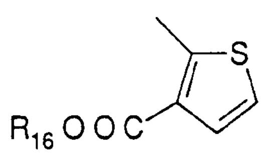

,

$R_{16}$ étant défini comme ci-dessus, ils pourront être préparés à partir du composé de formule

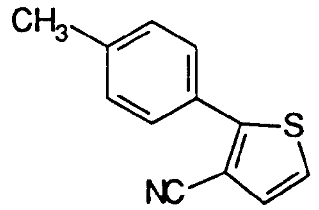

par hydrolyse classique de la fonction nitrile puis estérication de l'acide obtenu ou passage directement de la fonction nitrile à la fonction ester selon les méthodes connues de l'homme de l'art, suivie d'une chloration ou d'une bromation de l'ester par le N-chloro succinimide ou le N-bromo succinimide dans le tétrachlorure de carbone ou le dibromo éthane par exemple.

Dans les formules (VI), (VII) et (VIII), $R_1$, $R_{13}$, s, U et $R_{14}$ sont définis comme précédemment et V a la même définition que dans la formule (V).

Dans la formule (IX), V a la même définition que dans la formule (V) mais cette méthode de condensation ne sera utilisée que lorsque V possède une fonction que respecte l'hydrogénation. Dans le cas contraire ces composés benzylidènes, obtenus par condensation des aldéhydes, pourront être transformés sans hydrogénation en pyrimidine de formule (XI) en les condensant sur un aldéhyde de formule $R_2$-CHO dans le cas où $R_2$ est un alkyle inférieur de 1 à 6 atomes de carbone ou un noyau aromatique en présence d'ammoniaque selon la méthode décrite dans la littérature à la référence :

KROHNKE, SCHMIDT et ZECHER, Chem. Ber, 1964, 97, 1163.

Par action, d'une urée, d'une thiourée, d'une amidine, d'une guanidine, c'est à dire par action d'un composé de formule (X) :

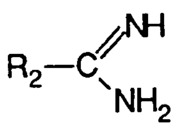

Formule (X)

dans laquelle $R_2$ est défini comme ci-dessus, sur les composés de formule (VI), (VII) ou (VIII) on obtiendra par condensation dans un alcool en présence d'un alcoolate de sodium ou de potassium à une température pouvant aller de l'ambiante à l'ébullition du solvant les composés de formule (XI) :

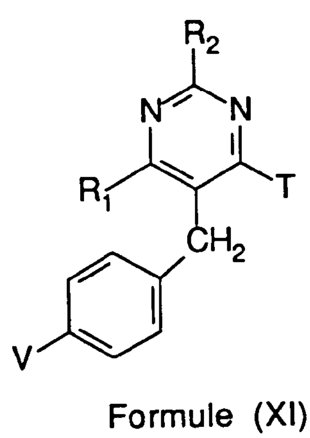

Formule (XI)

dans laquelle $R_1$, $R_2$ et V sont définis comme ci-desus, T pouvant être :

OH quand la condensation est effectuée avec les composés de formule (VI)

$NH_2$ à partir des composés de formule (VII)

$(CH_2)$s-U-$R_{14}$ à partir des composés de formule (VIII), s, U et $R_{14}$ étant définis comme ci-dessus.

On pourra trouver cette réaction de condensation dans la littérature aux références :
AROYAN, A.A; KRAMER, M.S; Arm. Khim. Zk. 1969, 22 (9), 835-41.
KRAMER, M.S. ; AROYAN, A.A; Arm. Khim. Zk. 1970, 23 (1), 69-73.

Les composés de formule (XI) dans lesquels $R_2$ est un atome d'hydrogène, un radical alkyle inférieur ou un phényl substitué ou non, et T est ($CH_2$) s U $R_{14}$, dans lesquels s, U et $R_{14}$ sont définis comme ci-dessus peuvent être préparés également de la façon suivante :

Par réaction d'un aldéhyde de formule (IX) sur un dérivé de formule (IV) on obtient les dérivés de formule (IV') suivants :

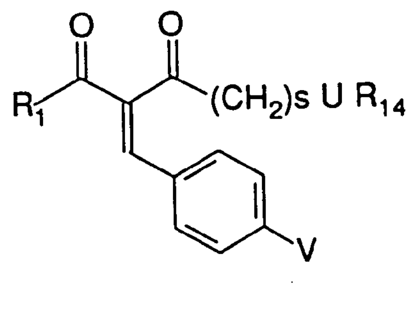

Formule (IV')

dans laquelle $R_1$, s, U, $R_{14}$ et V sont définis comme ci-dessus.

La réaction de ces composés de formule (IV') avec des aldéhydes de formule $R_2CHO$, $R_2$ étant défini comme ci-dessus, en présence d'acétate d'ammonium et d'acide acétique, selon la référence :
KRÖHNKE F. ; SCHMIDT E. ; ZECHER W. ; Chem. Ber., 1964, 97, 1163 - 1178, permet d'obtenir les dérivés de formule (XI) où T représente -$(CH_2)$s-U-$R_{14}$ dans laquelle s, U, $R_{14}$ sont définis comme ci-dessus

Les composés de formule (XII) et (XII') :

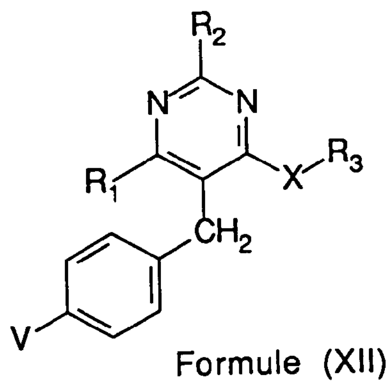

Formule (XII)

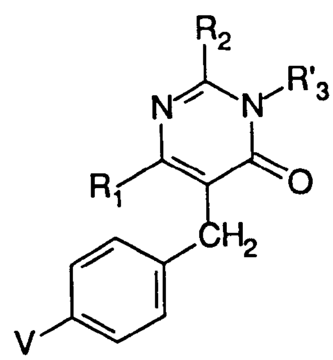

Formule (XII')

dans lesquelles $R_1$, $R_2$, $R_3$, X et V pour la formule (XII) et $R_1$, $R_2$, $R'_3$, V pour la formule (XII') sont définis comme précédemment pourront être obtenus à partir des composés de formule (XI) de la façon suivante :

Le dérivé de formule (XI) dans lequel T est un groupement OH pourra être métallé. Selon les conditions de métallation, on orientera la substitution sur l'atome d'oxygène pour conduire aux composés de formule (XII) ou sur l'atome d'azote pour conduire aux composés de formule (XII'). En particulier, l'utilisation d'un carbonate de potassium ou de sodium ou d'une base comme la soude ou la potasse en milieu eau, alcool, acétonitrile, toluène, ou dans une cétone comme l'acétone ou la méthyl éthyl cétone en présence ou non d'un sel d'ammonium quaternaire, privilégiera la O-substitution pour conduire préférentiellement aux composés de formule (XII) alors que l'utilisation d'un agent métallant comme l'hydrure de sodium ou de lithium dans le diméthylformamide ou le tétrahydrofurane par exemple, conduira en priorité aux dérivés N-substitués de formule (XII').

Par réaction avec des dérivés halogénés de formule :

A-$(CH_2)_n$-$CH_3$

A-$(CH_2)_n$-aromatique

$A-(CH_2)_n$-hétérocycle
$A-(CH_2)_n-CN$
$A-(CH_2)_n-COOR_7$
$A-(CH_2)_n-O-R_7$
$A-(CH_2)_n-S\ R_7$
$A-(CH_2)_p-\ CONR_8R_9$
$A-(CH_2)_p-\ NR_8R_9$

dans lesquelles A est un atome d'halogène plus particulièrement le chlore ou le brome et n, p, $R_7$, $R_8$ et $R_9$ ont la même signification que précédemment, on obtiendra les dérivés de formule (XII) où X $R_3$ représente :

$-O-(CH_2)_n-CH_3$
$-O-(CH_2)_n$-aromatique
$-O-(CH_2)_n$-hétérocycle
$-O-(CH_2)_n-CN$
$-O-(CH_2)_n-COOR_7$
$-O-(CH_2)_n-OR_7$
$-O-(CH_2)_n-SR_7$
$-O-(CH_2)_p-CONR_8R_9$
$-O-(CH_2)_p-NR_8R_9$

ou on obtiendra les composés de formule (XII') où $R'_3$ représente :

$-(CH_2)_n-CH_3$
$-(CH_2)_n$-aromatique
$-(CH_2)_n$-hétérocycle
$-(CH_2)_n-CN$
$-(CH_2)_n-COOR_7$
$-(CH_2)_n-OR_7$
$-(CH_2)_n-SR_7$
$-(CH_2)_p-CONR_8R_9$
$-(CH_2)_p-NR_8R_9$

Par chauffage dans du $POCl_3$ des dérivés de formule (XI) où T est OH, on obtiendra les dérivés de formule (XII) où $XR_3$ est le chlore. Par réaction sur ce dérivé chloré, selon des méthodes connues en soi et que l'on peut trouver dans les références suivantes :

KRAMER, M.S. ; AROYAN, A.A. ; Arm. Khim. Zk. 197O, 23(1 ), 69-73.
AROYAN, A.A.; KRAMER, M.S.; GARIBDZHANYAN, B.T.;
Arm. Khim. Zk. 1969, 22(7), 617-22.
SUNLEY R.L.; SNOWLING, G.D.; Ger. Offen 2, 533, 710

de composés de formule :

$HBR_7$
$HB-(CH_2)_n-CN$
$HB-(CH_2)_n-COOR_7$
$HB-(CH_2)_n-OR_7$
$HB-(CH_2)_n-OCOR_7$

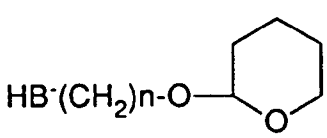

$HB-(CH_2)_n-SR_7$
$HB-(CH_2)_p-CONR_8R_9$
$HB-(CH_2)_p-NR_8R_9$
$HB-(CH_2)_q-NH-(CH_2)_r-COOR_{10}$
$HB-(CH_2)_q-NH-CO-NHR_{11}$
$NH_2-NH-COOR_7$
$NH_2-NR_8R_9$
$NH-R_8R_9$

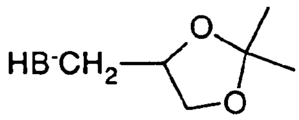

préalablement métallés si nécessaire, et dans lesquelles n, p, q, r, $R_7$,$R_8$, $R_9$, $R_{10}$ et $R_{11}$ ont la même signification que ci-dessus et B représente un atome d'oxygène, de soufre ou un groupement NH, on obtiendra les composés de formule (XII) où $XR_3$ représente :

-BR7
$-B-(CH_2)_n-CN$
$-B-(CH_2)_n-COOR_7$
$-B-(CH_2)_n-OR_7$

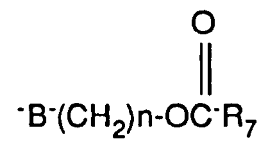

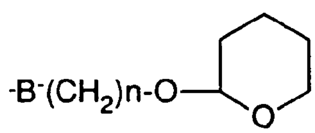

$-B-(CH_2)_n-SR_7$
$-B-(CH_2)_p-CONR_8R_9$
$-B-(CH_2)_p-NR_8R_9$
$-B-(CH_2)_q-NH-(CH_2)_r-COOR_{10}$
$-B-(CH_2)_q-NH-CO-NHR_{11}$
$-NH-NH-COOR_7$
$-NH-NR_8R_9$
$-N-R_8R_9$

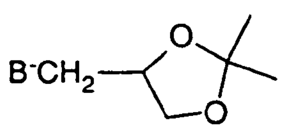

Par traitement des composés de formule (XII) où $XR_3$ est le chlore par la thiourée, puis par hydrolyse basique de l'intermédiaire obtenu, on synthétisera les composés de formule (XII) où $XR_3$ est un groupement SH. Par alkylation de ces composés mercapto avec des halogénures d'alkyle de formule $Y'R_7$ dans laquelle Y'est un halogène, préférentiellement brome ou iode et $R_7$ a la même signification que précédemment, on obtiendra des dérivés de formule (XII) dans laquelle $XR_3$ est un groupement $SR_7$.
Cette séquence réactionnelle est décrite dans la publication :

AROYAN, A.A.; KRAMER, M.S. ; Arm. Khim. Zk. 1973, 26 (5), 402-405.

Par une réaction de Sandmeyer, diazotation par $NaNO_2$ puis traitement par le sel de cuivre correspondant selon les références :

H.H. HODGSON Chem. Revs. 1947, 40, 251

W.A. COWDREY et D.S. DAVIES, Quart. Revs (London) 1952, 6, 358 sur les dérivés de formule (XI) où T est $NH_2$, on pourra obtenir les dérivés de formule (XII) où $XR_3$ représente l'atome de Iode, Brome, Chlore ou Fluor ou encore le groupement CN qui par hydrolyse pourra conduire à un groupement COOH qui pourra être estérifié en $COOR_7$ dans lequel $R_7$ a la même signification que ci-dessus ou encore transformé via le chlorure d'acide, en amide $CONR_8R_9$ où $R_8$ et $R_9$ ont la même signification que ci-dessus. L'hydrogénation du groupement CN pourra conduire aux dérivés où $XR_3$ représente $CH_2$ $NH_2$ qui traités par un isocyanate ou un isothio-

cyanate permettraient d'obtenir les composés où $XR_3$ est $-CH_2$ -NH CONH $R_{11}$ ou $-CH_2$-NH-CS-$NHR_{11}$ où $R_{11}$ a la même signification que ci-dessus.

Les dérivés de formule (XI) où T représente $-(CH_2)_s$-O-$CH_3$ ou $-(CH_2)_s$-$OC_2H_5$ pourront être transformés en dérivé $-(CH_2)_s$-Br par chauffage dans l'acide bromhydrique. Ces composés bromés pourront être transformés par action du cyanure de sodium ou de potassium dans un solvant comme un alcool ou le diméthyl sulfoxyde à une température de 20 à 100°C en des dérivés de formule (XII) où $XR_3$ représente $-(CH_2)_s$-CN, comme précédemment ces composés pourront être transformés par hydrolyse puis par estérification en composés de formule (XII) où $XR_3$ représente $-(CH_2)_s$-$COOR_7$. L'hydrogénation catalytique de ces nitriles pourra également conduire aux composés où $XR_3$ représente $-(CH_2)_n$-$CH_2$-$NH_2$ qui par action d'isocyanate ou de thioisocyanate conduiront aux composés où $XR_3$ est le groupement $-(CH_2)_s$-$CH_2$-NH-CO-NH-$R_{11}$ ou le groupement $-(CH_2)_s$-$CH_2$-NH-CS-NH-$R_{11}$.

De même, la condensation des dérivés bromés avec l'acétamido malonate d'éthyle préalablement métallé avec un alcoolate de sodium ou de potassium au reflux d'un alcool conduira aux composés de formule (XII) où $XR_3$ représentera

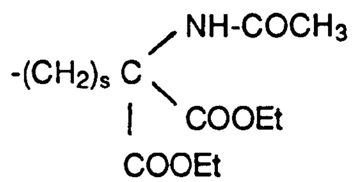

Ces composés par hydrolyse puis décarboxylation permettront d'obtenir les amino acides de formule (XII) où $XR_3$ représente

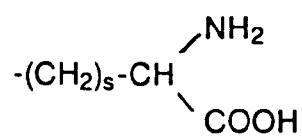

qui pourront être estérifiés et / ou transformés en amide selon des méthodes connues de l'homme de l'art par exemple par chauffage dans un alcool en présence de chlorure de thionyle ou par action d'un chlorure d'acide pour conduire aux dérivés de formule (XII) où $XR_3$ représente

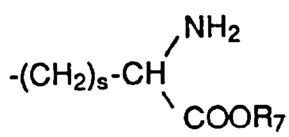

ou

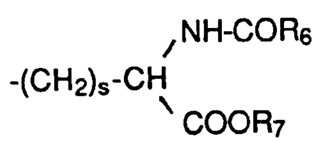

Les mêmes séquences de réaction pourront être appliquées sur les dérivés de formule (XII') dans lesquels $R'_3$ représente un groupement $-(CH_2)_n$-OH, n étant défini comme ci-dessus mais différent de zéro pour conduire aux dérivés où $R'_3$ représente les mêmes fonctions amino acides, amino esters ou amido acides,le traitement de l'alcool pouvant se faire ici par le chlorure de thionyle qui conduira au dérivé chloré lequel réagira comme les dérivés bromés précédents avec l'acétamido malonate d'éthyle métallé.

Dans certains cas, les fonctions amines ou acides pourront être protégées par des groupements benzyloxy carbonyl pour les amines ou terbutyloxy pour les acides puis libérées si nécessaire par hydrogénolyse ou par traitement à l'acide trifluoroacétique selon les méthodes classiques connues de l'homme de l'art.

Les composés de formule (XII) et (XII') dans lesquels :

- V est un groupement nitro pourront subir une hydrogénation catalytique, par exemple en présence de Nickel de Raney, dans un alcool à pression atmosphérique ou sous pression pour conduire aux composés de formule (XII) et (XII′) où V est un groupement amino.

Par action sur ce dérivé aminé d'un anhydride phtalique convenablement substitué on obtiendra les composés de formule générale (I) et (I′) où $R_4$ représente le groupement

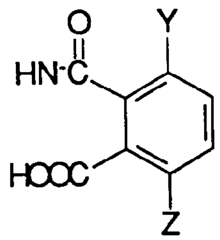

Y et Z étant défini comme ci-dessus l'acide obtenu pouvant être ensuite esterifié pour obtenir le groupement

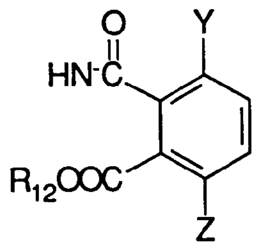

De même par action d'un anhydride benzosulfonique sur ces composés aminés on obtiendra les composés de formules générales (I) et (I′) où $R_4$ a représente le groupement

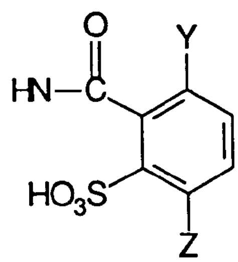

De même par action du chlorure de l'acide N-(trifluorométhyl sulfonyl) anthranylique dont on peut trouver la préparation dans les références :

CA 96 (13) : 103651 Z

CA 97 (7) : 55500W

sur ces composés aminés on obtiendra les composés de formules générales (I) et (I′) où $R_4$ représente le groupement

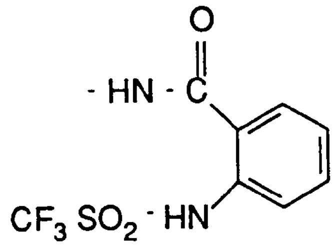

- V est un groupement $-COOR_{11}$ pourront être hydrolysés en milieu acide ou basique ou hydrogénés dans le cas où $R_{11}$ est un benzyle afin de respecter les autres fonctions esters présentes, pour conduire aux composés

de formules (I) et (I') où $R_4$ est un groupement -COOH.

Ces dérivés acides pourront conduire, après avoir été transformés en chlorure d'acide avec le chlorure de thionyle ou en anhydride mixte avec le chloroformiate d'éthyle, par réaction sur des dérivés anthraniliques de formule

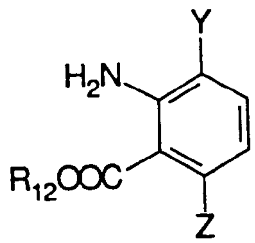

dans lesquels Y, Z et $R_{12}$ sont définis comme ci-dessus, aux composés de formules générales (I) et (I') où $R_4$ représente le groupement

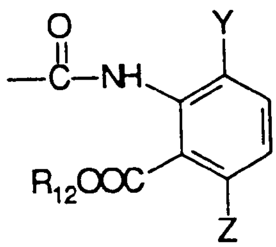

- V est le groupement

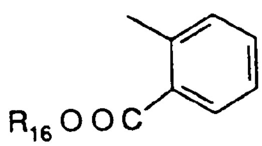

selon la même manière seront hydrolysés ou hydrogénés en présence d'un catalyseur tel que le palladium dans le cas où $R_{12}$ est un benzyle, pour conduire aux composés de formules (I) et (I') où $R_4$ est un groupement

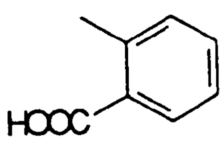

-V est un groupement

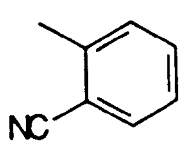

pourront réagir avec un équivalent d'azoture de sodium dans un solvant tel que le diméthyl formamide en présence d'un sel d'ammonium comme le chlorure d'ammonium ou par chauffage dans le toluène avec l'azoture de triméthyl étain suivi d'un traitement par l'acide chlorhydrique gazeux dans le tétrahydrofurane pour conduire aux composés de formule générale (I) ou (I') où $R_4$ représente un groupement

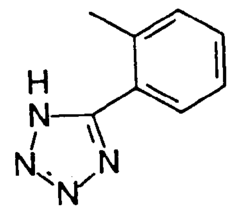

Pour mettre en oeuvre cette réaction, dans le cas où $R_3$ ou $R'_3$ possède une fonction alcool aliphatique il peut être souhaitable de la protéger selon les méthodes connues de l'homme de l'art par un acétate, un tétrahydropyrane puis de la libérer si nécessaire après formation du tétrazole.

- V est un groupement

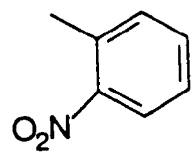

pourront subir une hydrogénation catalytique par exemple en présence de Nickel de Raney, dans un alcool à pression atmosphérique ou sous pression pour conduire à des composés de formule générale (I) ou (I′) où $R_4$ représente un groupement

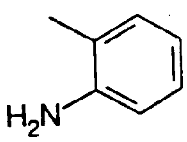

Par action du chlorure ou de l'anhydride de l'acide trifluorométhane sulfonique sur ces derniers dans un solvant tel que le chloroforme ou dans un solvant aromatique comme le toluène en présence d'une base comme la triéthylamine ou la pyridine ou dans la pyridine, on obtiendra les composés de formule générale (I) ou (I′) où $R_4$ représente un groupement

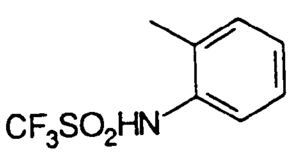

- V représente le groupement

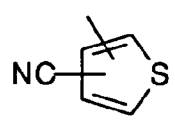

pourront être traités par un azoture de trialkyl étain dans le toluène au reflux puis par l'acide chlorhydrique gazeux dans le tétrahydrofurane pour conduire aux dérivés de formule (I) ou (I′) dans lesquels $R_4$ représente le groupement

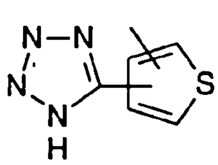

Pour mettre en oeuvre cette réaction, dans le cas où $R_3$ ou $R'_3$ possède une fonction alcool aliphatique, il peut

être souhaitable de la protéger selon les méthodes connues de l'homme de l'art par un acétate, un tétrahydropyrane puis de la libérer si nécessaire après formation du tétrazole.
- V représente le groupement

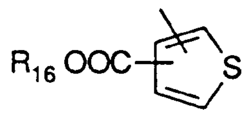

pourront être hydrolysés ou hydrogénés en présence d'un catalyseur tel que le Palladium sur charbon dans le cas où $R_{16}$ est un benzyle, pour conduire aux composés de formule (I) dans lesquels $R_4$ représente le groupement

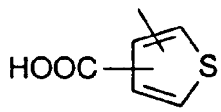

Des sels d'addition de certains composés de formules (I) et (I') peuvent s'obtenir, en particulier des sels d'addition pharmaceutiquement acceptables. On peut citer en particulier lorsque $R_2$, $R_3$, $R'_3$ ou $R_4$ présentent une fonction acide les sels de sodium, potassium, calcium, d'amine comme la dicyclohexylamine ou d'amino acide comme la lysine. Lorsque $R_2$, $R_3$, $R'_3$ ou $R_4$ présentent une fonction amine un sel d'acide minéral ou organique comme chlorhydrate, méthane sulfonate, acétate, maléate, succinate, fumarate, sulfate, lactate, citrate.

Les nouveaux composés selon l'invention possèdent des propriétés pharmacologiques remarquables comme antagonistes des récepteurs à l'angiotensine II et peuvent être utilisés en thérapeutique pour le traitement de maladies cardiovasculaires en particulier pour traiter l'hypertension et l'insuffisance cardiaque.

Ainsi, l'invention s'étend aux compositions pharmaceutiques renfermant comme principe actif, les médicaments constitués par une quantité pharmaceutiquement efficace d'au moins un composé de formule (I) ou (I'), tel que précédemment défini, ainsi qu'éventuellement ses sels d'addition pharmaceutiquement acceptables.

Ces compositions peuvent être administrées par voie buccale, rectale, par voie parentérale, par voie transdermique ou par voie occulaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques et les collyres. Elles sont préparées selon les méthodes usuelles. Le principe actif, constitué par une quantité pharmaceutiquement efficace d'au moins un composé de formule (I) ou (I') défini comme ci-dessus ou un de ses sels d'addition pharmaceutiquement acceptable, peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végérale, les glycols, les divers agents mouillants, dispersants ou émulsiants, les gels de silicone, certains polymères ou copolymères, les conservateurs, arômes et colorants.

L'invention couvre encore une composition pharmaceutique à activité antagoniste des récepteurs à l'angiotensine II permettant notamment de traiter favorablement les maladies cardiovasculaires en particulier l'hypertension et l'insuffisance cardiaque caractérisée en ce qu'elle comprend une quantité pharmaceutiquement efficace d'au moins un composé de formule (I) ou (I') précité ou un de ses sels d'addition pharmaceutiquement acceptable, pouvant être incorporé dans un excipient, véhicule ou support pharmaceutiquement acceptable.

La posologie varie notamment en fonction de la voie d'administration, de l'affection traitée et du sujet en cause.

Par exemple, chez l'adulte de poids moyen 60 à 70 kg, elle peut varier entre 1 et 400 mg de principe actif en une ou plusieurs doses journalières par voie orale, ou de 0,01 à 50 mg en une ou plusieurs doses journalières par voie parentérale.

L'invention couvre encore un procédé de préparation d'une composition pharmaceutique, caractérisé en ce qu'on incorpore une quantité pharmaceutiquement efficace d'au moins un composé de formule (I) ou (I') tel que défini précédemment, ou un de ses sels d'addition pharmaceutiquement acceptable, dans un excipient, véhicule ou support pharmaceutiquement acceptable, cette composition pharmaceutique étant par exemple formulée sous forme de gélules, de comprimés dosés de 1 à 400 mg ou sous forme de préparations injectables

dosées de 0,01 à 50 mg.

L'invention couvre encore un procédé de traitement thérapeutique des mammifères, caractérisé en ce que l'on administre à ce mammifère une quantité thérapeutiquement efficace d'au moins un composé de formule (I) ou (I') tel que précédemment défini, ou un de ses sels d'addition pharmaceutiquement acceptable.

En thérapeutique animale, la dose journalière utilisable devrait habituellement se situer de 1 à 100 mg par kg.

D'autres caractéristiques et avantages de l'invention seront mieux compris à la lecture qui va suivre de quelques exemples de préparation nullement limitatifs, mais donnés à titre d'illustration.

Exemple 1 : **oxo-3 hexanoate d'éthyle**

**Formule (II)** : $R_1$ = n-Propyl $R_{13}$ = Ethyl

176 g de diméthyl-2,2 dioxo-4,6 dioxanne-1,3 (acide de Meldrum) sont dissous dans 550 ml de dichlorométhane et 188 ml de pyridine. Le mélange est refroidi à 0°C par un bain d'eau et de glace et 133 ml de chlorure de butyryle sont ajoutés goutte à goutte. A la fin de l'addition le mélange est agité trois heures à température ambiante. La solution est lavée avec une solution d'acide chlorhydrique dilué, séchée sur sulfate de magnésium et évaporée sous vide pour donner une huile. Cette huile est dissoute dans 700 ml d'éthanol et le mélange est chauffé au reflux pendant six heures. L'éthanol est évaporé sous vide et le résidu obtenu est distillé pour donner 145,4 g d'oxo-3 hexanoate d'éthyle sous forme d'un liquide de point d'ébullition $Eb_{20}$ = 98-100°C.

Exemple 2 : **Oxo-3 heptanoate d'éthyle**

**Formule (II)** : $R_1$ = n-Butyl, $R_{13}$ = Ethyl

Préparé selon le mode opératoire de l'exemple 1.

Liquide de point d'ébullition $Eb_{20}$ = 115-120°C.

Exemple 3 : **(nitro-4 benzyl)-2 oxo-3 hexanoate d'éthyle**

**Formule (VI)**: $R_1$ = n-Propyl V = $NO_2$ $R_{13}$ = Ethyl

127,7 g d'oxo-3 hexanoate d'éthyle sont dissous dans 700 ml de tétrahydrofuranne. 174,5 g de bromure de nitro-4 benzyle et 35 g de chlorure de lithium sont ajoutés et le mélange est agité à température ambiante. On introduit alors goutte à goutte, 286 ml de diisopropyl éthyl amine, ce qui provoque un léger effet exothermique. Le mélange est ensuite agité trois heures à température ambiante, puis dix heures au reflux. Les solvants sont évaporés sous vide et le résidu est repris à l'eau puis extrait au chloroforme. La phase organique est décantée puis lavée avec une solution diluée d'acide chlorhydrique, séchée sur sulfate de magnésium et évaporée sous vide. Le résidu huileux obtenu est repris à l'éther isopropylique et les cristaux formés sont filtrés. Les eaux mères sont concentrées sous vide et le résidu est chauffé sous 20 mm de mercure à 130°C afin d'éliminer les produits de départ résiduels. On obtient ainsi 174 g de (nitro-4 benzyl)-2 oxo-3 hexanoate d'éthyle sous forme d'une huile utilisée telle quelle pour la suite.

Exemple 4 : **(nitro-4 benzyl)-2 oxo-3 heptanoate d'éthyle**

**Formule (VI)** : $R_1$ = n-Butyl, V = $NO_2$, $R_{13}$ = Ethyl

Préparé selon le mode opératoire de l'exemple 3.

Huile utilisée telle quelle pour la suite.

Exemple 5 : **(méthoxy carbonyl-2' biphényl-yl-4) méthyl-2 oxo-3 hexanoate d'éthyle**

**Formule (VI)** : $R_1$ = n-Propyl, V = 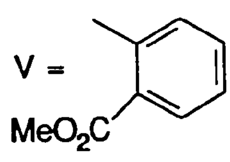 , $R_{13}$ = Ethyl

Préparé selon le mode opératoire de l'exemple 3, à partir de l'oxo-3 hexanoate d'éthyle préparé à l'exemple 1 et du (bromométhyl-4' biphényl-yl-2) carboxylate de méthyle.

Huile utilisée telle quelle pour la suite.

Préparation du (bromométhyl-4'biphényl-yl-2) carboxylate de méthyle

A) (Méthyl-4'biphényl-yl-2) carboxylate de méthyle.

A 300 ml de méthanol refroidi à 0°C, sont ajoutés 15 ml de chlorure d'acétyle. Le mélange est agité 10 minutes à cette température puis 15g d'acide (méthyl-4'biphényl-yl-2) carboxylique, préparé selon MEYERS.A.I ; MIHELICH E.D., J.Am.Chem.Soc, 1975, 97 (25), 7383 à partir du bromure de (méthyl-4 phényl) magnésium sur la (méthoxy-2 phényl)-2 diméthyl-4,4-oxazolidine, sont ajoutés. Le mélange est alors chauffé au reflux pendant 4 heures, les solvants sont évaporés sous vide pour donner 16g de (méthyl-4'biphényl-yl-2) carboxylate de méthyle sous forme d'une huile utilisée telle quelle pour la suite.

B) (bromométhyl-4'biphényl-yl-2) carboxylate de méthyle.

16g de (méthyl-4'biphényl-yl-2) carboxylate de méthyle préparés en A) sont dissous dans 120 ml de tetrachlorure de carbone en présence de 12,6g de N-bromo succinimide et de 0,5g de peroxyde de benzoyle. Le mélange est porté au reflux durant 6 heures, les cristaux sont filtrés et la solution restante est lavée avec une solution de bicarbonate de sodium puis évaporée sous vide. Le résidu est repris à l'éther puis la solution est filtrée sur charbon et évaporée sous vide pour donner 14,5g de (bromométhyl-4'biphényl-yl-2) carboxylate de méthyle sous forme d'une huile utilisée telle quelle pour la suite.

Exemple 6 : **(cyano-2' biphényl-yl-4) méthyl-2 oxo-3 hexanoate d'éthyle**

**Formule (VI)** : $R_1$ = n-Propyl, $R_{13}$ = Ethyl

V =

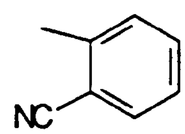

Préparé selon le mode opératoire de l'exemple 3 à partir du bromométhyl-4'cyano-2 biphényl.
Huile utilisée telle quelle pour la suite.

Préparation du bromométhyl-4'cyano-2 biphényl :

A) Méthyl-4'cyano-2 biphényl:

18,5g d'acide (méthyl-4'biphényl-yl-2) carboxylique préparé comme à l'exemple 5 A), sont chauffés au reflux dans 60 ml de chlorure de thionyle pendant deux heures. Le chlorure de thionyle est concentré sous vide et le résidu est versé sur une solution à 28% d'hydroxyde d'ammonium, le mélange est agité pendant 30 minutes et les cristaux obtenus sont essorés et lavés à l'éther puis séchés pour donner 14,5g de (méthyl-4'biphényl-yl-2) carboxamide sous forme de cristaux de point de fusion 128°C. Ces cristaux sont repris dans 50 ml de chlorure de thionyle et le mélange est chauffé 3 heures au reflux puis concentré sous vide pour donner 9g de méthyl-4'cyano-2 biphényl sous forme de cristaux de point de fusion 45-46°C.

B) Bromométhyl-4'cyano-2 biphényl :

7,9g de méthyl-4'cyano-2 biphényl préparé en A) sont dissous dans 100 ml de tétrachlorure de carbone en présence de 7,3g de N-bromo succinimide et de 0,3g de peroxyde de benzoyle. Le mélange est chauffé au reflux pendant 6 heures et les cristaux sont filtrés, la solution restante est concentrée sous vide et le résidu est cristallisé dans l'éther pour donner 6,6g de bromométhyl-4'cyano-2 biphényl sous forme de cristaux de point de fusion 115-118°C.

Exemple 7 : **n-Propyl-6 méthyl-2 hydroxy-4 (nitro-4 benzyl)-5 pyrimidine**

**Formule (XI)** : $R_1$ = n-Propyl, $R_2$ = Méthyl, T = OH, V = $NO_2$

3,5 g de sodium sont dissous dans 175 ml d'éthanol. A cette solution sont ajoutés 9,5 g de chlorhydrate d'acétamidine et le mélange est agité cinq minutes à température ambiante. 20 g de (nitro-4 benzyl)-2 oxo-3

hexanoate d'éthyle, préparé à l'exemple 3, sont alors ajoutés et le mélange est agité durant quatre jours à température ambiante. Les solvants sont ensuite évaporés sous vide et le résidu est repris avec une solution d'acide chlorhydrique et extraite à l'acétate d'éthyle. La phase organique est séchée sur sulfate de magnésium puis évaporée sous vide pour donner un résidu huileux qui cristallise dans un mélange acétone/éther. Les cristaux essorés et séchés donnent 10,9 g de n-propyl-6 méthyl-2 hydroxy-4 (nitro-4 benzyl)-5 pyrimidine sous forme de cristaux de point de fusion 200°C.

Exemple 8 : **n-Butyl-6 méthyl-2 hydroxy-4 (nitro-4 benzyl)-5 pyrimidine**

**Formule (XI)** : $R_1$ = n-Butyl, $R_2$ = Méthyl, T = OH, V = $NO_2$

Préparé selon le mode opératoire de l'exemple 7.
Cristaux de point de fusion 205°C.

Exemple 9 : **n-Propyl-6 méthyl-2 hydroxy-4 (méthoxy carbonyl-2′ biphényl-yl-4) méthyl-5 pyrimidine**

**Formule (XI)** : $R_1$ = n-Propyl, V = 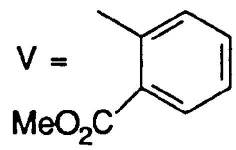 ,

$R_2$ = Méthyl, T = OH

Préparé selon le mode opératoire de l'exemple 7.
Cristaux de point de fusion 206°C.

Exemple 10 : **n-Propyl-6 méthyl-2 hydroxy-4 (cyano-2′ biphényl-yl-4) méthyl-5 pyrimidine**

**Formule (XI)** : $R_1$ = n-Propyl, $R_2$ = Méthyl, T = OH

V = 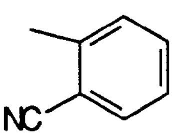

Préparé selon le mode opératoire de l'exemple 7.
Cristaux de point de fusion 206°C.

Exemple 11 : **[n-Propyl-4 méthyl-2 (nitro-4 benzyl)-5 oxo-6 pyrimidine-yl-1 (6H)-] acétate d'éthyle**

**Formule (XII′)** : $R_1$ = n-Propyl, $R_2$ = Méthyl, $R'_3$ = $CH_2CO_2Et$, V = $NO_2$

4 g de n-propyl-6 méthyl-2 hydroxy-4 (nitro-4 benzyl)-5 pyrimidine, préparé à l'exemple 7, sont dissous dans 30 ml de N,N diméthyl formamide anhydre. 0,6 g d'hydrure de sodium à 60 % sont ajoutés et le mélange est agité trente minutes à température ambiante. 2 ml de bromo acétate d'éthyle sont ensuite ajoutés et le mélange est alors chauffé une heure à 60°C. Après refroidissement, 200 ml d'eau sont rajoutés et la solution est extraite à l'éther, la phase organique est séchée sur sulfate de magnésium et évaporée sous vide pour donner un résidu qui cristallise dans un mélange éther/pentane pour donner 2 g de [n-Propyl-4 méthyl-2 (nitro-4 benzyl)-5 oxo-6 pyrimidine-yl-1 (6H)-] acétate d'éthyle sous forme de cristaux de point de fusion 120°C.

Exemple 12 : **[n-Propyl-4 méthyl-2 (amino-4 benzyl)-5 oxo-6 pyrimidine-yl-1 (6H)-] acétate d'éthyle**

**Formule (XII′)** : $R_1$ = n-Propyl, $R_2$ = Méthyl, $R'_3$ = $CH_2CO_2Et$, V = $NH_2$

2 g de [n-propyl-4 méthyl-2 (nitro-4 benzyl)-5 oxo-6 pyrimidine-yl-1 (6H)-] acétate d'éthyle, préparés à l'exemple 11, sont dissous dans 25 ml d'éthanol en présence de 0,5 g de Nickel de Raney, et mis à hydrogéner sous pression atmosphérique et à température ambiante. Quand la prise d'hydrogène a cessé, le catalyseur

est filtré et l'éthanol est évaporé sous vide pour donner 1,8 g de [n-Propyl-4 méthyl-2 (amino-4 benzyl)-5 oxo-6 pyrimidine-yl-1 (6H)-] acétate d'éthyle sous forme d'une huile utilisée telle quelle pour la suite.

Exemple 13 : **[n-propyl-4 méthyl-2 oxo-6 [(sulfoxy-2 phényl) carbonyl amino-4 phényl méthyl]-5 pyrimidine-yl-1 (6H)-] acétate d'éthyle**

**Formule (I')** : $R_1$ = n-Propyl, $R_2$ = Méthyl, $R'_3 = CH_2CO_2Et$,

$R_4$ = 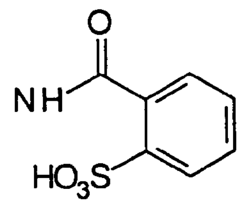

1 g de [n-propyl-4 méthyl-2 (amino-4 benzyl)-5 oxo-6 pyrimidine-yl-1 (6H)-] acétate d'éthyle préparé à l'exemple 12, sont dissous dans 10 ml d'acétonitrile. Une solution de 0,6 g d'anhydride sulfobenzoïque dans 5 ml d'acétonitrile est ajoutée et le mélange est abandonné un jour à température ambiante. Les cristaux formés sont essorés et lavés à l'acétonitrile puis à l'éther et séchés pour donner 0,9 g [n-propyl-4 méthyl-2 oxo-6 [(sulfoxy-2 phényl) carbonyl amino-4 phényl méthyl]-5 pyrimidine-yl-1 (6H)-] acétate d'éthyle sous forme de cristaux de point de fusion 268-269°C.

Exemple 14 : **Acide [n-propyl-4 méthyl-2 oxo-6 [(carboxy-2 dichloro-3,6-phényl) carbonyl amino-4 phényl méthyl]-5 pyrimidine-yl-1 (6H)-] acétique**

**Formule (I')** : $R_1$ = n-Propyl, $R_2$ = Méthyl, $R'_3 = CH_2CO_2H$,

$R_4$ = 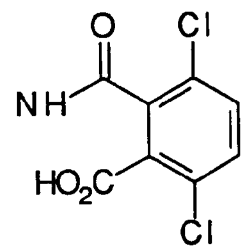

1,1 g de [n-propyl-4 méthyl-2 (amino-4 benzyl)-5 oxo-6 pyrimidine-yl-1 (6H)-] acétate d'éthyle, préparé à l'exemple 12, sont dissous dans 10 ml d'acétonitrile en présence de 0,7 g d'anhydride dichloro-3,6-phtalique. Le mélange est abandonné à température ambiante pendant une nuit et l'acétonitrile est évaporé sous vide sans trop chauffer. Le résidu obtenu est repris dans l'eau en présence de 0,6 g de soude en pastilles et agité 30 minutes à 45°C. La solution est ensuite neutralisée par l'acide chlorhydrique concentré et les cristaux obtenus sont essorés et lavés à l'eau puis séchés pour donner 0,6 g Acide [n-propyl-4 méthyl-2 oxo-6 [(carboxy-2 dichloro-3,6-phényl) carbonyl amino-4 phényl méthyl]-5 pyrimidine-yl-1 (6H)-] acétique sous forme de cristaux de point de fusion 167-168°C.

Exemple 15 : **n-Propyl-6 méthyl-2 hydroxy éthyl oxy-4 (méthoxy carbonyl-2′ biphényl-yl-4) méthyl-5 pyrimidine**

**Formule (I)** : $R_1$ = n-Propyl, $R_2$ = Méthyl, $R_3 = CH_2CH_2OH$,

X = O, $R_4$ = 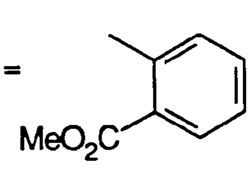

5,5 g de n-propyl-6 méthyl-2 hydroxy-4 (méthoxy carbonyl-2′ biphényl-yl-4) méthyl-5 pyrimidine, préparé

à l'exemple 9, sont dissous dans 50 ml d'acétonitrile en présence de 3 g de carbonate de potassium et 0,3 g de chlorhydrate de triéthyl benzylamine. 2 ml de bromo-2 éthanol sont ajoutés et le mélange est chauffé au reflux pendant dix heures. Le solvant est évaporé à sec et le résidu est repris à l'eau puis extrait à l'éther. La phase organique est séchée puis évaporée à sec et le résidu huileux obtenu est chromatographié sur silice dans l'éther pour donner 3 g de n-propyl-6 méthyl-2 hydroxy éthyl oxy-4 (méthoxy carbonyl-2'biphényl-yl-4) méthyl-5 pyrimidine sous forme d'une huile incolore utilisée telle quelle pour la suite.

Exemple 16 : **Acide [n-propyl-6 méthyl-2 hydroxy éthyl oxy-4 pyrimidine-yl-5] méthyl-4' biphényl-2 carboxylique**

**Formule (I)** : $R_1$ = n-Propyl, $R_2$ = Méthyl, $R_3 = CH_2CH_2OH$,

$X = O$ , $R_4$ = 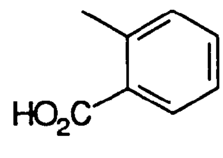

1,8 g de n-propyl-6 méthyl-2 hydroxy éthyl oxy-4 (méthoxy carbonyl-2'biphényl-yl-4) méthyl-5 pyrimidine, préparée à l'exemple 15, sont dissous dans 20 ml d'éthanol. Une solution de 1 g de soude dans 10 ml d'eau distillée est rajoutée et le mélange est chauffé deux heures à 50°C. Les solvants sont évaporés à sec et le résidu, repris à l'eau, est lavé par de l'acétate d'éthyle. La phase aqueuse est acidifiée par barbotage de dioxide de soufre et extraite au chloroforme. La phase organique est séchée sur sulfate de magnésium et évaporée à sec sous vide. Le résidu obtenu cristallise dans l'éther isopropylique pour donner 1,2 g d'acide [n-propyl-6 méthyl-2 hydroxy éthyl oxy-4 pyrimidine-yl-5] méthyl-4'biphényl-2 carboxylique sous forme de cristaux de point de fusion 143-144°C.

Exemple 17: **[n-propyl-4 méthyl-2 oxo-6 (nitro- benzyl)-5 pyrimidine-yl-1 (6H)-] acétyl morpholine**

**Formule (XII')** : $R_1$ = n-Propyl, $R_2$ = Méthyl,

$R'_3 = CH_2$ 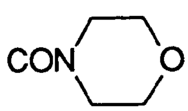 , $V = NO_2$

Préparé selon le mode opératoire de l'exemple 11, à partir de la N-(chloro acétyl) morpholine et du n-propyl-6 méthyl-2 hydroxy-4 (nitro-4 benzyl)-5 pyrimidine, préparé à l'exemple 7.

Cristaux de point de fusion 174°C.

Exemple 18 : **[n-propyl-4 méthyl-2 oxo-6 (amino-4 benzyl)-5 pyrimidine-yl-1 (6H)-] acétyl morpholine**

**Formule (XII')** : $R_1$ = n-Propyl, $R_2$ = Méthyl,

$R'_3 = CH_2$ 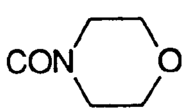 , $V = NH_2$

Préparé selon le mode opératoire de l'exemple 12.

Cristaux de point de fusion 160°C.

Exemple 19 : **n-Propyl-6 méthyl-2 (nitro-4 benzyl)-5 chloro-4 pyrimidine**

**Formule (XII)** : $R_1$ = n-Propyl, $R_2$ = Méthyl, $XR_3$ = Cl, $V = NO_2$

32 g de n-propyl-6 méthyl-2 (nitro-4 benzyl)-5 hydroxy-4 pyrimidine, préparée à l'exemple 7, sont mis en suspension dans 45 ml d'oxychlorure de phosphore. Le mélange est porté au reflux durant 6 heures, puis concentré sous vide. Le résidu est repris à l'eau et extrait au dichlorométhane. La phase organique est lavée par une solution de carbonate de potassium puis séchée sur sulfate de magnésium et évaporée à sec pour donner 24 g de n-propyl-6 méthyl-2 (nitro-4 benzyl)-5 chloro-4 pyrimidine sous forme de cristaux de point de fusion 65°C.

Exemple 20 : **n-Butyl-6 méthyl-2 (nitro-4 benzyl)-5 chloro-4 pyrimidine**

**Formule (XII)** : $R_1$ = n-Butyl, $R_2$ = Méthyl, $XR_3$ = Cl, V = $NO_2$

Préparé selon le mode opératoire de l'exemple 19.

Cristaux de point de fusion 45°C.

Exemple 21 : **n-Propyl-6 méthyl-2 (cyano-2′ biphényl-yl-4) méthyl-5 chloro-4 pyrimidine**

**Formule (XII)** : $R_1$ = n-Propyl, $R_2$ = Méthyl, $XR_3$ = Cl

V = 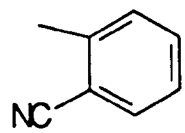

Préparé selon le mode opératoire de l'exemple 19.

Cristaux de point de fusion 95°C.

Exemple 22 : **[n-Propyl-6 méthyl-2 (nitro-4 benzyl)-5 pyrimidine-yl-4] mercapto acétate d'éthyle**

**Formule (XII)** : $R_1$ = n-Propyl, $R_2$ = Méthyl, X = S, $R_3$ = $CH_2CO_2Et$, V = $NO_2$

4 g de n-propyl-6 méthyl-2 (nitro-4 benzyl)-5 chloro-4 pyrimidine et 1,9 g de mercapto acétate d'éthyle sont dissous dans 50 ml d'éthanol. 1,2 g d'acétate de sodium anhydre sont ajoutés et le mélange est agité 6 heures à température ambiante, puis 6 heures au reflux L'éthanol est évaporé sous vide et le résidu est repris à l'eau puis extrait au dichlorométhane. La phase organique est séchée sur sulfate de magnésium et évaporée à sec sous vide pour donner une huile qui cristallise dans un mélange pentane/éther isopropylique pour donner 2,9 g de [n-propyl-6 méthyl-2 (nitro-4 benzyl)-5 pyrimidine-yl-4] mercapto acétate d'éthyle sous forme de cristaux de point de fusion 66-67°C.

Exemple 23 : **[n-Propyl-6 méthyl-2 (amino-4 benzyl)-5 pyrimidine-yl-4] mercapto acétate d'éthyle**

**Formule (XII)** : $R_1$ = n-Propyl, $R_2$ = Méthyl, X = S, $R_3$ = $CH_2CO_2Et$, V = $NH_2$

Préparé selon le mode opératoire de l'exemple 12.

Huile utilisée telle quelle pour la suite.

Exemple 24 : **Acide [[n-Propyl-6 méthyl-2 (éthoxycarbonyl méthyl mercapto)-4 pyrimidine-yl-5] méthyl phényl-yl-4] amino carbonyl-2 benzène sulfonique**

**Formule (I)** : $R_1$ = n-Propyl, $R_2$ = Méthyl, $R_3$ = $CH_2CO_2Et$

X = S  $R_4$ = 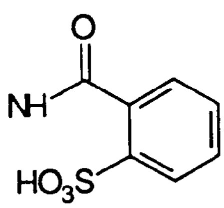

Préparé selon le mode opératoire de l'exemple 13.

Cristaux de point de fusion 252-253°C.

Exemple 25 : **n-Propyl-6 méthyl-2 (N,N diméthyl amino carbonyl oxy)-4 (nitro-4 benzyl)-5 pyrimidine**

**Formule (XII)** : $R_1$ = n-Propyl, $R_2$ = Méthyl, X = O

$R_3$ = 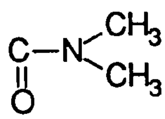 , V = $NO_2$

5 g de n-propyl-6 méthyl-2 hydroxy-4 (nitro-4 benzyl)-5 pyrimidine, préparé à l'exemple 7, sont dissous dans 100 ml de dichlorométhane en présence de 2,6 ml de triéthylamine. 1,7 ml de chlorure de l'acide N,N-diméthyl carbamique sont ajoutés et le mélange est chauffé 12 heures au reflux. Après refroidissement la solution est lavée par une solution diluée d'acide chlorhydrique, séchée sur sulfate de magnésium et évaporée à sec. Le résidu est chromatographié sur gel de silice, dans un éluant dichlorométhane 9 / acétone 1, pour donner 3,1 g de n-propyl-6 méthyl-2 (N,N-diméthyl amino carbonyl oxy)-4 (nitro-4 benzyl)-5 pyrimidine sous forme de cristaux de point de fusion 110°C.

Exemple 26 : **n-Propyl-6 méthyl-2 (N,N diméthyl amino carbonyl oxy)-4 (amino-4 benzyl)-5 pyrimidine**

**Formule (XII)** : $R_1$ = n-Propyl, $R_2$ = Méthyl, X = O

$R_3$ = 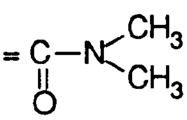 , V = $NH_2$

Préparé selon le mode opératoire de l'exemple 12.
Huile utilisée telle quelle pour la suite.

Exemple 27 : **Acide [[n-propyl-6 méthyl-2 (N,N-diméthyl amino carbonyl oxy)-4 pyrimidine-yl-5] méthyl phényl-yl-4] amino carbonyl-2 benzène sulfonique**

Formule (I) : $R_1$ = n-Propyl, $R_2$ = Méthyl, X = O

$R_3$ = 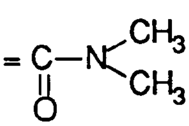 , $R_4$ = 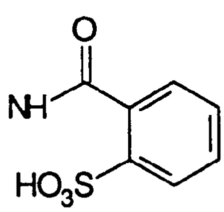

Préparé selon le mode opératoire de l'exemple 13.
Cristaux de point de fusion 215-218°C.

Exemple 28 : **[n-Propyl-4 méthyl-2 oxo-6 (cyano-2′ biphényl-yl-4) méthyl-5 pyrimidine-yl-1 (6H)-] acétate d'éthyle**

**Formule (XII')** : $R_1$ = n-Propyl, $R_2$ = Méthyl, $R'_3$ = $CH_2CO_2Et$,

V = 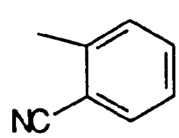

Préparé selon le mode opératoire de l'exemple 11.
Cristaux de point de fusion 98°C.

Exemple 29 : **[n-Propyl-6 méthyl-2 (cyano-2′ biphényl-yl-4) méthyl-5 pyrimidine-yl-4] mercapto acétate d'éthyle**

**Formule (XII)** : $R_1$ = n-Propyl, $R_2$ = Méthyl, $R_3$ = $CH_2CO_2Et$

X = S, V =

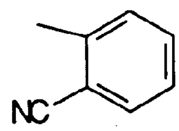

Préparé selon le mode opératoire de l'exemple 22.
Huile utilisée telle quelle pour la suite.

Exemple 30 : **n-Propyl-6 méthyl-2 méthoxy-4 (cyano-2′ biphényl-yl-4) méthyl-5 pyrimidine**

**Formule (XII)** : $R_1$ = n-Propyl, $R_2$ = méthyl, $R_3$ = Méthyl

X = O, V =

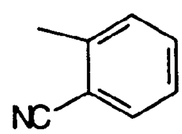

12 g de n-Propyl-6 méthyl-2 chloro-4 (cyano-2′biphényl-4) méthyl-5 pyrimidine préparé à l'exemple 21 sont dissous dans 100 ml de méthanol. 1,2 g de sodium en solution dans 20 ml de méthanol sont ajoutés et le mélange est agité 3 heures à température ambiante puis une heure à 40°C et enfin une heure au reflux. Le mélange réactionnel est concentré puis additionné d'eau et extrait à l'éther isopropylique. La phase organique est séchée puis évaporée sous vide et le résidu obtenu cristallise dans un mélange éther isopropylique/pentane pour donner 8,9 g de n-Propyl-6 méthyl-2 méthoxy-4 (cyano-2′biphényl-yl-4) méthyl-5 pyrimidine sous forme de cristaux de point de fusion 108°C.

Exemple 31 : **n-Propyl-6 méthy1-2 méthoxy-4 [(tétrazol-yl-5)-2′ biphényl-yl-4] méthyl-5 pyrimidine**

**Formule (I)** : $R_1$ = n-Propyl, $R_2$ = Méthyl, $R_3$ = Méthyl

X = O , $R_4$ =

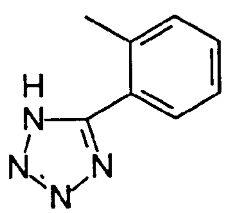

8,9 g de n-Propyl-6 méthyl-2 méthoxy-4 (cyano-2′biphényl-yl-4) méthyl-5 pyrimidine préparé à l'exemple 30 et 6,3 g d'azoture de triméthyl étain sont chauffés 12 heures au reflux dans 100 ml de toluène. Les cristaux obtenus sont filtrés à chaud pour donner 6,6 g de n-Propyl-6 méthyl-2 méthoxy-4 [(triméthyl stanyl-1 tétrazol-yl-5)-2′biphényl-yl-4] méthyl-5 pyrimidine sous forme de cristaux de point de fusion 235°C. Ces cristaux sont dissous dans un mélange toluène/THF (60 ml/10 ml) et traités par de l'acide chlorhydrique gazeux pendant une heure à température ambiante. Le mélange est concentré et repris au chloroforme puis lavé à l'eau. La phase organique est séchée sur sulfate de magnésium et évaporée sous vide pour donner une huile qui cristallise dans un mélange éther/acétone pour donner 4,2 g de n-Propyl-6 méthyl-2 méthoxy-4 [(tétrazol-yl-5)-2′biphényl-yl-4] méthyl-5 pyrimidine de point de fusion 132-4°C.

Exemple 32 : **[n-Propyl-4 méthyl-2 oxo-6 [(tétrazol-yl-5)-2′ biphényl-yl-4] méthyl-5 pyrimidine-yl-1 (6H)-] acétate d'éthyle**

**Formule (I')** : $R_1$ = n-Propyl, $R_2$ = Méthyl,

$R'_3 = CH_2CO_2Et$, $R_4$ = 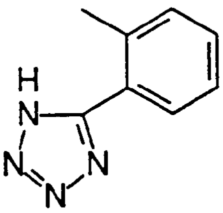

Préparé selon le mode opératoire de l'exemple 31.
Cristaux de point de fusion 164-5°C.

Exemple 33 : **[ n-Propyl-6 méthyl-2 [(tétrazol-yl-5)-2′ biphényl-yl-4] méthyl-5 pyrimidine-yl-4] mercapto acétate d'éthyle**

**Formule (I)** : $R_1$ = n-Propyl, $R_2$ = Méthyl, X = S,

$R_3 = CH_2CO_2Et$, $R_4$ = 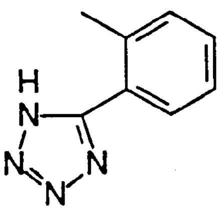

Préparé selon le mode opératoire de l'exemple 31.
Cristaux de point de fusion 137-8°C.

Exemple 34 : **[n-Butyl-6 méthyl-2 (nitro-4 benzyl)-5 pyrimidine-yl-4] mercapto-2 éthanol**

**Formule (XII)** : $R_1$ = n-Butyl, $R_2$ = Méthyl, X = S, $R_3 = CH_2CH_2OH$, V = $NO_2$

10 g de n-Butyl-6 méthyl-2 (nitro-4 benzyl)-5 chloro-4 pyrimidine, préparés à l'exemple 20, sont dissous dans 150 ml de butanone-2. 7 g de carbonate de potassium et 3,7 g de mercapto-2 éthanol sont rajoutés et le mélange est porté 6 heures au reflux. Après concentration du solvant sous vide, le résidu est repris à l'eau puis extrait à l'éther, la phase organique est lavée à l'eau puis séchée sur sulfate de magnésium et évaporée à sec pour donner un résidu qui cristallise dans l'éther isopropylique pour donner 5,5 g de [n-Butyl-6 méthyl-2 (nitro-4 benzyl)-5 pyrimidine-yl-4] mercapto-2 éthanol de point de fusion 65°C.

Exemple 35 : **[n-Propyl-6 méthyl-2 (nitro-4 benzyl)-5 pyrimidine-yl-4] mercapto-2 éthanol**

**Formule (XII)** : $R_1$ = n-Propyl, $R_2$ = Méthyl, X = S, $R_3 = CH_2CH_2OH$, V = $NO_2$

Préparé selon le mode opératoire de l'exemple 34.
Cristaux de point de fusion 110°C.

Exemple 36 : **[n-Butyl-6 méthyl-2 (nitro-4 benzyl)-5 pyrimidine-yl-4] mercapto acétate d'éthyle**

**Formule (XII)** : $R_1$ = n-Butyl, $R_2$ = Méthyl, X = S, $R_3 = CH_2CO_2Et$, V = $NO_2$

Préparé selon le mode opératoire de l'exemple 22.
Huile utilisée telle quelle pour la suite.

Exemple 37 : **n-Butyl-6 méthyl-2 amino-4 (nitro-4 benzyl)-5 pyrimidine**

**Formule (XII)** : $R_1$ = n-Butyl, $R_2$ = Méthyl, $XR_3 = NH_2$, V = $NO_2$

25,4 g de n-Butyl-6 méthyl-2 chloro-4 (nitro-4 benzyl)-5 pyrimidine préparés à l'exemple 20, sont chauffés

dans 400 ml de méthanol ammoniacal à 170°C, pendant 12 heures, dans un autoclave. Après refroidissement, la solution est évaporée sous vide et le résidu obtenu est repris à l'eau puis extrait au dichlorométhane. La phase organique est séchée sur sulfate de magnésium et évaporée sous vide. Le résidu obtenu cristallise dans l'éther pour donner 15,4 g de n-Butyl-6 méthyl-2 amino-4 (nitro-4 benzyl)-5 pyrimidine sous forme de cristaux de point de fusion 135°C.

Exemple 38 : **n-Propyl-6 Méthyl-2 amino-4 (nitro-4 benzyl)-5 pyrimidine**

**Formule (XII)** : $R_1$ = n-Propyl, $R_2$ = Méthyl, $XR_3 = NH_2$, $V = NO_2$

Préparé selon le mode opératoire de l'exemple 37.
Cristaux de point de fusion 152°C.

Exemple 39 : **N-[n-butyl-6 méthyl-2 (nitro-4 benzyl)-5 pyrimidine-yl-4] morpholine**

**Formule (XII)** : $R_1$ = n-Butyl, $R_2$ = Méthyl, $V = NO_2$,

$XR_3$ = 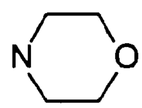

8 g de n-butyl-6 méthyl-2 chloro-4 (nitro-4 benzyl)-5 pyrimidine, préparés à l'exemple 20, sont dissous dans 100 ml de xylène. 10 ml de morpholine sont ajoutés et le mélange est chauffé au refux pendant 16 heures. Après refroidissement le xylène est évaporé sous vide et le résidu repris à l'éther est lavé par une solution de soude diluée, puis à l'eau. La phase éthérée est séchée sur sulfate de magnésium et évaporée sous vide pour donner un résidu qui cristallise dans l'éther isopropylique pour donner 5 g de N-[n-butyl-6 méthyl-2 (nitro-4 benzyl)-5 pyrimidine-yl-4] morpholine sous forme de cristaux de point de fusion 124°C.

Exemple 40 : **N-[n-Propyl-6 méthyl-2 (nitro-4 benzyl)-5 pyrimidine-yl-4] morpholine**

**Formule (XII)** : $R_1$ = n-Propyl, $R_2$ = Méthyl, $V = NO_2$,

$XR_3$ = 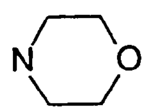

Préparé selon le mode opératoire de l'exemple 39.
Cristaux de point de fusion 125°C.

Exemple 41 : **N-[n-Propyl-6 méthyl-2 (nitro-4 benzyl)-5 pyrimidine-yl-4] thiomorpholine**

**Formule (XII)** : $R_1$ = n-Propyl, $R_2$ = Méthyl, $V = NO_2$

$XR_3$ = 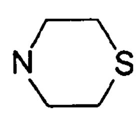

Préparé selon le mode opératoire de l'exemple 39.
Cristaux de point de fusion 133°C.

Exemple 42 : **[n-Propyl-6 méthyl-2 (nitro-4 benzyl)-5 pyrimidine-yl-4]-4 (méthoxy-2 phényl)-1 pipérazine**

**Formule (XII)** : $R_1$ = n-Propyl, $R_2$ = Méthyl, V = $NO_2$,

$XR_3$ = 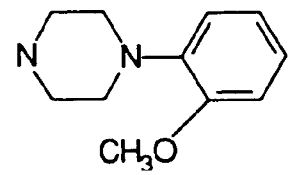

Préparé selon le mode opératoire de l'exemple 39.
Cristaux de point de fusion 110°C.

Exemple 43 : **n-propyl-6 méthyl-2 (nitro-4 benzyl)-5 mercapto-4 pyrimidine**

**Formule (XII)** : $R_1$ = n-Propyl, $R_2$ = Méthyl, $XR_3$ = SH, V = $NO_2$

24 g de n-Propyl-6 méthyl-2 (nitro-4 benzyl)-5 chloro-4 pyrimidine préparés à l'exemple 19, et 5,5 g de thiourée sont chauffés 6 heures au reflux dans 75 ml d'acétone en présence de 0,05 ml d'acide chlorhydrique concentré. Les cristaux obtenus sont essorés et lavés à l'acétone puis repris dans un mélange composé de 150 ml d'éthanol, 150 ml d'eau et 25 g de soude en pastille. Le mélange est porté au reflux 5 heures puis les solvants sont évaporés sous vide, le résidu repris à l'eau puis acidifié par l'acide chlorhydrique concentré, les cristaux obtenus sont essorés et lavés à l'eau pour donner 12 g de n-Propyl-6 méthyl-2 (nitro-4 benzyl)-5 mercapto-4 pyrimidine de point de fusion 212°C.

Exemple 44 : **n-Propyl-6 méthyl-2 méthylthio-4 (nitro-4 benzyl)-5 pyrimidine**

**Formule (XII)** : $R_1$ = n-Propyl, $R_2$ = Méthyl, $XR_3$ = $SCH_3$, V = $NO_2$

4 g de n-Propyl-6 méthyl-2 mercapto-4 (nitro-4 benzyl)-5 pyrimidine, préparés à l'exemple 43, sont dissous dans 75 ml d'éthanol. 0,6 g de soude, en solution dans 10 ml d'eau, sont rajoutés et le mélange est agité à température ambiante durant quelques minutes. 1,5 ml d'iodure de méthyle sont alors introduits dans le milieu réactionnel et la solution est agitée 2 heures à température ambiante puis 2 heures au reflux. Après évaporation sous vide des solvants, le résidu est repris à l'eau puis extrait à l'éther ; la phase éthérée est séchée sur sulfate de magnésium, puis évaporée à sec pour donner 3,7 g de n-Propyl-6 méthyl-2 méthylthio-4 (nitro-4 benzyl)-5 pyrimidine sous forme d'une huile utilisée telle quelle pour la suite.

Exemple 45 : **[n-Propyl-6 méthyl-2 (amino-4 benzyl)-5 pyrimidine-yl-4] mercapto-2 éthanol**

**Formule (XII)** : $R_1$ = n-Propyl, $R_2$ = Méthyl, $R_3$ = $CH_2CH_2OH$, X = S, V = $NH_2$

Préparé selon le mode opératoire de l'exemple 12.
Huile utilisée telle quelle pour la suite.

Exemple 46 : **N-[n-Propyl-6 méthyl-2 (amino-4 benzyl)-5 pyrimidine-yl-4] morpholine**

**Formule (XII)** : $R_1$ = n-Propyl, $R_2$ = Méthyl, V = $NH_2$

$XR_3$ = 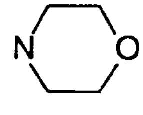

Préparé selon le mode opératoire de l'exemple 12.
Huile utilisée telle quelle pour la suite.

Exemple 47: **N-[n-Propyl-6 méthyl-2 (amino-4 benzyl)-5 pyrimidine-yl-4] thiomorpholine**

**Formule (XII)** : $R_1$ = n-Propyl, $R_2$ = Méthyl, V = $NH_2$

$XR_3$ = 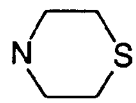

Préparé selon le mode opératoire de l'exemple 12. Huile utilisée telle quelle pour la suite.

Exemple 48 : **[n-Propyl-6 méthyl-2 (amino-4 benzyl)-5 pyrimidine-yl-4]-4 (méthoxy-2 phényl)-1 pipérazine**

**Formule (XII) :** $R_1$ = n-Propyl, $R_2$ = Méthyl, V = $NH_2$

$XR_3$ = 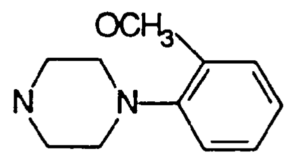

Préparé selon le mode opératoire de l'exemple 12. Huile utilisée telle quelle pour la suite.

Exemple 49 : **[n-Butyl-6 méthyl-2 (amino-4 benzyl)-5 pyrimidine-yl-4] mercapto acétate d'éthyle**

**Formule (XII)** : $R_1$ = n-Butyl, $R_2$ = méthyl , V = $NH_2$, $R_3$ = $CH_2CO_2Et$, X = S

Préparé selon le mode opératoire de l'exemple 12. Huile utilisée telle quelle pour la suite.

Exemple 50 : **[n-Butyl-6 méthyl-2 (amino-4 benzyl)-5 pyrimidine-yl-4] mercapto-2 éthanol**

**Formule (XII)** : $R_1$ = n-Butyl, $R_2$ = Méthyl, V = $NH_2$, $R_3$ = $CH_2CH_2OH$, X = S

Préparé selon le mode opératoire de l'exemple 12. Huile utilisée telle quelle pour la suite.

Exemple 51 : **n-Propyl-6 méthyl-2 thiométhyl-4 (amino-4 benzyl)-5 pyrimidine**

**Formule (XII)** : $R_1$ = n-Propyl, $R_2$ = Méthyl, V = $NH_2$, $XR_3$ = $SCH_3$

Préparé selon le mode opératoire de l'exemple 12. Huile utilisée telle quelle pour la suite.

Exemple 52 : **n-Butyl-6 méthyl-2 amino-4 (amino-4 benzyl)-5 pyrimidine**

**Formule (XII)** : $R_1$ = n-Butyl, $R_2$ = Méthyl, V = $NH_2$, $XR_3$ = $NH_2$

Préparé selon le mode opératoire de l'exemple 12. Huile utilisée telle quelle pour la suite.

Exemple 53 : **n-Propyl-6 méthyl-2 amino-4 (amino-4 benzyl)-5 pyrimidine**

**Formule (XII)** : $R_1$ = n-Propyl, $R_2$ = Méthyl, V = $NH_2$, $XR_3$ = $NH_2$

Préparé selon le mode opératoire de l'exemple 12. Huile utilisée telle quelle pour la suite.

Exemple 54 : **Acide [(n-Propyl-6 méthyl-2 hydroxyéthylmercapto-4 pyrimidine-yl-5) methyl phenyl-yl-4] amino carbonyl-2 benzène sulfonique**

**Formule (I)** : $R_1$ = n-Propyl, $R_2$ = Méthyl, $R_3$ = $CH_2CH_2OH$

X = S, $R_4$ = 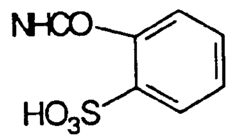

Préparé selon le mode opératoire de l'exemple 13.
Cristaux de point de fusion 226-8°C.

Exemple 55 : **Acide [(n-butyl-6 méthyl-2 hydroxyéthylmercapto-4 pyrimidine-yl-5) méthyl phényl-yl-4] amino carbonyl-2 benzène sulfonique**

**Formule (I)** : $R_1$ = n-Butyl, $R_2$ = Méthyl, $R_3$ = $CH_2CH_2OH$

X = S, $R_4$ = 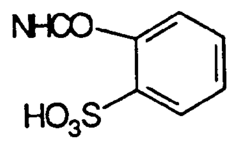

Préparé selon le mode opératoire de l'exemple 13.
Cristaux de point de fusion 198-9°C.

Exemple 56 : **Acide [[n-Propyl-6 méthyl-2 (morpholine-yl-1 )-4 pyrimidine-yl-5] méthyl phényl-yl-4] amino carbonyl-2 benzène sulfonique**

**Formule (I)** : $R_1$ = n-Propyl, $R_2$ = Méthyl,

$XR_3$ = 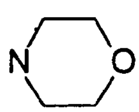, $R_4$ = 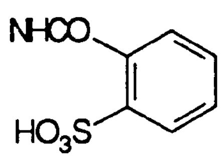

Préparé selon le mode opératoire de l'exemple 13.
Cristaux de point de fusion 213-6°C.

Exemple 57 : **Acide [(n-Butyl-6 méthyl-2 amino-4 pyrimidine-yl-5) méthyl phényl-yl-4] amino carbonyl-2 benzène sulfonique**

**Formule (I) :** $R_1$ = n-Butyl, $R_2$ = Méthyl, $XR_3$ = $NH_2$

$R_4$ = 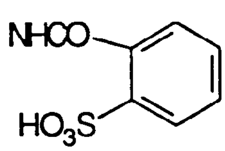

Préparé selon le mode opératoire de l'exemple 13.
Cristaux de point de fusion 239-240°C.

Exemple 58 : **Acide [[n-Propyl-6 méthyl-2 ((méthoxy-2 phényl) pipérazine-yl-4)-4 pyrimidine-yl-5] méthyl phényl-yl-4] amino carbonyl-2 benzène sulfonique**

**Formule (I)** : $R_1$ = n-Propyl, $R_2$ = Méthyl,

$XR_3$ = 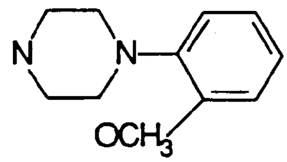 , $R_4$ = 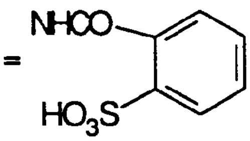

Préparé selon le mode opératoire de l'exemple 13.
Cristaux de point de fusion 215-7°C.

Exemple 59 : **Acide [[n-Propyl-6 méthyl-2 méthylthio-4 pyrimidine-yl-5] méthyl phényl-yl-4] amino carbonyl-2 benzène sulfonique**

**Formule (I)** : $R_1$ = n-Propyl, $R_2$ = Méthyl, $XR_3$ = $SCH_3$,

$R_4$ = 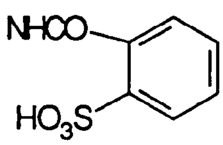

Préparé selon le mode opératoire de l'exemple 13.
Cristaux de point de fusion 254-7°C.

Exemple 60 : **Acide [[n-Propyl-6 méthyl-2 (thiomorpholine-yl-4)-4 pyrimidine-yl-5] méthyl phényl-yl-4] amino carbonyl-2 benzène sulfonique**

**Formule (I)** : $R_1$ = n-Propyl, $R_2$ = Méthyl,

$XR_3$ = 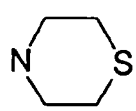 , $R_4$ = 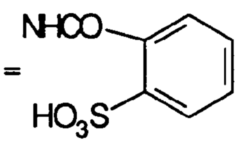

Préparé selon le mode opératoire de l'exemple 13.
Cristaux de point de fusion 207-210°C.

Exemple 61 : **Acide [[n-Butyl-6 Méthyl-2 (éthoxycarbonyl méthyl thio)-4 pyrimidine-yl-5] méthylphényl-yl-4] amino carbonyl-2 benzène sulfonique**

**Formule (I)** : $R_1$ = n-Butyl, $R_2$ = Méthyl, X = S,

$R_3 = CH_2CO_2Et$ , $R_4 =$ 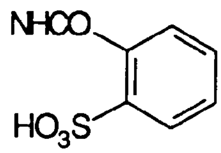

Préparé selon le mode opératoire de l'exemple 13.
Cristaux de point de fusion 262-3°C.

Exemple 62 : **Acide [[n-Propyl-6 Méthyl-2 (éthoxycarbonyl méthylthio)-4 pyrimidine-yl-5] méthyl phényl-yl-4] amino carbonyl-2 méthyl-3 benzène sulfonique**

**Formule (I)** : $R_1$ = n-Propyl, $R_2$ = Méthyl, X = S,

$R_3 = CH_2CO_2Et$ , $R_4 =$ 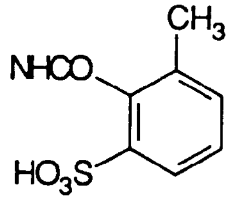

Préparé selon le mode opératoire de l'exemple 13, à partir de l'anhydride méthyl-3 sulfobenzoïque.
Cristaux de point de fusion 224-227°C.

Préparation de l'anhydride méthyl-3 sulfobenzoïque :

A) Méthyl-6 anthranylate d'éthyle :

16,2 g d'acide méthyl-6 anthranylique sont dissous dans 150 ml de tétrahydrofurane. 17,5 g de N,N carbonyl diimidazole sont ajoutés et le mélange est agité une heure à température ambiante puis 6 heures au reflux. Le mélange est concentré sous vide et 200 ml d'éthanol sont ajoutés et le mélange est porté au reflux pendant 6 heures. Après évaporation du solvant sous vide, le résidu est repris à l'éther isopropylique, les cristaux sont essorés et le filtrat est passé au noir puis évaporé pour donner 9,5 g de méthyl-6 anthranylate d'éthyle sous forme d'huile utilisée telle quelle pour la suite.

B) Chlorure de l'acide méthyl-3 éthoxycarbonyl-2 benzène sulfonique :

10,4 g de méthyl-6 anthranylate d'éthyle sont ajoutés à - 5°C dans 40 ml d'acide chlorhydrique concentré. Une solution de 4,4 g de nitrite de sodium dans 10 ml d'eau est ajoutée goutte à goutte à -5°C et le mélange est agité 30 minutes à cette température.

D'un autre côté 8 g de dioxyde de soufre sont dissous dans 50 ml d'acide acétique et 1,9 g de chlorure de cuivre (II) sont ajoutés. La solution obtenue est additionnée goutte à goutte dans le mélange réactionnel toujours à -5°C. Après avoir été agité une heure à 20°C, puis une heure à 40°C le mélange est versé sur de la glace et extrait à l'éther. La phase organique est séchée sur sulfate de magnésium pour donner 6,7 g de chlorure de l'acide méthyl-3 éthoxy carbonyl-2 benzène sulfonique sous forme d'une huile utilisée telle quelle pour la suite.

C) Anhydride méthyl-3 sulfobenzoïque :

6,7 g de chlorure de l'acide méthyl-3 éthoxycarbonyl-2 benzène sulfonique sont chauffés 3 heures au reflux dans 60 ml d'eau distillée et 5 ml d'acide chlorhydrique concentré. La solution obtenue est filtrée sur charbon actif puis évaporée à sec. Le résidu obtenu est chauffé 3 heures au reflux dans 50 ml d'anhydride acétique. La solution est évaporée à sec sous vide et le résidu obtenu cristallise dans un mélange éther/pentane. Les cristaux obtenus sont essorés et séchés pour donner 3,2 g d'anhydride méthyl-3 sulfobenzoïque sous forme de cristaux de point de fusion 130°C.

Exemple 63 : **n-Butyl-6 isopropyl-2 hydroxy-4 (nitro-4 benzyl)-5 pyrimidine**

**Formule (XI)** : $R_1$ = n-Butyl, $R_2$ = Isopropyl, T = OH, V = $NO_2$

2,8 g de sodium sont dissous dans 150 ml d'éthanol et la solution est refroidie à 5°C 15,1 g de chlorhydrate d'isobutyramidine sont ajoutés et le mélange est agité pendant quinze minutes 25 g de (nitro-4 benzyl)-2 oxo-3 heptanoate d'éthyle préparé à l'exemple 4 sont ajoutés et le mélange est agité pendant 3 heures à température ambiante puis versé dans de l'eau et acidifié par l'acide chlorhydrique concentrée. Les cristaux obtenus sont essorés, lavés à l'eau puis à l'éther isopropylique et à l'acétone pour donner 16,1 g de n-butyl-6 isopropyl-2 hydroxy-4 (nitro-4 benzyl)-5 pyrimidine sous forme de cristaux de point de fusion 143°C.

Exemple 64 : **n-Butyl-6 phényl-2 hydroxy-4 (nitro-4 benzyl)-5 pyrimidine**

**Formule (XI)** : $R_1$ = n-Butyl, $R_2$ = Phényl, T = OH, V = $NO_2$

Préparé selon le mode opératoire de l'exemple 63, à partir du chlorhydrate de benzamidine.

Cristaux de point de fusion 270°C.

Exemple 65 : **n-Butyl-6 isopropyl-2 chloro-4 (nitro-4 benzyl)-5 pyrimidine**

**Formule (XII)** : $R_1$ = n-Butyl, $R_2$ = isopropyl, $XR_3$ = Cl, V = $NO_2$

Préparé selon le mode opératoire de l'exemple 19.

Huile utilisée telle quelle pour la suite.

Exemple 66 : **n-Butyl-6 phényl-2 chloro-4 (nitro-4 benzyl)-5 pyrimidine**

**Formule (XII)** : $R_1$ = n-Butyl, $R_2$ = phényl, $XR_3$ = Cl, V = $NO_2$

Préparé selon le mode opératoire de l'exemple 19.

Cristaux de point de fusion 85°C.

Exemple 67 : **[n-Butyl-6 phényl-2 (nitro-4 benzyl)-5 pyrimidine-yl-4] mercapto acétate d'éthyle**

**Formule (XII)** : $R_1$ = n-Butyl, $R_2$ = Phényl, X = S, $R_3$ = $CH_2CO_2Et$, V = $NO_2$

Préparé selon le mode opératoire de l'exemple 22.

Cristaux de point de fusion 102°C.

Exemple 68 : **[n-Butyl-6 isopropyl-2 (nitro-4 benzyl)-5 pyrimidine-yl-4] mercapto acétate d'éthyle**

**Formule (XII)** : $R_1$ = n-Butyl, $R_2$ = isopropyl, X = S, $R_3$ = $CH_2CO_2Et$, V = $NO_2$

Préparé selon le mode opératoire de l'exemple 22.

Huile utilisée telle quelle pour la suite.

Exemple 69 : **[n-Butyl-6 phényl-2 (nitro-4 benzyl)-5 pyrimidine-yl-4] mercapto-2 éthanol**

**Formule (XII)** : $R_1$ = n-Butyl, $R_2$ = phényl, X = S, $R_3$ = $CH_2CH_2OH$, V = $NO_2$

Préparé selon le mode opératoire de l'exemple 34.

Cristaux de point de fusion 98°C.

Exemple 70 : **[n-Butyl-6 isopropyl-2 (nitro-4 benzyl)-5 pyrimidine-yl-4] mercapto-2 éthanol**

**Formule (XII)** : $R_1$ = n-Butyl, $R_2$ = isopropyl, X = S, $R_3$ = $CH_2CH_2OH$, V = $NO_2$

Préparé selon le mode opératoire de l'exemple 34.

Huile utilisée telle quelle pour la suite.

Selon le mode opératoire de l'exemple 12 ont été préparés :

Exemple 71 : **[n-Butyl-6 phényl-2 (amino-4 benzyl)-5 pyrimidine-yl-4] mercapto acétate d'éthyle**

**Formule (XII)** : $R_1$ = n-Butyl, $R_2$ = phényl, X = S, $R_3$ = $CH_2CO_2Et$, V = $NH_2$

Huile utilisée telle quelle pour la suite.

Exemple 72 : **[n-Butyl-6 isopropyl-2 (amino-4 benzyl)-5 pyrimidine-yl-4] mercapto acétate d'éthyle**

**Formule (XII)** : $R_1$ = n-Butyl, $R_2$ = isopropyl, X = S, $R_3$ = $CH_2CO_2Et$, V = $NH_2$
Huile utilisée telle quelle pour la suite.

Exemple 73 : **[n-Butyl-6 phényl-2 (amino-4 benzyl)-5 pyrimidine-yl-4] mercapto-2 éthanol**

**Formule (XII)** : $R_1$ = n-Butyl, $R_2$ = phényl, X = S, $R_3$ = $CH_2CH_2OH$, V = $NH_2$
Cristaux de point de fusion 103°C.

Exemple 74 : **[n-Butyl-6 isopropyl-2 (amino-4 benzyl)-5 pyrimidine-yl-4] mercapto-2 éthanol**

**Formule (XII)** : $R_1$ = n-Butyl, $R_2$ = isopropyl, X = S, $R_3$ = $CH_2CH_2OH$, V = $NH_2$
Huile utilisée telle quelle pour la suite.

Selon le mode opératoire de l'exemple 13, les composés suivants ont été préparés :

Exemple 75 : **Acide [[n-butyl-6 phényl-2 (éthoxy carbonyl méthyl thio)-4 pyrimidine-yl-5] méthyl phényl-yl-4] amino carbonyl-2 benzène sulfonique**

**Formule (I)** : $R_1$ = n-Butyl, $R_2$ = phényl, X = S,

$R_3 = CH_2CO_2Et$ , $R_4$ =

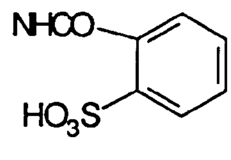

Cristaux de point de fusion 241-242°C.

Exemple 76 : **Acide [[n-butyl-6 isopropyl-2 (éthoxy carbonyl méthyl thio)-4 pyrimidine-yl-5] méthyl phényl-yl-4] amino carbonyl-2 benzène sulfonique**

**Formule (I)** : $R_1$ = n-Butyl, $R_2$ = isopropyl, X = S,

$R_3 = CH_2CO_2Et$ , $R_4$ =

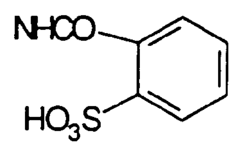

Cristaux de point de fusion 227-229°C.

Exemple 77 : **Acide [[n-butyl-6 phényl-2 (hydroxy éthyl thio)-4 pyrimidine-yl-5] méthyl phényl-yl-4] amino carbonyl-2 benzène sulfonique**

**Formule (I)** : $R_1$ = n-Butyl, $R_2$ = phényl, X = S,

$R_3 = CH_2CH_2OH$ , $R_4$ =

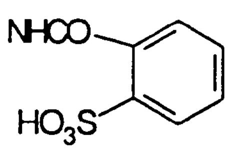

Cristaux de point de fusion 220-221 °C.

Exemple 78 : **Acide [[n-butyl-6 isopropyl-2 (hydroxy éthyl thio)-4 pyrimidine-yl-5] méthyl phényl-yl-4] amino carbonyl-2 benzène sulfonique**

**Formule (I) :** $R_1$ = n-Butyl, $R_2$ = isopropyl, X = S,

$R_3 = CH_2CH_2OH$ , $R_4$ = 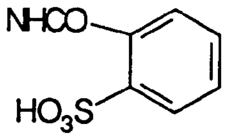

Cristaux de point de fusion 240-241°C.

Exemple 79 : **Méthoxy-1 oxo-4 heptanone-2**

**Formule (IV)**: $R_1$ = n-Propyl, s = 1, U = O, $R_{14} = CH_3$

Une solution de 127,5 ml de méthoxy acétate de méthyle et 214,5 ml de pentanone-2 dans 100 ml de toluène est ajoutée goutte à goutte sur 33,5 g de sodium divisé à chaud dans 600 ml de toluène, la température étant maintenue entre 60°C et 70°C. Après la fin de l'addition, le mélange est chauffé 2 heures à 55-60°C, puis 30 ml de méthanol sont ajoutés goutte à goutte et le mélange obtenu est repris à l'eau, lavé à l'éther et la phase aqueuse est acidifiée par l'acide chlorhydrique concentré, puis extraite à l'éther. La phase organique est séchée sur sulfate de magnésium et évaporée sous vide. Le résidu huileux est distillé sous pression réduite pour donner 103,8 g de méthoxy-1 oxo-4 heptanone-2 de point d'ébullition Eb = 100-110°C.

Exemple 80 : **(acétamido-4 benzylidène)-3 méthoxy-1 oxo-4 heptanone-2**

**Formule (IV')** : $R_1$ = n-Propyl, s = 1, U = O, $R_{14}$ = méthyl, V = NH $COCH_3$

63,2 g de méthoxy-1 oxo-4 heptanone-2 et 65, 2 g d'acétamido-4 benzaldéhyde sont dissous dans 480 ml de toluène en présence de 20 ml d'acide acétique et 4 ml de pipéridine. Le mélange est porté au reflux pendant 5 heures et 10,5 ml d'eau sont éliminés par l'intermédiaire d'un Dean Starck. Les cristaux obtenus après refroidissement sont essorés et lavés à l'eau, au dichlorométhane et à l'isopropanol, puis recristallisés dans 200 ml de méthanol pour donner 43 g d'(acétamido-4 benzylidène)-3 méthoxy-1 oxo-4 heptanone-2 sous forme de cristaux de point de fusion 160°C.

Exemple 81 : **n-Propyl-6 phényl-2 méthoxy méthyl-4 (acétamido-4 benzyl)-5 pyrimidine**

**Formule (XII)** : $R_1$ = n-Propyl, $R_2$ = Phényl, $XR_3 = CH_2OCH_3$, V = $NHCOCH_3$

42 g d'(acétamido-4 benzylidène)-3 méthoxy-1 oxo-4 heptanone-2, préparés à l'exemple 80, sont dissous dans 1250 ml d'acide acétique. 128,7 g d'acétate d'ammonium sec sont ajoutés et le mélange est chauffé à 60-70°C pendant 10 minutes. 30 ml de benzaldéhyde sont ajoutés à 60°C et le mélange est agité 2 heures à cette température. 500 ml de benzène sont ajoutés et le mélange est alors chauffé au reflux, 9,5 ml d'eau étant éliminés par l'intermédiaire d'un Dean Starck. Les solvants sont concentrés sous vide et le résidu, repris à l'eau, est extrait à l'acétate d'éthyle. La phase organique est lavée à l'eau, séchée sur sulfate de magnésium et évaporée sous vide. Le résidu obtenu est chromatographié sur gel de silice dans un éluant chloroforme 95 / acétone 5 pour donner 3,5 g de n-Propyl-6 méthoxy méthyl-4 phényl-2 (acétamido-4 benzyl)-5 pyrimidine sous forme de cristaux de point de fusion 135°C.

Exemple 82 : **n-Propyl-6 phényl-2 méthoxy méthyl-4 (amino-4 benzyl)-5 pyrimidine**

**Formule (XII)** : $R_1$ = n-Propyl, $R_2$ = phényl, $XR_3 = CH_2OCH_3$ V = $NH_2$

1,8 g de n-Propyl-6 phényl-2 méthoxy méthyl-4 (acétamido-4 benzyl)-5 pyrimidine préparés à l'exemple 81, sont dissous dans un mélange composé de 25 ml d'éthanol et 25 ml d'eau. 2 g de soude en pastille sont ajoutés et le mélange est chauffé 15 heures au reflux, puis repris à l'eau et extrait à l'éther. La phase organique est séchée puis évaporée sous vide. Le résidu obtenu est chromatographié sur gel de silice avec l'éther comme éluant pour donner 1,2 g de n-Propyl-6 phényl-2 méthoxy méthyl-4 (amino-4 benzyl)-5 pyrimidine sous forme de cristaux de point de fusion 115°C.

Exemple 83 : **Acide [[n-Propyl-6 méthoxy méthyl-4 phényl-2 pyrimidine-yl-5] méthyl phényl-yl-4] amino carbonyl-2 benzène sulfonique**

**Formule (I)** : $R_1$ = n-Propyl, $R_2$ = phényl, $XR_3 = CH_2\ O\ CH_3$,

$R_4$ = 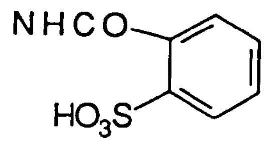

Préparé selon le mode opératoire de l'exemple 13.
Cristaux de point de fusion 186-187°C.

Exemple 84 : **Acide [(n-Propyl-6 méthyl-2 amino-4 pyrimidine -yl-5) méthyl phényl-yl-4] amino carbonyl-2 benzène sulfonique**

**Formule (I)** : $R_1$ = n-Propyl, $R_2$ =méthyl, $XR_3 = NH_2$

$R_4$ = 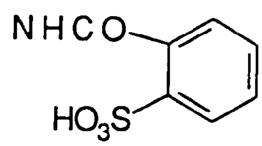

Préparé selon le mode opératoire de l'exemple 13.
Cristaux de point de fusion 310-312°C.

Exemple 85 : **N-[n-Butyl-6 méthyl-2 (amino-4 benzyl)-5 pyrimidine-yl-4] morpholine**

**Formule (XII)** : $R_1$ = n-Butyl, $R_2$ = Méthyl, $V = NH_2$ ,

$XR_3$ = 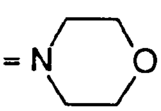

Préparé selon le mode opératoire de l'exemple 12.
Huile utilisée telle quelle pour la suite.

Exemple 86 : **Acide [(n-Butyl-6 méthyl-2 (morpholine-yl-1 )-4 pyrimidine-yl-5) méthyl phényl-yl-4] amino carbonyl-2 benzène sulfonique**

**Formule (I) :** $R_1$ = n-Butyl, $R_2$ = Méthyl,

$XR_3$ = 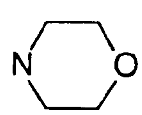 , $R_4$ = 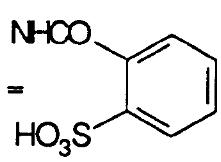

Préparé selon le mode opératoire de l'exemple 13.
Cristaux de point de fusion 213-6°C.

Exemple 87: **n-Propyl-6 méthyl-2 hydroxy-4 [(tétrazolyl-5)-2′ biphényl-yl-4] méthyl-5 pyrimidine**

**Formule (I)** : $R_1$ = n-Propyl, $R_2$ = Méthyl, $XR_3$ = OH

$R_4$ =

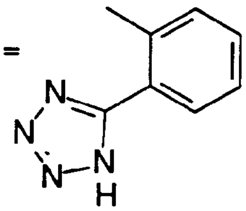

Préparé selon le mode opératoire de l'exemple 31 à partir du n-Propyl-6 méthyl-2 hydroxy-4 (cyano-2′biphényl-yl-4) méthyl-5 pyrimidine préparé à l'exemple 10.

Cristaux de point de fusion 204-6°C.

Exemple 88 : **n-Propyl-6 méthyl-2 (hydroxy-2 éthyl) oxy-4 (cyano-2′ biphényl-yl-4) méthyl-5 pyrimidine**

**Formule (XII)** : $R_1$ = n-Propyl, $R_2$ = Méthyl, X = O,

$R_3 = CH_2CH_2OH$ , V =

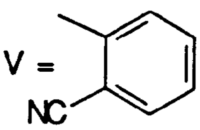

8 g de n-Propyl-6 méthyl-2 hydroxy-4 (cyano-2′biphényl-yl-4) méthyl-5 pyrimidine préparé à l'exemple 10 sont dissous dans 80 ml d'acétonitrile en présence de 4 g de carbonate de potassium et 3 ml de bromo-2 éthanol sont ajoutés. Le mélange est porté au reflux et chauffé pendant 8 heures ; les solvants sont concentrés sous vide et le résidu est repris à l'eau puis extrait au dichlorométhane. La phase organique est séchée sur sulfate de magnésium et évaporée sous vide pour donner un résidu huileux qui est chromatographié sur gel de silice. L'élution avec un mélange dichlorométhane / acétone 80/20 permet d'obtenir pur 2,1 g de n-Propyl-6 méthyl-2 (hydroxy-2 éthyl) oxy-4 (cyano-2′biphényl-yl-4) méthyl-5 pyrimidine sous forme de cristaux de point de fusion 103°C.

En poursuivant l'élution avec un mélange dichlorométhane / acétone 60/40 on récupère 2,8 g de n-Propyl-6 méthyl-2 (cyano-2′biphényl-yl-4) méthyl-5 (hydroxy-2 éthyl)-3 dihydro-3,4 oxo-4 pyrimidine sous forme d'une huile utilisée telle quelle pour la suite.

Exemple 89 : **n-Propyl-6 méthyl-2 (hydroxy-2 éthyl) oxy-4 [(tetrazolyl-5)-2′ biphényl-yl-4] méthyl-5 pyrimidine**

**Formule (I)** : $R_1$ = n-Propyl, $R_2$ = Méthyl, X = O,

$R_3 = CH_2CH_2OH$ , $R_4$ =

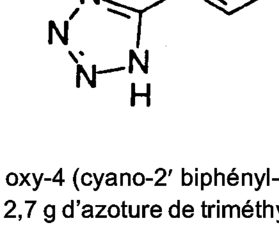

3,8 g de n-Propyl-6 méthyl-2 (hydroxy-2 éthyl) oxy-4 (cyano-2′ biphényl-yl-4) méthyl-5 pyrimidine préparé à l'exemple 88 sont dissous dans 80 ml de toluène. 2,7 g d'azoture de triméthyl étain sont ajoutés et le mélange est chauffé 35 heures au reflux, refroidi et extrait avec une solution de potasse diluée qui est lavée à l'acétate d'éthyle. La phase aqueuse est ensuite acifiﬁée par barbotage de dioxyde de soufre et extraite au chloroforme. Après séchage et évaporation du solvant, le résidu obtenu est chromatographié sur gel de silice dans un éluant

chloroforme / éthanol 85/15 pour donner 2 g de n-Propyl-6 méthyl-2 (hydroxy-2 éthyl) oxy-4 [(tétrazolyl-5)-2′biphényl-yl-4] méthyl-5 pyrimidine sous forme de cristaux de point de fusion 154-155°C.

Exemple 90 : **Chlorhydrate de n-Propyl-6 méthyl-2 (hydroxy-2 éthyl)-3 [(tétrazolyl-5)-2′ biphényl-yl-4] méthyl-5 dihydro-3,4- oxo-4 pyrimidine**

**Formule (I')** : $R_1$ = n-Propyl, $R_2$ = Méthyl, $R'_3$= $CH_2CH_2OH$,

$R_4$ = 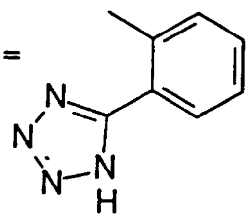

Préparé selon le mode opératoire de l'exemple 89 à partir de la n-Propyl-6 méthyl-2 (hydroxy-2 éthyl)-3 (cyano-2′biphényl-yl-4) méthyl-5 dihydro-3,4- oxo-4 pyrimidine, préparée à l'exemple 88. Le chlorhydrate est obtenu par barbottage d'acide chlorhydrique dans une solution du composé sous forme de base dans du tétrahydrofurane.

Cristaux de point de fusion 240-1°C.

Exemple 91 : **n-Propyl-6 méthyl-2 (propyl-2) oxy-4 (cyano-2′ biphényl-yl-4) méthyl-5 pyrimidine**

**Formule (XII)** : $R_1$ = n-Propyl, $R_2$ = Méthyl,

$XR_3$ = 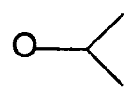 , V = 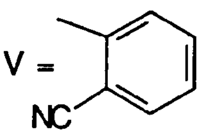

Préparé selon le mode opératoire de l'exemple 30.

Huile utilisée telle quelle pour la suite.

Exemple 92 : **n-Propyl-6 méthyl-2 (propyl-2) oxy-4 [(tétrazolyl-5) -2′ biphényl-yl-4] méthyl-5 pyrimidine**

**Formule (I)** : $R_1$ = n-Propyl, $R_2$ = Méthyl,

$XR_3$ = 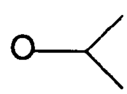 , $R_4$ = 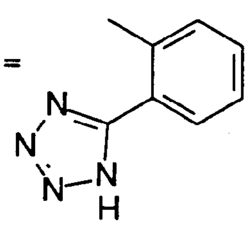

Préparé selon le mode opératoire de l'exemple 89.

Cristaux de point de fusion 190-1°C.

Exemple 93 : **N-[n-Propyl-6 méthyl-2 (cyano-2′ biphenyl-yl-4) méthyl-5 pyrimidine-yl-4] thiomorpholine**

**Formule (XII)** : $R_1$ = n-Propyl, $R_2$ = Méthyl,

$XR_3$ = 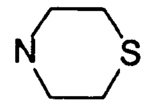 , V = 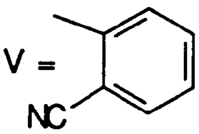

Préparé selon le mode opératoire de l'exemple 39.
Cristaux de point de fusion 153-4°C.

Exemple 94 : **Chlorhydrate de N-[n-Propyl-6 méthyl-2 [(tétrazolyl-5)-2′ bi phényl-yl-4) méthyl-5 pyrimidine-yl-4] thiomorpholine**

**Formule (I)** : $R_1$ = n-Propyl, $R_2$ = Méthyl,

$XR_3$ = 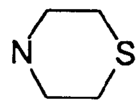 , $R_4$ = 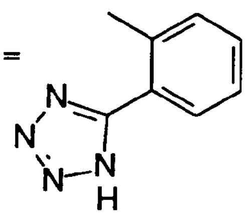

Préparé selon le mode opératoire de l'exemple 89. Le chlorhydrate est obtenu par dissolution du composé sous forme de base dans un mélange acétone éther et barbottage d'acide chlorhydrique.
Cristaux de point de fusion 240-241°C.

Exemple 95 : **n-Propyl-6 méthyl-2 mercapto-4 (cyano-2′biphényl-yl-4)-méthyl-5 pyrimidine**

**Formule (XII)** : $R_1$ = n-Propyl, $R_2$ = Méthyl, $XR_3$ = SH,

V = 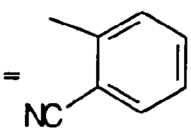

Préparé selon le mode opératoire de l'exemple 43.
Cristaux de point de fusion 202-3°C.

Exemple 96 : **n-Propyl-6 méthyl-2 mercapto-4 [(tétrazolyl-5)-2′ biphényl-yl-4] méthyl-5 pyrimidine**

**Formule (I)** : $R_1$ = n-Propyl, $R_2$ = Méthyl, $XR_3$ = SH,

$R_4$ = 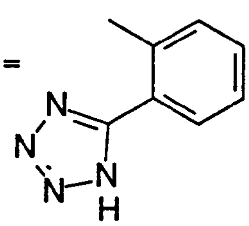

Préparé selon le mode opératoire de l'exemple 89.

Cristaux de point de fusion 247-248°C.

<u>Exemple 97</u> : **n-Propyl-6 méthyl-2 méthylthio-4 (cyano-2′ biphényl-yl-4) méthyl-5 pyrimidine**

**Formule (XII)** : $R_1$ = n-Propyl, $R_2$ = Méthyl, $XR_3$ = $SCH_3$,

V =

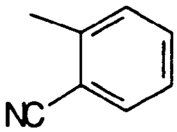

Préparé selon le mode opératoire de l'exemple 44.
Cristaux de point de fusion 134°C.

<u>Exemple 98</u> : **n-Propyl-6 méthyl-2 méthylthio-4 [(tétrazolyl-5)-2′ biphényl-yl-4] méthyl-5 pyrimidine**

**Formule (I)** : $R_1$ = n-Propyl, $R_2$ = Méthyl, $XR_3$ = $SCH_3$,

$R_4$ =

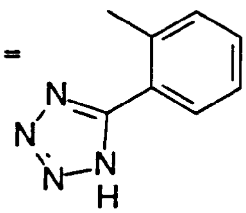

Préparé selon le mode opératoire de l'exemple 89.
Cristaux de point de fusion 173-174°C.

<u>Exemple 99</u> : **n-Propyl-6 méthyl-2 (hydroxy-2 éthyl) mercapto-4 (cyano-2′ biphényl-yl-4) méthyl-5 pyrimidine**

**Formule (XII)** : $R_1$ = n-Propyl, $R_2$ = Méthyl, X = S,

$R_3 = CH_2CH_2OH$ , V =

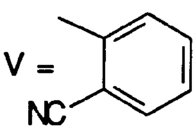

Préparé selon le mode opératoire de l'exemple 34.
Cristaux de point de fusion 97°C.

<u>Exemple 100</u> : **n-Propyl-6 méthyl-2 (acétoxy-2 éthyl) mercapto-4 (cyano-2′ biphényl-yl-4) méthyl-5 pyrimidine**

**Formule (XII)** : $R_1$ = n-Propyl, $R_2$ = Méthyl, X = S,

$R_3 = CH_2CH_2\,O\,CO\,CH_3$, V =

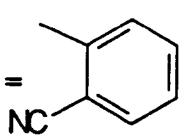

6,7 g de n-Propyl-6 méthyl-2 (hydroxy-2 éthyl) mercapto-4 (cyano-2′ biphényl-yl-4) méthyl-5 pyrimidine préparé à l'exemple 99 sont chauffés au reflux dans 35 ml d'anhydride acétique, pendant 3 heures. L'anhydride acétique est évaporée sous vide et le résidu est repris à l'éther, la solution éthérée est filtrée sur noir animal puis évaporée sous vide pour donner 5,9 g de n-Propyl-6 méthyl-2 (acétoxy-2 éthyl) mercapto-4 (cyano-2'biphényl-yl-4) méthyl-5 pyrimidine sous forme d'une huile utilisée telle quelle pour la suite.

Exemple 101 : **n-Propyl-6 méthyl-2 (hydroxy-2 éthyl) mercapto-4 [(tétrazolyl-5)-2' biphényl-yl-4] méthyl-5 pyrimidine**

**Formule (I)** : $R_1$ = n-Propyl, $R_2$ = Méthyl, X = S,

$R_3 = CH_2CH_2OH$, $R_4$ = 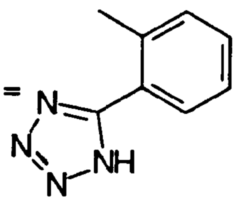

5,9 g de n-Propyl-6 méthyl-2 (acétoxy-2 éthyl) mercapto-4 (cyano-2' biphényl-yl-4) méthyl-5 pyrimidine préparé à l'exemple 100, sont dissous dans 100 ml de toluène et 3,7 g d'azoture de triméthyl étain sont ajoutés. Le mélange est chauffé 12 heures au reflux, refroidi, repris à l'éther et extrait avec une solution de potasse diluée. La phase aqueuse est acidifiée par barbottage de dioxyde de soufre puis extraite au chloroforme. La phase chloroformique est séchée sur sulfate de magnésium et évaporée sous vide ; le résidu obtenu est chromatographié sur gel de silice avec un éluant chloroforme / méthanol 85/15 pour donner 2,5 g d'un résidu composé d'un mélange d'alcool attendu et de dérivé acétylé. Ce mélange est repris dans une solution de potasse diluée et laissé une nuit à température ambiante. La solution aqueuse est lavée à l'éther puis acidifiée par barbottage de dioxyde de soufre et les cristaux obtenus sont essorés et lavés à l'éther pour donner 2,3 g de n-Propyl-6 méthyl-2 (hydroxy-2 éthyl) mercapto-4 [(tétrazolyl-5)-2'biphényl-yl-4] méthyl-5 pyrimidine sous forme de cristaux de point de fusion 186-189°C.

Exemple 102 : **(Cyano-2' biphényl-yl-4) méthyl-2 oxo-3 heptanoate d'éthyle**

**Formule (VI)** : $R_1$ = n-Butyl, $R_3$ = Ethyl,

V = 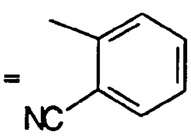

Préparé selon le mode opératoire de l'exemple 6.
Huile utilisée telle quelle pour la suite.

Exemple 103 : **n-Butyl-6 méthyl-2 hydroxy-4 (cyano-2' biphényl -yl-4) méthyl-5 pyrimidine**

**Formule (XI)** : $R_1$ = n-Butyl, $R_2$ = Méthyl, T = OH

V = 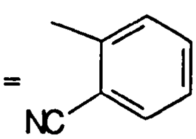

Préparé selon le mode opératoire de l'exemple 7.
Cristaux de point de fusion 173°C.

Exemple 104 : **n-Butyl-6 méthyl-2 chloro-4 (cyano-2′ biphényl -yl-4) méthyl-5 pyrimidine**

**Formule (XII)** : $R_1$ = n-Butyl, $R_2$ = Méthyl, $XR_3$ = Cl

V = 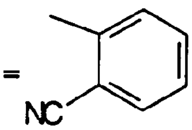

Préparé selon le mode opératoire de l'exemple 19.
Cristaux de point de fusion 75°C.

Exemple 105 : **n-Butyl-6 méthyl-2 méthoxy-4 (cyano-2′biphényl -yl-4) méthyl-5 pyrimidine**

**Formule (XII)** : $R_1$ = n-Butyl, $R_2$ = Méthyl, $XR_3$ = $OCH_3$

V = 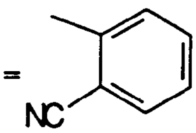

Préparé selon le mode opératoire de l'exemple 30.
Huile utilisée telle quelle pour la suite.

Exemple 106 : **n-Butyl-6 méthyl-2 méthoxy-4 [(tétrazolyl-5)-2′ biphényl-yl-4] méthyl-5 pyrimidine**

**Formule (I)** : $R_1$ = n-Butyl, $R_2$ = Méthyl, $XR_3$ = $OCH_3$

$R_4$ = 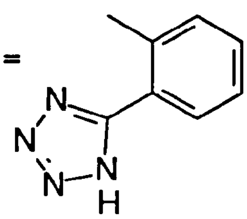

Préparé selon le mode opératoire de l'exemple 89.
Cristaux de point de fusion 148-149°C.

Exemple 107 : **n-Butyl-6 méthyl-2 hydroxy-4 [(tétrazolyl-5)-2′ biphényl-yl-4] méthyl-5 pyrimidine**

**Formule (I)** : $R_1$ = n-Butyl, $R_2$ = Méthyl, $XR_3$ = OH

$R_4$ = 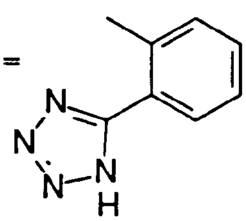

Préparé selon le mode opératoire de l'exemple 31.
Cristaux de point de fusion 248-249°C.

Exemple 108 : **n-Propyl-6 méthyl-2 (méthoxy-2 éthyl) oxy-4 (cyano-2′ biphényl-yl-4) méthyl-5 pyrimidine**

**Formule (XII)** : $R_1$ = n-Propyl, $R_2$ = Méthyl, X = O,

$R_3 = CH_2CH_2\ O\ CH_3$ , V = 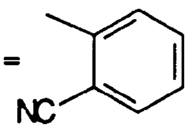

Préparé selon le mode opératoire de l'exemple 30 à partir du méthoxy-2 éthanol.
Huile utilisée telle quelle pour la suite.

Exemple 109 : **n-Propyl-6 méthyl-2 (méthoxy-2 éthyl) oxy-4 [(tétrazolyl-5)-2′ biphényl-yl-4] méthyl-5 pyrimidine**

**Formule (I)** : $R_1$ = n-Propyl, $R_2$ = Méthyl, X = O,

$R_3 = CH_2CH_2\ O\ CH_3$ , $R_4$ = 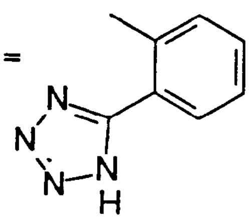

Préparé selon le mode opératoire de l'exemple 89.
Cristaux de point de fusion 169-170°C.

Exemple 110 : **n-Propyl-6 méthyl-2 (diméthylamino-2 éthyl) oxy-4 (cyano-2′ biphényl-yl-4) méthyl-5 pyrimidine**

**Formule (XII)** : $R_1$ = n-Propyl, $R_2$ = Méthyl, X = O,

$R_3 = CH_2CH_2$ 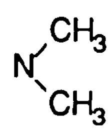 , V = 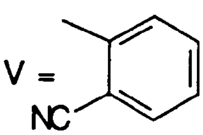

6 g de n-Propyl-6 méthyl-2 (cyano-2′biphényl-yl-4) méthyl-5 chloro-4 pyrimidine préparé à l'exemple 21 sont ajoutés à une solution obtenue par addition de 1 g d'hydrure de sodium à 60 % dans 75 ml de THF contenant 2,7 g de diméthyl amino-2 éthanol. Le mélange est agité 4 heures au reflux, puis le solvant est concentré sous vide, le résidu est repris à l'eau et extrait à l'éther. La phase organique est lavée à l'eau puis séchée sur sulfate de magnésium pour donner 5,1 g de n-Propyl-6 méthyl-2 (cyano-2′ biphényl-yl-4) méthyl-5 (diméthyl amino-2 éthyl) oxy-4 pyrimidine sous forme d'une huile utilisée telle quelle pour la suite.

Exemple 111 : **n-Propyl-6 méthyl-2 (diméthylamino-2 éthyl) oxy-4 [(tétrazolyl-5)-2′ biphényl-yl-4] méthyl-5 pyrimidine**

**Formule (I)** : $R_1$ = n-Propyl, $R_2$ = Méthyl, X = O,

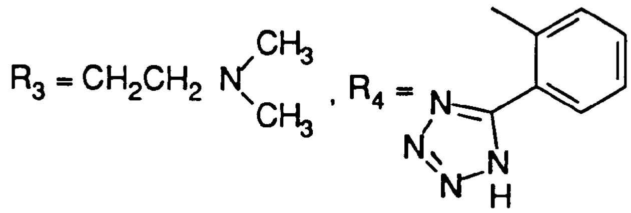

Préparé selon le mode opératoire de l'exemple 89.
Cristaux de point de fusion 175-176°C.

Exemple 112 : **n-Propyl-6 méthyl-2 (morpholino-2 éthyl) amino-4 (cyano-2′ biphényl-yl-4) méthyl-5 pyrimidine**

**Formule (XII)** : $R_1$ = n-Propyl, $R_2$ = Méthyl, X = NH,

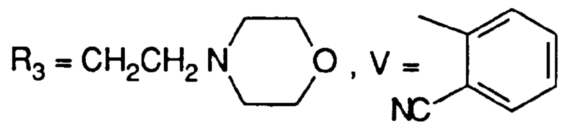

Préparé selon le mode opératoire de l'exemple 39 à partir de la N-(amino-2 éthyl) morpholine.
Huile utilisée telle quelle pour la suite.

Exemple 113 : **n-Propyl-6 méthyl-2 (morpholino-2 éthyl) amino-4 [(tétrazolyl-5)-2′ biphényl-yl-4] méthyl-5 pyrimidine**

**Formule (I)** : $R_1$ = n-Propyl, $R_2$ = Méthyl, X = NH,

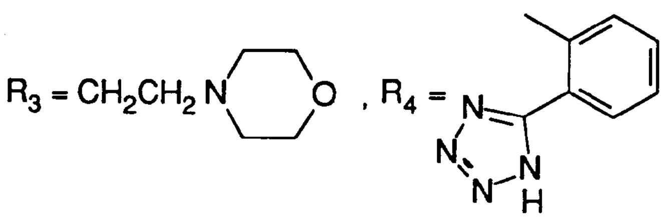

Préparé selon le mode opératoire de l'exemple 89.
Cristaux de point de fusion 270-2°C.

Exemple 114 : **n-Propyl-6 méthyl-2 (cyano-2′ biphényl-yl-4) méthyl-5 (hydroxy-2 éthyl) amino-4 pyrimidine**

**Formule (XII)** : $R_1$ = n-Propyl, $R_2$ = Méthyl, X = NH,

$R_3 = CH_2CH_2OH$ , V = 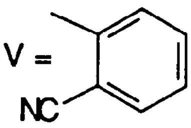

Préparé selon le mode opératoire de l'exemple 39 à partir de l'éthanol amine.
Cristaux de point de fusion 135°C.

Exemple 115 : **n-Propyl-6 méthyl-2 [(tétrazolyl-5)-2′ biphényl -yl-4] méthyl-5 (hydroxy-2 éthyl) amino-4 pyrimidine**

**Formule (I) :** $R_1$ = n-Propyl, $R_2$ = Méthyl, X = NH,

$R_3 = CH_2CH_2OH$, $R_4$ = 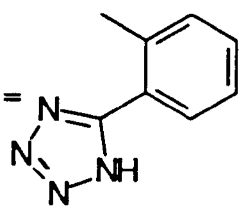

Préparé selon le mode opératoire de l'exemple 89.
Cristaux de point de fusion 149-150°C.

Exemple 116 : **n-Propyl-6 méthyl-2 (méthylthio-2 éthyl) oxy-4 (cyano-2′ biphenyl-yl-4) méthyl-5 pyrimidine**

**Formule (XII)** : $R_1$ = n-Propyl, $R_2$ = Méthyl, X = O,

$R_3 = CH_2CH_2\,S\,CH_3$ , V = 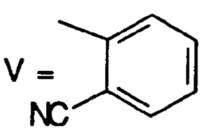

Préparé selon le mode opératoire de l'exemple 110 à partir du méthylthio-2 éthanol.
Huile utilisée telle quelle pour la suite.

Exemple 117 : **n-Propyl-6 méthyl-2 (méthynhio-2 éthyl) oxy-4 [(tétrazolyl-5)-2′ biphényl-yl-4] méthyl-5 pyrimidine**

**Formule (I)** : $R_1$ = n-Propyl, $R_2$ = Méthyl, X = O,

$R_3 = CH_2CH_2\,S\,CH_3$ , $R_4$ = 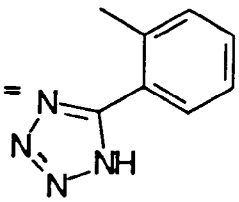

Préparé selon le mode opératoire de l'exemple 89.
Cristaux de point de fusion 121-122°C.

Exemple 118 : **n-Butyl-6 méthyl-2 (méthoxy-2 éthyl) oxy-4 (cyano-2′ biphényl-yl-4) méthyl-5 pyrimidine**

**Formule (XII)** : $R_1$ = n-Butyl, $R_2$ = Méthyl, X = O,

$R_3 = CH_2CH_2\,OCH_3$, V = 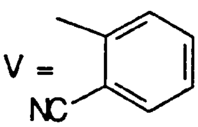

Préparé selon le mode opératoire de l'exemple 30.
Huile utilisée telle quelle pour la suite.

Exemple 119 : **n-Butyl-6 méthyl-2 (méthoxy-2 éthyl) oxy-4 [(tétrazolyl-5)-2′ biphényl-yl-4] méthyl-5 pyrimidine**

**Formule (I)** : $R_1$ = n-Butyl, $R_2$ = Méthyl, X = O,

$R_3 = CH_2CH_2\,OCH_3$ , $R_4$ = 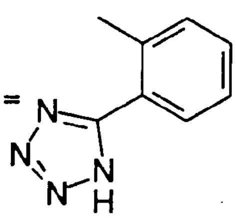

Préparé selon le mode opératoire de l'exemple 89.
Cristaux de point de fusion 128-129°C.

Exemple 120 : **n-Butyl-6 méthyl-2 (diméthyl amino-2 éthyl) oxy-4 (cyano-2′ biphényl-yl-4) méthyl-5 pyrimidine**

**Formule (XII)** : $R_1$ = n-Butyl, $R_2$ = Méthyl, X = O,

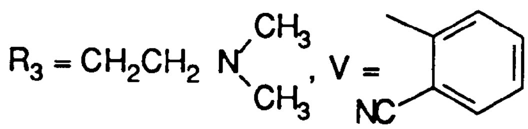

Préparé selon le mode opératoire de l'exemple 110.
Huile utilisée telle quelle pour la suite.

Exemple 121 : **n-Butyl-6 méthyl-2 (diméthyl amino-2 éthyl) oxy-4 [(tétrazolyl-5)-2′ biphényl-yl-4) méthyl-5 pyrimidine**

**Formule (I)** : $R_1$ = n-Butyl, $R_2$ = Méthyl, X = O,

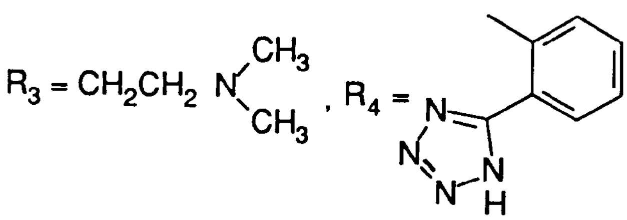

Préparé selon le mode opératoire de l'exemple 89.
Cristaux de point de fusion 166-167°C.

Exemple 122 : **Oxo-3 octanoate d'éthyle**

**Formule (II)** : $R_1$ = n-Pentyl, $R_{13}$ = Ethyl

Préparé selon le mode opératoire de l'exemple 1.
Huile de point d'ébullition $Eb_{15}$ = 110-115°C.

Exemple 123 : **(Cyano-2′ biphényl-yl-4) méthyl-2 oxo-3 octanoate d'éthyle**

**Formule (VI)** : $R_1$ = n-Pentyl, V = 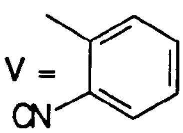 ,

$R_{13}$ = Ethyl

Préparé selon le mode opératoire de l'exemple 3.
Huile utilisée telle quelle pour la suite.

Exemple 124 : **n-Pentyl-6 méthyl-2 hydroxy-4 (cyano-2′ biphényl-yl-4) méthyl-5 pyrimidine**

**Formule (XI)** : $R_1$ = n-Pentyl, $R_2$ = Méthyl, T = OH,

V = 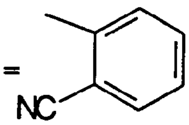

Préparé selon le mode opératoire de l'exemple 7.
Cristaux de point de fusion 162°C.

Exemple 125 : **n-Pentyl-6 méthyl-2 hydroxy-4 [(tétrazolyl-5)-2' biphényl-yl-4] méthyl-5 pyrimidine**

**Formule (I)** : $R_1$ = n-Pentyl, $R_2$ = Méthyl, $XR_3$ = OH,

$R_4$ = 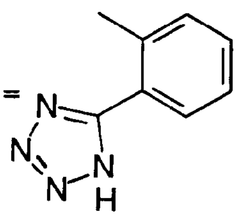

Préparé selon le mode opératoire de l'exemple 31.
Cristaux de point de fusion 195-196°C.

Exemple 126 : **n-Pentyl-6 diméthyl-2,3 (cyano-2' biphényl-yl-4) méthyl-5 dihydro-3,4 oxo-4 pyrimidine**

**Formule (XII')** : $R_1$ = n-Pentyl, $R_2$ = Méthyl, $R'_3$ = Méthyl,

V = 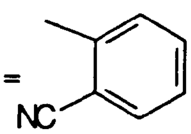

7 g de n-Pentyl-6 méthyl-2 (cyano-2'biphényl-yl-4) méthyl-5 hydroxy-4 pyrimidine, préparé à l'exemple 124, sont dissous dans 70 ml de diméthyl formamide. Après addition de 0,8 g d'hydrure de sodium à 60 %, le mélange est agité 30 minutes à température ambiante 2 ml d'iodure de méthyle sont ajoutés et la solution est chauffée 2 heures à 80°C. Le solvant est évaporé sous vide et le résidu est repris à l'eau et extrait à l'éther. La phase éthérée est séchée sur sulfate de magnésium et évaporée sous vide, le résidu est chromatographié sur gel de silice dans un éluant chloroforme / acétone 90 / 10 pour donner 5,8 g de n-Pentyl-6 diméthyl-2,3 (cyano-2'biphényl-yl-4) méthyl-5 dihydro-3,4 oxo-4 pyrimidine sous forme d'une huile utilisée telle quelle pour la suite.

Exemple 127 : **Chlorhydrate de n-Pentyl-6 diméthyl-2,3 [(tétrazolyl-5)-2' biphenyl-yl-4] méthyl-5 dihydro-3,4 oxo-4 pyrimidine**

**Formule (I')** : $R_1$ = n-Pentyl, $R_2$ = Méthyl, $R'_3$ = Méthyl,

$R_4$ = 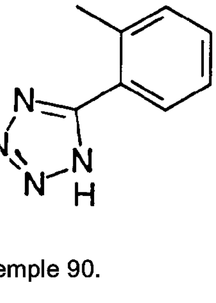

Préparé selon le mode opératoire de l'exemple 90.

Cristaux de point de fusion 215-217°C.

Exemple 128 : **n-Pentyl-6 méthyl-2 chloro-4 (cyano-2′ biphényl -yl-4) méthyl-5 pyrimidine**

**Formule (XII)** : $R_1$ = n-Pentyl, $R_2$ = Méthyl, $XR_3$ = Cl,

V = 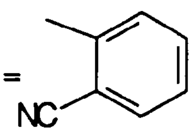

Préparé selon le mode opératoire de l'exemple 19.
Huile utilisée telle quelle pour la suite.

Exemple 129 : **[n-Pentyl-6 méthyl-2 (cyano-2′ biphenyl-yl-4) méthyl-5 pyrimidine-yl-4] oxy-2 acétate d'éthyle**

**Formule (XII)** : $R_1$ = n-Pentyl, $R_2$ = Méthyl, X = O,

$R_3 = CH_2CO_2$ Et , V = 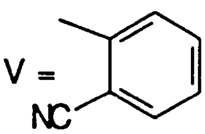

3 g d'hydroxy-2 acétate d'éthyle sont dissous dans 50 ml de tétrahydrofurane anhydre. 1,1 g d'hydrure de sodium à 60 % sont ajoutés et le mélange est agité 30 minutes à 50-60°C. 5,4 g de n-Pentyl-6 méthyl-2 chloro-4 (cyano-2′biphényl-yl-4) méthyl-5 pyrimidine, préparé à l'exemple 128, sont ajoutés et le mélange est chauffé 8 heures au reflux. Le solvant est évaporé sous vide, le résidu repris à l'eau et extrait à l'acétate d'éthyle. La phase organique est séchée sur sulfate de magnésium et concentrée sous vide pour donner une huile qui cristallise dans l'éther isopropylique pour donner 3,4 g de [n-Pentyl-6 méthyl-2 (cyano-2′biphényl-yl-4) méthyl-5 pyrimidine-yl-4] oxy-2 acétate d'éthyle sous forme de cristaux de point de fusion 68°C.

Exemple 130 : **[n-Pentyl-6 méthyl-2 [(tétrazolyl-5)-2′ biphényl -yl-4] méthyl-5 pyrimidine-yl-4] oxy-2 acétate d'éthyle**

**Formule (I)** : $R_1$ = n-Pentyl, $R_2$ = Méthyl, X = O,

$R_3 = CH_2CO_2$ Et , $R_4$ = 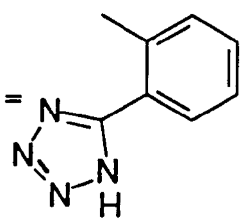

Préparé selon le mode opératoire de l'exemple 89.
Cristaux de point de fusion 146-148°C.

Exemple 131 : **[n-Propyl-6 méthyl-2 (cyano-2′ biphényl -yl-4) méthyl-5 pyrimidine-yl-4] oxy-2 acétate d'éthyle**

**Formule (XII)** : $R_1$ = n-Propyl, $R_2$ = Méthyl, X = O,

$R_3 = CH_2CO_2\,Et$, V = 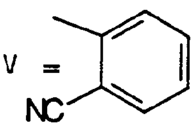

Préparé selon le mode opératoire de l'exemple 129.
Huile utilisée telle quelle pour la suite.

Exemple 132 : **[n-Propyl-6 méthyl-2 [(tétrazolyl-5)-2′ biphényl -yl-4] méthyl-5 pyrimidine-yl-4] oxy-2 acétate d'éthyle**

**Formule (I)** : $R_1$ = n-Propyl, $R_2$ = Méthyl, X = O,

$R_3 = CH_2CO_2\,Et$, $R_4$ = 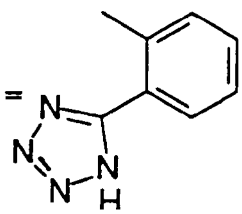

Préparé selon le mode opératoire de l'exemple 89.
Cristaux de point de fusion 149-150°C.

Exemple 133 : **n-Propyl-6 mercapto-2 hydroxy-4 (nitro-4 benzyl)-5 pyrimidine**

**Formule (XI)** : $R_1$ = n-Propyl, $R_2$ = SH, T = OH, V = $NO_2$

5,7 g de sodium sont dissous dans 150 ml de méthanol et 19 g de thiourée sont ajoutés. Le mélange est agité 5 minutes à température ambiante et 55 g d'oxo-3 hexanoate d'éthyle, préparé à l'exemple 1, sont additionnés. Après 8 heures de reflux le solvant est concentré au demi sous vide et le résidu est repris à l'eau et neutralisé par addition d'acide chlorhydrique dilué. Le mélange est abandonné une nuit à température ambiante et les cristaux formés sont essorés, lavés à l'éther et à l'éthanol, séchés pour donner 29 g de n-Propyl-6 mercapto-2 hydroxy-4 (nitro-4 benzyl)-5 pyrimidine sous forme de cristaux de point de fusion 240°C.

Exemple 134 : **n-Propyl-6 méthylthio-2 hydroxy-4 (nitro-4 benzyl)-5 pyrimidine**

**Formule (XI)** : $R_1$ = n-Propyl, $R_2$ = $SCH_3$, T = OH, V = $NO_2$

26 g de n-Propyl-6 mercapto-2 hydroxy-4 (nitro-4 benzyl)-5 pyrimidine, préparé à l'exemple 133, et 8,3 g de potasse sont dissous dans 200 ml de méthanol. Le mélange est agité pendant 10 minutes à température ambiante et 5,3 ml d'iodure de méthyle sont ajoutés. Le mélange est alors chauffé 2 heures au reflux. Le solvant est évaporé sous vide et le résidu est repris à l'eau. Après acidification par une solution d'acide chlorhydrique dilué, la phase aqueuse est extraite au chloroforme. La phase organique est séchée sur sulfate de magnésium puis évaporée sous vide et le résidu cristallise dans un mélange chloroforme / méthanol pour donner 13,4 g de n-Propyl-6 méthylthio-2 hydroxy-4 (nitro-4 benzyl)-5 pyrimidine sous forme de cristaux de point de fusion 223°C.

Exemple 135 : **n-Propyl-6 méthylthio-2 chloro-4 (nitro-4 benzyl)-5 pyrimidine**

**Formule (XII)** : $R_1$ = n-Propyl, $R_2$ = $SCH_3$, $XR_3$ = Cl, V = $NO_2$

Préparé selon le mode opératoire de l'exemple 19.
Cristaux de point de fusion 108°C.

Exemple 136 : **[n-Propyl-6 méthylthio-2 (nitro-4 benzyl)-5 pyrimidine-yl-4] mercapto-2 éthanol**

**Formule (XII)** : $R_1$ = n-Propyl, $R_2 = SCH_3$, X = S, $R_3 = CH_2CH_2OH$, V = $NO_2$

Préparé selon le mode opératoire de l'exemple 34.

Cristaux de point de fusion 97°C.

Exemple 137 : **[n-Propyl-6 méthylthio-2 (amino-4 benzyl)-5 pyrimidine-yl-4] mercapto-2 éthanol**

**Formule (XII)** : $R_1$ = n-Propyl, $R_2 = SCH_3$, X = S, $R_3 = CH_2CH_2OH$, V = $NH_2$

Préparé selon le mode opératoire de l'exemple 12.

Huile utilisée telle quelle pour la suite.

Exemple 138 : **Acide [[n-Propyl-6 methylthio-2 (hydroxy-2 éthyl) mercapto-4 pyrimidine-yl-5] méthyl phényl-yl-4] amino carbonyl-2 benzène sulfonique**

**Formule (I)** : $R_1$ = n-Propyl, $R_2 = SCH_3$, X = S,

$R_3 = CH_2CH_2OH$ , $R_4$ = 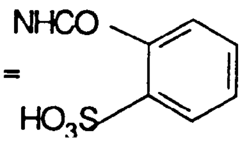

Préparé selon le mode opératoire de l'exemple 13.

Cristaux de point de fusion 198-201°C.

Exemple 139 : **[n-Propyl-6 méthyl-2 (cyano-2′ biphényl-yl-4) méthyl-5 pyrimidine-yl-4] mercapto-3 propanol**

**Formule (XII)** : $R_1$ = n-Propyl, $R_2$ = Méthyl, X = S,

$R_3 = CH_2CH_2CH_2OH$ , V = 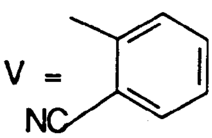

5 g de n-Propyl-6 méthyl-2 mercapto-4 (cyano-2′biphényl-yl-4) méthyl-5 pyrimidine préparé à l'exemple 95, sont dissous dans 50 ml d'éthanol. 0,4 g de sodium dissous dans 10 ml d'éthanol sont ajoutés et le mélange est agité 10 minutes à température ambiante. 2,5 g de bromo-3 propanol sont ajoutés et le mélange réactionnel est chauffé 6 heures au reflux. Le solvant est ensuite évaporé sous vide et le résidu est repris à l'eau et extrait à l'éther. La phase éthérée est séchée sur sulfate de magnésium et évaporée sous vide pour donner 5 g de [n-Propyl-6 méthyl-2 (cyano-2′biphényl-yl-4)méthyl-5 pyrimidine-yl-4] mercapto-3 propanol sous forme d'une huile utilisée telle quelle pour la suite.

Exemple 140 : **n-Propyl-6 méthyl-2 (acétoxy-3 propyl) mercapto-4 (cyano-2′ biphényl-yl-4) méthyl-5 pyrimidine**

**Formule (XII)** : $R_1$ = n-Propyl, $R_2$ = Méthyl, X = S,

$R_3 = CH_2CH_2CH_2\,O\,CO\,CH_3$, V = 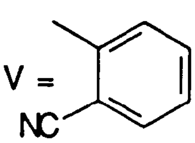

Préparé selon le mode opératoire de l'exemple 100.
Huile utilisée telle quelle pour la suite.

Exemple 141 : **n-Propyl-6 méthyl-2 (acétoxy-3 propyl) mercapto-4 [(tétrazolyl-5)-2′ biphényl-yl-4] méthyl-5 pyrimidine**

**Formule (I)** : $R_1$ = n-Propyl, $R_2$ = Méthyl, X = S,

$R_3 = CH_2CH_2CH_2\,O\,CO\,CH_3$, $R_4$ =

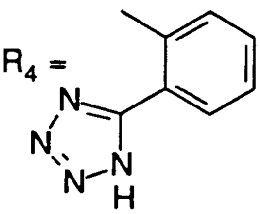

Préparé selon le mode opératoire de l'exemple 89.
Huile utilisée telle quelle pour la suite.

Exemple 142 : **[n-Propyl-6 méthyl-2 [(tétrazolyl-5)-2′ biphényl -yl-4] méthyl-5 pyrimidine-yl-4] mercapto-3 propanol**

**Formule (I)** : $R_1$ = n-Propyl, $R_2$ = Méthyl, X = S,

$R_3 = CH_2CH_2CH_2OH$, $R_4$ =

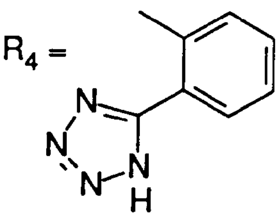

3 g de n-Propyl-6 méthyl-2 (acétoxy-3 propyl) mercapto-4 [(tétrazolyl-5)-2′biphényl-yl-4] méthyl-5 pyrimidine, préparé à l'exemple 141 sont dissous dans 30 ml d'éthanol en présence de 1 g de soude en pastille. Le mélange est chauffé une heure à 50°C. L'alcool est évaporé sous vide et le résidu est repris à l'eau tiède, la solution est acidifiée par barbottage de dioxyde de soufre et extraite au chloroforme, la phase organique est concentrée sous vide et le résidu obtenu cristallise dans l'acétone pour donner 2,2 g de [n-Propyl-6 méthyl-2 [(tétrazolyl-5)-2′ biphényl -yl-4] méthyl-5 pyrimidine-yl-4] mercapto-3 propanol sous forme de cristaux de point de fusion 157-158°C.

Exemple 143 : **n-Propyl-6 hydroxy-4 (cyano-2′ biphényl-yl-4) méthyl-5 pyrimidine**

**Formule (XI)** : $R_1$ = n-Propyl, $R_2$ = H, T = OH,

V =

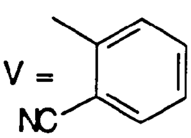

13 g de (cyano-2′biphényl-yl-4) méthyl-2 oxo-3 hexanoate d'éthyle, préparé à l'exemple 6, sont dissous dans 100 ml de diglyme. 8 g d'acétate de formamidine sont ajoutés et le mélange est chauffé 8 heures à 200°C puis repris à l'eau et extrait à l'acétate d'éthyle. La phase organique est lavée plusieurs fois avec une solution diluée de soude et les extraits aqueux sont neutralisés par l'acide chlorhydrique pour donner 2,6 g de n-Propyl-6 hydroxy-4 (cyano-2′biphényl-yl-4) méthyl-5 pyrimidine sous forme de cristaux de point de fusion 152°C.

Exemple 144 : **n-Propyl-6 chloro-4 (cyano-2′ biphényl-yl-4) méthyl-5 pyrimidine**

**Formule (XII)** : $R_1$ = n-Propyl, $R_2$ = H, $XR_3$ = Cl

V =

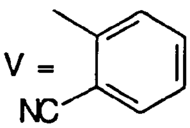

Préparé selon le mode opératoire de l'exemple 19.
Cristaux de point de fusion 58°C.

Exemple 145 : **n-Propyl-6 méthoxy-4 (cyano-2′ biphényl-yl-4) méthyl-5 pyrimidine**

**Formule (XII)** : $R_1$ = n-Propyl, $R_2$ = H, $XR_3$ = $OCH_3$

V =

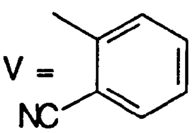

Préparé selon le mode opératoire de l'exemple 30.
Cristaux de point de fusion 110°C.

Exemple 146 : **n-Propyl-6 méthoxy-4 [(tétrazolyl-5)-2′ biphényl -yl-4] méthyl-5 pyrimidine**

**Formule (I)** : $R_1$ = n-Propyl, $R_2$ = H, X = O

$R_3 = CH_3$ , $R_4$ =

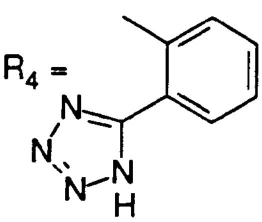

Préparé selon le mode opératoire de l'exemple 89.
Cristaux de point de fusion 148-149°C.

Exemple 147 : **n-Propyl-6 amino-2 hydroxy-4 (nitro-4 benzyl)-5 pyrimidine**

**Formule (XI)** : $R_1$ = n-Propyl, $R_2$ = $NH_2$, T = OH, V = $NO_2$

18,8 g de chlorhydrate de guanidine sont ajoutés à une solution d'éthylate de sodium obtenue par addition de 4,5 g de sodium dans 150 ml d'éthanol. Le mélange est agité 5 minutes et 50 g d'oxo-3 (nitro-4 benzyl)-2 hexanoate d'éthyle, préparé à l'exemple 3, sont ajoutés. Après 4 heures à température ambiante et 4 heures au reflux, le mélange est repris à l'eau et les cristaux sont essorés, lavés à l'éther et à l'acétone pour donner 22,1 g de n-Propyl-6 amino-2 hydroxy-4 (nitro-4 benzyl)-5 pyrimidine sous forme de cristaux de point de fusion 245-8°C.

Exemple 148 : **n-Propyl-6 amino-2 chloro-4 (nitro-4 benzyl)-5 pyrimidine**

**Formule (XII)** : $R_1$ = n-Propyl, $R_2$ = $NH_2$, $XR_3$ = Cl, V = $NO_2$

Préparé selon le mode opératoire de l'exemple 19.
Cristaux de point de fusion 140°C.

Exemple 149 : **[n-Propyl-6 amino-2 (nitro-4 benzyl)-5 pyrimidine -yl-4] mercapto-2 éthanol**

**Formule (XII)** : $R_1$ = n-Propyl, $R_2 = NH_2$, X= S, $R_3 = CH_2CH_2OH$, V = $NO_2$

Préparé selon le mode opératoire de l'exemple 34.
Cristaux de point de fusion 110°C.

Exemple 150 : **[n-Propyl-6 amino-2 (amino-4 benzyl)-5 pyrimidine -yl-4] mercapto-2 éthanol**

**Formule (XII)** : $R_1$ = n-Propyl, $R_2 = NH_2$, X= S, $R_3 = CH_2CH_2OH$, V = $NH_2$

Préparé selon le mode opératoire de l'exemple 12.
Cristaux de point de fusion 138°C.

Exemple 151 : **Acide [[n-Propyl-6 amino-2 (hydroxy-2 éthyl) mercapto-4 pyrimidine-yl-5] méthyl phényl-yl-4] amino carbonyl-2 benzène sulfonique**

**Formule (I)** : $R_1$ = n-Propyl, $R_2 = NH_2$, X= S,

$R_3 = CH_2CH_2OH$ , $R_4$ = 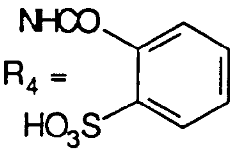

Préparé selon le mode opératoire de l'exemple 13.
Cristaux de point de fusion 228-230°C.

Exemple 152 : **N-[n-Butyl-6 méthyl-2 (cyano-2′ biphényl-yl-4) méthyl-5 pyrimidine-yl-4] oxy-2 éthyl phtalimide**

**Formule (XII)** : $R_1$ = n-Butyl, $R_2$ = Méthyl, X = O

$R_3 = CH_2CH_2$ 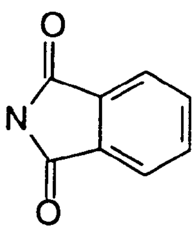 , V = 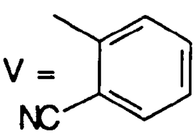

Préparé selon le mode opératoire de l'exemple 110 à partir du phtalimido-2 éthanol.
Cristaux de point de fusion 110°C.

Exemple 153 : **N-[n-Butyl-6 méthyl-2 [(tétrazolyl-5)-2′ biphényl -yl-4] méthyl-5 pyrimidine-yl-4] oxy-2 éthyl phtalimide**

**Formule (I)** : $R_1$ = n-Butyl, $R_2$ = Méthyl, X = O

$R_3 = CH_2CH_2$ 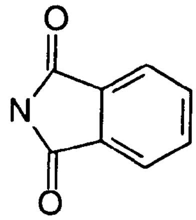 , $R_4$ = 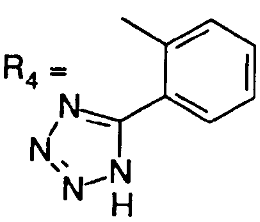

Préparé selon le mode opératoire de l'exemple 89.
Cristaux de point de fusion 200-201°C.

Exemple 154 : **n-Butyl-6 méthyl-2 (diméthyl amino-2 éthyl) mercapto-4 (cyano-2′ biphényl-yl-4) méthyl-5 pyrimidine**

**Formule (XII)** : $R_1$ = n-Butyl, $R_2$ = Méthyl, X = S,

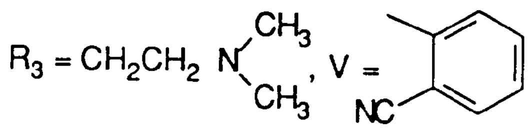

Préparé selon le mode opératoire de l'exemple 22 à partir de la N-mercapto-2 éthyl N,N diméthyl amine.
Huile utilisée telle quelle pour la suite.

Exemple 155 : **n-Butyl-6 méthyl-2 (diméthyl amino-2 éthyl) mercapto-4 [(tetrazolyl-5)-2′ biphenyl-yl-4] méthyl-5 pyrimidine**

**Formule (I)** : $R_1$ = n-Butyl, $R_2$ = Méthyl, X = S,

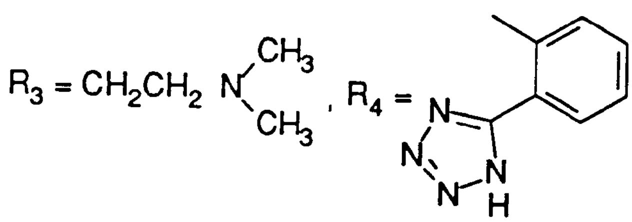

Préparé selon le mode opératoire de l'exemple 89.
Cristaux de point de fusion 187-188°C.

Exemple 156 : **n-Butyl-6 méthyl-2 (méthylthio-2 éthyl) oxy-4 (cyano-2′ biphényl-yl-4) méthyl-5 pyrimidine**

**Formule (XII)** : $R_1$ = n-Butyl, $R_2$ = Méthyl, X = O,

$R_3 = CH_2CH_2S\ CH_3$ ,V = 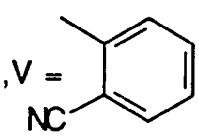

Préparé selon le mode opératoire de l'exemple 110 à partir du méthylthio-2 éthanol.
Huile utilisée telle quelle pour la suite.

Exemple 157 : **n-Butyl-6 méthyl-2 (méthylthio-2 éthyl) oxy-4 [(tétrazolyl-5)-2′ biphényl-yl-4] méthyl-5 pyrimidine**

**Formule (I)** : $R_1$ = n-Butyl, $R_2$ = Méthyl, X = O,

$R_3 = CH_2CH_2S\ CH_3$ , $R_4$ = 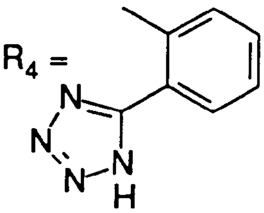

Préparé selon le mode opératoire de l'exemple 89.
Cristaux de point de fusion 121-122°C.

Exemple 158 : **[n-Butyl-6 méthyl-2 (cyano-2′ biphényl-yl-4) méthyl-5 pyrimidine-yl-4] mercapto-2 éthanol**

**Formule (XII)** : $R_1$ = n-Butyl, $R_2$ = Méthyl, X = S,

$R_3 = CH_2CH_2OH$ , V = 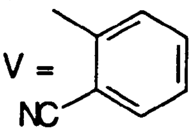

Préparé selon le mode opératoire de l'exemple 22.
Huile utilisée telle quelle pour la suite.

Exemple 159 : **Acétate de [n-Butyl-6 méthyl-2 (cyano-2′biphényl -yl-4) méthyl-5 pyrimidine-yl-4] mercapto-2 éthyle**

**Formule (XII)** : $R_1$ = n-Butyl, $R_2$ = Méthyl, X = S,

$R_3 = CH_2CH_2\ O\ CO\ CH_3$ , V = 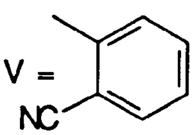

Préparé selon le mode opératoire de l'exemple 100.
Huile utilisée telle quelle pour la suite.

Exemple 160 : **Acétate de [n-Butyl-6 Méthyl-2 [(tétrazolyl-5)-2′ biphényl-yl-4] méthyl-5 pyrimidine-yl-4] mercapto-2 éthyle**

**Formule (I)** : $R_1$ = n-Butyl, $R_2$ = Méthyl, X = S,

$R_3 = CH_2CH_2O\ CO\ CH_3$ , $R_4$ =

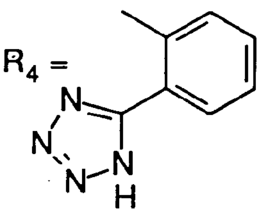

Préparé selon le mode opératoire de l'exemple 89.
Cristaux de point de fusion 112-114°C.

Exemple 161 : **[n-Butyl-6 méthyl-2 [(tétrazolyl-5)-2′ biphényl-yl-4] méthyl-5 pyrimidine-yl-4] mercapto-2 éthanol**

**Formule (I)** : $R_1$ = n-Butyl, $R_2$ = Méthyl, X = S,

$R_3 = CH_2CH_2OH$ ,$R_4$ =

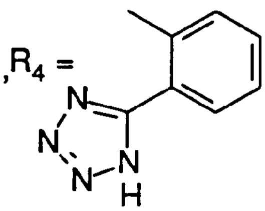

3,7 g d'acétate de [n-Butyl-6 méthyl-2 [(tétrazolyl-5)-2′biphényl-yl-4] méthyl-5 pyrimidine-yl-4] mercapto-2 éthyle préparé à l'exemple 160, sont dissous dans 300 ml d'éthanol et 100 ml d'eau en présence d'1 g de soude en pastille. Le mélange est agité 48 heures à température ambiante, les solvants sont concentrés, 200 ml d'eau sont ajoutés et la phase aqueuse est lavée à l'acétate d'éthyle puis à l'éther et acidifiée par barbottage de dioxyde de soufre, les cristaux obtenus sont essorés, lavés à l'eau et à l'éther et chromatographiés sur gel de silice avec un éluant dichlorométhane / méthanol 9/1 pour donner 1,7 g de [n-Butyl-6 méthyl-2 [(tétrazolyl-5)-2′biphényl-yl-4] méthyl-5 pyrimidine-yl-4] mercapto-2 éthanol sous forme de cristaux de point de fusion 161-162°C.

Exemple 162 : **[n-Butyl-6 méthyl-2 (cyano-2′ biphényl-yl-4) méthyl-5 pyrimidine-yl-4] oxy-2 acétate d'éthyle**

**Formule (XII)** : $R_1$ = n-Butyl, $R_2$ = Méthyl, X = O,

$R_3 = CH_2CO_2Et$, V =

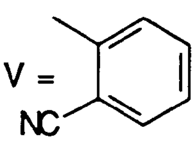

Préparé selon le mode opératoire de l'exemple 129.
Huile utilisée telle quelle pour la suite.

Exemple 163 : **[n-Butyl-6 méthyl-2 [(tétrazolyl-5)-2′ biphényl-yl-4] méthyl-5 pyrimidine-yl-4] oxy-2 acétate d'éthyle**

**Formule (I)** : $R_1$ = n-Butyl, $R_2$ = Méthyl, X = O,

$R_3 = CH_2CO_2Et$, $R_4$ = 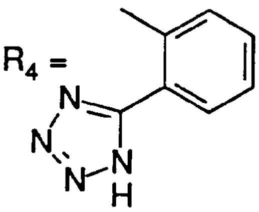

Préparé selon le mode opératoire de l'exemple 89.
Cristaux de point de fusion 133-135°C.

Exemple 164 : **[n-Propyl-6 méthyl-2 (cyano-2′ biphényl-yl-4) méthyl-5 pyrimidine-yl-4] oxy méthyl-4 diméthyl-2,2-dioxolane-1,3**

**Formule (XII)** : $R_1$ = n-Propyl, $R_2$ = Méthyl, X = O,

$R_3 = CH_2$ 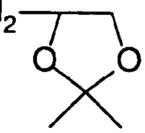 , V = 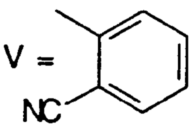

Préparé selon le mode opératoire de l'exemple 110.
Huile utilisée telle quelle pour la suite.

Exemple 165 : **Sel de sodium du [n-Propyl-6 méthyl-2 [(tétrazolyl-5)-2′ biphényl-yl-4] méthyl-5 pyrimidine-yl-4] oxyméthyl-4 diméthyl-2,2-dioxolane-1,3**

**Formule (I)** : $R_1$ = n-Propyl, $R_2$ = Méthyl, X = O,

$R_3 = CH_2$ 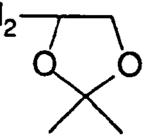 , $R_4$ = 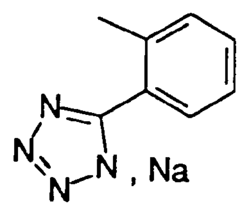

Préparé selon le mode opératoire de l'exemple 89, le sel de sodium étant obtenu par traitement du dérivé tétrazole avec 0,9 équivalent de soude en solution alcoolique.
Cristaux de point de fusion 224-225°C.

Exemple 166 : **[(cyano-3 thiényl-2)-4 benzyl]-2 oxo-3 hexanoate d'éthyle**

**Formule (VI)** : $R_1$ = n-Propyl, $R_{13}$ = Ethyl,

V = 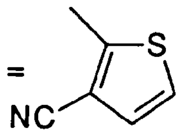

Préparé selon le mode opératoire de l'exemple 3 à partir du bromure de (cyano-3 thiényl-2)-4 benzyle. Huile utilisée telle quelle pour la suite.

Préparation du bromure de (cyano-3 thiényl-2)-4 benzyle :

A) Méthyl-4'chloro-4 butyrophénone :

53 ml de toluène et 70,5 g de chlorure de l'acide chloro-4 butyrique sont dissous dans 100 ml de dichlorométhane et la solution est ajoutée à 10°C à une suspension de 74 g de chlorure d'aluminium dans 200 ml de dichlorométhane. On laisse ensuite la température s'élever pendant un quart d'heure, le mélange est traité par de l'eau glacée. La phase organique est séchée sur sulfate de magnésium et évaporée sous vide pour donner 96,9 g de méthyl-4'chloro-4 butyrophénone sous forme d'une huile utilisée telle quelle pour la suite.

B) α-chloro β-(chloro-2 éthyl) méthyl-4'cinnamaldéhyde :

130 ml d'oxychlorure de phosphore sont ajoutés lentement, à 0°C, sur 130 ml de diméthyl formamide, puis 117,5 g de méthyl-4'chloro-4 butyrophénone, préparée en A), en solution dans 50 ml de diméthyl formamide sont ajoutés goutte à goutte. Le mélange est ensuite agité à température ambiante pendant une heure, puis à 50°C pendant 2 heures et à 70°C pendant 1 heure. Le mélange est alors versé sur de la glace et repris à l'éther, la phase éthérée est lavée avec une solution saturée de bicarbonate de sodium, séchée sur sulfate de sodium et évaporée sous vide pour donner 133,8 g d'α-chloro β-(chloro-2 éthyl) méthyl-4' cinnamaldéhyde sous forme d'une huile utilisée telle quelle pour la suite.

C) (méthyl-4 phényl)-2 dihydro-4,5-thiophène-3 carboxaldéhyde :

15,9 g d'α-chloro β-(chloro-2 éthyl) méthyl-4'cinnamaldéhyde préparés en B) et 22 g de sulfure de sodium (9 $H_2O$) sont ajoutés à 200 ml de THF. Une quantité d'eau suffisante est ajoutée afin que le sulfure de sodium passe entièrement en solution et le mélange est ensuite chauffé pendant 3 heures au reflux, refroidi puis repris à l'éther. La phase organique est décantée, lavée à l'eau puis séchée sur sulfate de magnésium et évaporée sous vide pour donner 13,5 g de (méthyl-4 phényl)-2 dihydro-4,5-thiophène-3 carboxaldéhyde sous forme d'une huile utilisée telle quelle pour la suite.

D) (Méthyl-4 phényl)-2 cyano-3 dihydro-4,5-thiophène :

15 g de (méthyl-4 phényl)-2 dihydro-4,5-thiophène-3 carboxaldéhyde, préparés en C) et 6,5 g de chlorhydrate d'hydroxylamine sont mélangés dans 40 ml d'éthanol et 10 ml d'eau. Une solution de 4,7 g de carbonate de sodium dans 10 ml d'eau est ajoutée. Le mélange est agité à température ambiante pendant une demi-heure, puis extrait à l'éther. La phase éthérée est lavée à l'eau, puis séchée sur sulfate de sodium et évaporée sous vide pour donner 15,2 g d'un résidu jaune gommeux. Ce résidu est ajouté à 13 ml d'anhydride acétique et le mélange chauffe légèrement, brunit et devient liquide. Le mélange est ensuite chauffé 1 heure au reflux, puis versé sur de la glace et extrait au dichlorométhane et lavé par une solution saturée de bicarbonate de soude, puis la phase organique est séchée sur sulfate de magnésium et évaporée sous vide, le résidu obtenu est chromatographié sur gel de silice dans le dichlorométhane pour donner 10 g de (méthyl-4 phényl)-2 cyano-3 dihydro-4,5-thiophène sous forme d'une huile utilisée telle quelle pour la suite.

E) (Méthyl-4 phényl)-2 cyano-3 thiophène :

49,9 g de (Méthyl-4 phényl)-2 cyano-3 dihydro-4,5-thiophène, préparés en D) sont dissous dans 200 ml de tétrachlorure de carbone, le mélange est chauffé au reflux et, au bout de deux heures, 11 g de brome en solution dans 200 ml de tétrachlorure de carbone sont ajoutés goutte à goutte. Le reflux est poursuivi jusqu'à cessation du dégagement d'acide bromhydrique puis le solvant est évaporé sous vide. Le résidu est repris dans 200 ml de tétrahydrofurane anhydre et 28 g de tertiobutylate de potassium sont ajoutés. Le mélange est chauffé une heure au reflux puis refroidi et additionné d'eau et de chlorure de sodium et extrait à l'éther. La phase organique est évaporée sous vide pour donner 31,8 g de (méthyl-4 phényl)-2 cyano-3 thiophène sous forme d'huile utilisée telle quelle pour la suite.

F) Bromure de (cyano-3 thiényl-yl-2)-4 benzyle :

24,5 g de (méthyl-4 phényl)-2 cyano-3 thiophène, préparés en E), sont dissous dans 200 ml de tétrachlorure de carbone. 21,9 g de N-bromo succinimide sont ajoutés ainsi que 0,1 g de péroxyde de benzoyle. Le mélange est chauffé 24 heures au reflux. Les cristaux de succinimide sont filtrés et le solvant évaporé sous vide. Le résidu est repris dans un mélange d'hexane et d'acétate d'éthyle et la solution est gardée 24 heures au congélateur. Les cristaux formés sont essorés pour donner 14 g de bromure de (cyano-3 thiényl-yl-2)-4 benzyle sous forme de cristaux de point de fusion 80°C.

Exemple 167 : **n-Propyl-6 méthyl-2 hydroxy-4 [(cyano-3 thiényl-2)-4 benzyl]-5 pyrimidine**

**Formule (XI)** : $R_1$ = n-Propyl, $R_2$ = Méthyl, T = OH,

V =

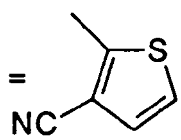

Préparé selon le mode opératoire de l'exemple 7.
Cristaux de point de fusion 180°C.

Exemple 168 : **n-Propyl-6 méthyl-2 chloro-4 [(cyano-3 thiényl-2)-4 benzyl]-5 pyrimidine**

**Formule (XII)** : $R_1$ = n-Propyl, $R_2$ = Méthyl, $XR_3$ = Cl

V =

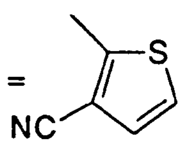

Préparé selon le mode opératoire de l'exemple 19.
Huile utilisée telle quelle pour la suite.

Exemple 169 : **n-Propyl-6 méthyl-2 méthoxy-4 [(cyano-3 thiényl-2)-4 benzyl]-5 pyrimidine**

**Formule (XII)** : $R_1$ = n-Propyl, $R_2$ = Méthyl, $XR_3$ = $OCH_3$,

V =

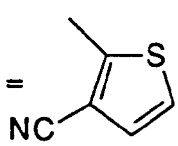

Préparé selon le mode opératoire de l'exemple 30.
Huile utilisée telle quelle pour la suite.

Exemple 170 : **n-Propyl-6 méthyl-2 méthoxy-4 [(tétrazolyl-5)-3 thiényl-2]-4 benzyl]-5 pyrimidine**

**Formule (I)** : $R_1$ = n-Propyl, $R_2$ = Méthyl, $XR_3 = OCH_3$,

$R_4$ =

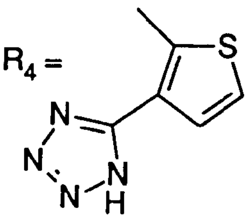

Préparé selon le mode opératoire de l'exemple 89.
Cristaux de point de fusion 155°C.

Exemple 171 : **(méthoxy carbonyl-2′ biphényl-yl-4) méthyl-2 oxo-3 heptanoate d'éthyle**

**Formule (VI)** : $R_1$ = n-Butyl, $R_{13}$ = Ethyl,

V =

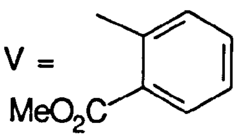

Préparé selon le mode opératoire de l'exemple 5.
Huile utilisée telle quelle pour la suite.

Exemple 172 : **n-Butyl-6 méthyl-2 hydroxy-4 (méthoxy carbonyl-2′ biphényl-yl-4) méthyl-5 pyrimidine**

**Formule (XI)** : $R_1$ = n-Butyl, $R_2$ = Méthyl, T = OH,

V =

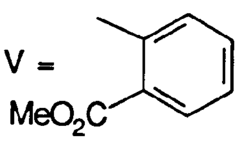

Préparé selon le mode opératoire de l'exemple 7.
Cristaux de point de fusion 209-210°C.

Exemple 173 : **n-Butyl-6 méthyl-2 chloro-4 (méthoxy carbonyl-2′ biphényl-yl-4) méthyl-5 pyrimidine**

**Formule (XII)** : $R_1$ = n-Butyl, $R_2$ = Méthyl, $XR_3$ = Cl

V =

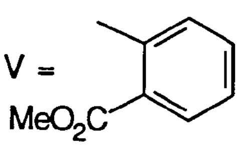

Préparé selon le mode opératoire de l'exemple 19.
Huile utilisée telle quelle pour la suite.

Exemple 174 : **n-Butyl-6 méthyl-2 méthoxy-4 (méthoxy carbonyl-2′ biphényl-yl-4) méthyl-5 pyrimidine**

**Formule (XII)** : $R_1$ = n-Butyl, $R_2$ = Méthyl, $XR_3$ = $OCH_3$

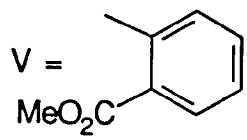

Préparé selon le mode opératoire de l'exemple 30.
Huile utilisée telle quelle pour la suite.

Exemple 175 : **Acide [n-butyl-6 méthyl-2 méthoxy-4 pyrimidine-yl-5] méthyl-4′ biphényl-2 carboxylique**

**Formule (I)** : $R_1$ = n-Butyl, $R_2$ = Méthyl, $XR_3$ = $OCH_3$,

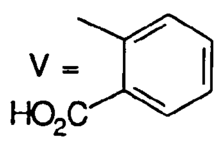

2,1 g de n-Butyl-6 méthyl-2 méthoxy-4 (méthoxy carbonyl-2′biphényl-yl-4) méthyl-5 pyrimidine préparé à l'exemple 174 et 0,35 g de soude en pastille sont dissous dans 15 ml de méthanol et 10 ml d'eau. La solution est chauffé à 60°C pendant 10 heures, les solvants sont évaporés sous vide et le résidu est repris à l'eau, la phase aqueuse est acidifiée par l'acide chlorhydrique dilué. Les cristaux sont essorés, lavés à l'eau plusieurs fois et séchés pour donner 1,8 g d'acide [n-Butyl-6 méthyl-2 méthoxy-4 pyrimidine-yl-5] méthyl-4′biphényl-2 carboxylique sous forme de cristaux de point de fusion 168°C.

Exemple 176 : **n-Propyl-6 méthyl-2 méthoxy-4 (nitro-4 benzyl)-5 pyrimidine**

**Formule (XII)** : $R_1$ = n-Propyl, $R_2$ = Méthyl, $XR_3$ = $OCH_3$, V = $NO_2$

Préparé selon le mode opératoire de l'exemple 30.
Huile utilisée telle quelle pour la suite.

Exemple 177 : **n-Propyl-6 méthyl-2 méthoxy-4 (amino-4 benzyl)-5 pyrimidine**

**Formule (XII)** : $R_1$ = n-Propyl, $R_2$ = Méthyl, $XR_3$ = OMe, V = $NH_2$

5,3 g de n-Propyl-6 méthyl-2 méthoxy-4 (nitro-4 benzyl)-5 pyrimidine préparé à l'exemple 176 sont dissous dans 70 ml de diglyme. 1,15 g de borohydrure de lithium sont ajoutés et le mélange est chauffé au reflux 8 ml de méthanol sont additionnés goutte à goutte en deux heures et le mélange est ensuite chauffé 2 heures supplémentaires au reflux, puis repris à l'eau. Les cristaux sont essorés et dissous dans l'éther isopropylique. La solution éthérée est séchée sur sulfate de magnésium et évaporée sous vide pour donner 3,8 g de n-Propyl-6 méthyl-2 méthoxy-4 (amino-4 benzyl)-5 pyrimidine sous forme de cristaux de point de fusion 179-181°C.

Exemple 178 : **Acide [(n-Propyl-6 méthyl-2 méthoxy-4 pyrimidine-yl-5) méthyl phényl-yl-4] amino carbonyl-2 benzène sulfonique**

**Formule (I)** : $R_1$ = n-Propyl, $R_2$ = Méthyl, $XR_3$ = OMe,

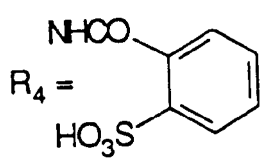

Préparé selon le mode opératoire de l'exemple 13.
Cristaux de point de fusion 200-204°C.

Exemple 179 : **n-Butyl-6 méthyl-2 (nitro-4 benzyl)-5 (propionyl oxy-2 éthyl) oxy-4 pyrimidine**

**Formule (XII)** : $R_1$ = n-Butyl, $R_2$ = Méthyl, X = O, $R_3$ = $CH_2CH_2O$ CO $CH_2CH_3$, V = $NO_2$

Préparé selon le mode opératoire de l'exemple 100 à partir du propionate d'hydroxy-2 éthyle obtenu par hydrogénation catalytique en présence de Nickel de Raney de l'acrylate d'hydroxy-2 éthyle commercial.
Huile utilisée telle quelle pour la suite.

Exemple 180 : **[n-Butyl-6 méthyl-2 (nitro-4 benzyl)-5 pyrimidine-yl-4] oxy-2 éthanol**

**Formule (XII)** : $R_1$ = n-Butyl, $R_2$ = Méthyl, X = O, $R_3$ = $CH_2CH_2OH$, V = $NO_2$

Préparé selon le mode opératoire de l'exemple 142.
Huile utilisée telle quelle pour la suite.

Exemple 181 : **[n-Butyl-6 méthyl-2 (amino-4 benzyl)-5 pyrimidine-yl-4] oxy-2 éthanol**

**Formule (XII)** : $R_1$ = n-Butyl, $R_2$ = Méthyl, X = O, $R_3$ = $CH_2CH_2OH$, V = $NH_2$

Préparé selon le mode opératoire de l'exemple 12.
Huile utilisée telle quelle pour la suite.

Exemple 182 : **Acide [[n-Butyl-6 méthyl-2 (hydroxy-2 éthyl) oxy-4 pyrimidine-yl-5] méthyl phényl-yl-4] amino carbonyl-2 benzène sulfonique**

**Formule (I)** : $R_1$ = n-Butyl, $R_2$ = Méthyl, X = O,

$R_3$ = $CH_2CH_2OH$ , $R_4$ = 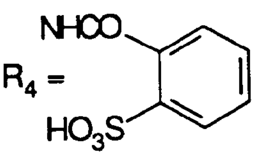

Préparé selon le mode opératoire de l'exemple 13.
Cristaux de point de fusion : 238-9°C.

Exemple 183 : **[n-Butyl-6 méthyl-2 (cyano-2′ biphényl-yl-4) méthyl-5 dihydro-3,4 oxo-4 pyrimidine-yl-3] acétate d'éthyle**

**Formule (XII')** : $R_1$ = n-Butyl, $R_2$ = Méthyl,

$R'_3$ = $CH_2CO_2Et$ , V = 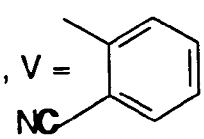

Préparé selon le mode opératoire de l'exemple 88.
Huile utilisée telle quelle pour la suite.

Exemple 184 : **[n-Butyl-6 méthyl-2 [(tétrazolyl-5)-2′ biphényl-yl -4] méthyl-5 dihydro-3,4 oxo-4 pyrimidine-yl-3] acétate d'éthyle**

**Formule (I')** : $R_1$ = n-Butyl, $R_2$ = Méthyl, $R'_3$ = $CH_2CO_2Et$,

$R_4$ = 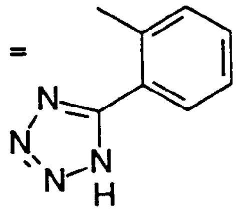

Préparé selon le mode opératoire de l'exemple 89.
Cristaux de point de fusion 170-1 °C.

Exemple 185 : **n-Butyl-6 diméthyl-2,3 (cyano-2′ biphényl-yl-4) méthyl-5 dihydro-3,4 oxo-4 pyrimidine**

**Formule (XII')** : $R_1$ = n-Butyl, $R_2$ = Méthyl, $R'_3$ = méthyl,

V = 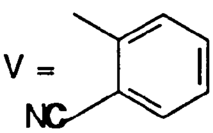

Préparé selon le mode opératoire de l'exemple 126.
Huile utilisée telle quelle pour la suite.

Exemple 186 : **n-Butyl-6 diméthyl-2,3 [(tétrazolyl-5)-2′ biphényl -yl-4] méthyl-5 dihydro-3,4 oxo-4 pyrimidine**

**Formule (I')** : $R_1$ = n-Butyl, $R_2$ = Méthyl, $R'_3$ = méthyl,

$R_4$ = 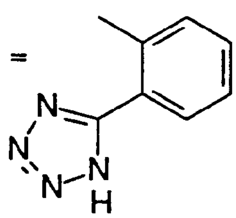

Préparé selon le mode opératoire de l'exemple 127.

Exemple 187 : **n-Butyl-6 méthyl-2 (chloro-2 benzyl)-3 (cyano-2′ biphényl-yl-4) méthyl-5 dihydro-3,4 oxo-4 pyrimidine**

**Formule (XII')** : $R_1$ = n-Butyl, $R_2$ = Méthyl,

$R'_3$ = $CH_2$— 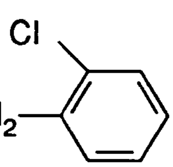 , V = 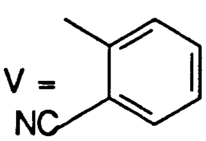

Préparé selon le mode opératoire de l'exemple 88.
Huile utilisée telle quelle pour la suite.

Exemple 188 : **n-Butyl-6 méthyl-2 (chloro-2 benzyl)-3 [(tétrazolyl -5)-2′ biphényl-yl-4] méthyl-5 dihydro-3,4 oxo-4 pyrimidine**

**Formule (I')** : $R_1$ = n-Butyl, $R_2$ = Méthyl,

$R'_3 = CH_2$ 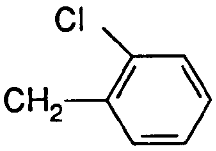 , $R_4$ = 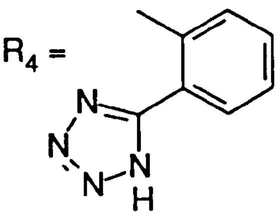

Préparé selon le mode opératoire de l'exemple 89.

Exemple 189 : **n-Butyl-6 méthyl-2 (cyano-2′ biphényl-yl-4) méthyl-5 oxo-4 dihydro-3,4 (acétoxy-2 éthyl)-3 pyrimidine**

**Formule (XII')** : $R_1$ = n-Butyl, $R_2$ = Méthyl,

$R'_3 = CH_2CH_2O\ CO\ CH_3$ , V = 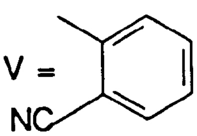

Préparé selon le mode opératoire de l'exemple 88.
Cristaux de point de fusion 107°C.

Exemple 190 : **n-Butyl-6 méthyl-2 [(tétrazolyl-5)-2′ biphényl -yl-4] méthyl-5 dihydro-3,4 oxo-4 (acétoxy-2 éthyl)-3 pyrimidine**

**Formule (I')** : $R_1$ = n-Butyl, $R_2$ = Méthyl,

$R'_3 = CH_2CH_2O\ CO\ CH_3$ , $R_4$ = 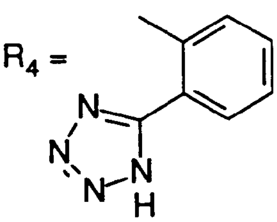

Préparé selon le mode opératoire de l'exemple 89.

Exemple 191 : **Acétate de [n-Butyl-6 méthyl-2 (cyano-2′biphényl -yl-4) méthyl-5 pyrimidine-yl-4] oxy-3 propyle**

**Formule (XII)** : $R_1$ = n-Butyl, $R_2$ = Méthyl, X = O,

$R_3 = CH_2CH_2CH_2\ O\ CO\ CH_3$ , V = 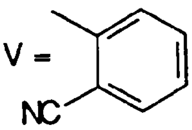

Préparé selon le mode opératoire de l'exemple 88 (éluant acétate d'éthyle / cyclohexane 5/5). Huile utilisée telle quelle pour la suite.

Exemple 197 : **n-Butyl-6 méthyl-2 (cyano-2′ biphényl-yl-4) méthyl-5 dihydro-3,4 oxo-4 (acétoxy-3 propyl)-3 pyrimidine**

**Formule (XII')** : $R_1$ = n-Butyl, $R_2$ = méthyl,

$R'_3 = CH_2CH_2CH_2\ O\ CO\ CH_3$ , V = 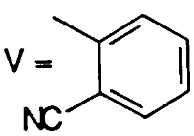

Préparé selon le mode opératoire de l'exemple 88 (éluant acétate d'éthyle / cyclohexane 7/3). Cristaux de point de fusion 108°C.

Exemple 193 : **n-Butyl-6 méthyl-2 [(tétrazolyl-5)-2′ biphényl-yl-4] méthyl-5 dihydro-3,4 oxo-4 (acétoxy-3 propyl)-3 pyrimidine**

**Formule (I')** : $R_1$ = n-Butyl, $R_2$ = méthyl,

$R'_3 = CH_2CH_2CH_2\ O\ CO\ CH_3$, $R_4$ = 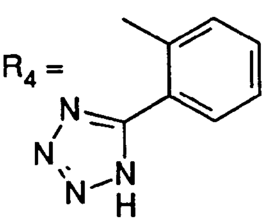

Préparé selon le mode opératoire de l'exemple 89.

Exemple 194 : **Acétate de [n-Butyl-6 méthyl-2 [(tétrazolyl-5)-2′ biphényl-yl-4] méthyl-5 pyrimidine-yl-4] oxy-3 propyle**

**Formule (I)** : $R_1$ = n-Butyl, $R_2$ = méthyl, X = O,

$R_3 = CH_2CH_2CH_2\ O\ CO\ CH_3$, $R_4$ =

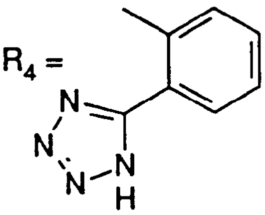

Préparé selon le mode opératoire de l'exemple 89.

Exemple 195 : **[n-Butyl-6 méthyl-2 [(tétrazolyl-5)-2′ biphényl -yl-4] méthyl-5 dihydro-3,4 oxo-4 pyrimidine -yl-3]-3 propanol**

**Formule (I')** : $R_1$ = n-Butyl, $R_2$ = méthyl,

$R'_3 = CH_2CH_2CH_2OH$, $R_4$ =

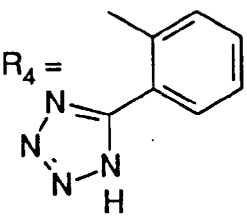

Préparé selon le mode opératoire de l'exemple 142.
Cristaux de point de fusion 181-2°C.

Exemple 196 : **[n-Butyl-6 méthyl-2 [(tétrazolyl-5)-2′ biphényl -yl-4] méthyl-5 pyrimidine-yl-4] oxy-2 éthanol**

**Formule (I)** : $R_1$ = n-Butyl, $R_2$ = méthyl, X = O,

$R_3 = CH_2CH_2OH$, $R_4$ =

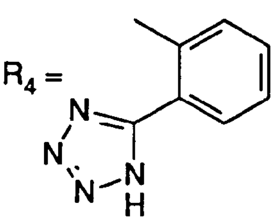

Préparé selon le mode opératoire de l'exemple 142.
Cristaux de point de fusion 173-4°C.

Exemple 197 : **[n-Butyl-6 méthyl-2 [(tétrazolyl-5)-2′biphényl -yl-4] méthyl-5 dihydro-3,4 oxo-4 pyrimidine -yl-3]-2 éthanol**

**Formule (I')** : $R_1$ = n-Butyl, $R_2$ = méthyl,

$R'_3 = CH_2CH_2OH$, $R_4$ = 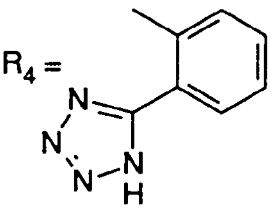

Préparé selon le mode opératoire de l'exemple 142.
Cristaux de point de fusion 195-6°C.

Exemple 198 : **n-propyl-6 mercapto-2 hydroxy-4 (cyano-2′ biphényl-yl-4) méthyl-5 pyrimidine**

**Formule (XI)** : $R_1$ = n-Propyl, $R_2$ = SH, T = OH

V = 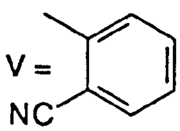

Préparé selon le mode opératoire de l'exemple 133.
Cristaux de point de fusion 191°C.
Selon les modes opératoires précédemment décrits, on pourra préparer les produits suivants :

- n-Propyl-6 dihydroxy-2,4 [(tétrazol-yl-5)-2′biphényl-yl-4] méthyl-5 pyrimidine
- n-Butyl-6 dihydroxy-2,4 [(tétrazol-yl-5)-2′biphényl-yl-4] méthyl-5 pyrimidine
- n-Propyl-6 dichloro-2,4 (carboxy-2′biphényl-yl-4) méthyl-5 pyrimidine
- n-Butyl-6 dichloro-2,4 (carboxy-2′biphényl-yl-4) méthyl-5 pyrimidine
- n-Propyl-6 chloro-2 méthoxy-4 (carboxy-2′biphényl-yl-4) méthyl-5 pyrimidine
- n-Butyl-6 chloro-2 méthoxy-4 (carboxy-2′biphényl-yl-4) méthyl-5 pyrimidine
- n-Propyl-6 chloro-2 hydroxy-4 (carboxy-2′biphényl-yl-4) méthyl-5 pyrimidine
- n-Butyl-6 chloro-2 hydroxy-4 (carboxy-2′biphényl-yl-4) méthyl-5 pyrimidine
- n-Propyl-6 diméthoxy-2,4 (carboxy-2′biphényl-yl-4) méthyl-5 pyrimidine
- n-Butyl-6 diméthoxy-2,4 (carboxy-2′biphényl-yl-4) méthyl-5 pyrimidine
- n-Propyl-6 dihydroxy-2,4 (carboxy-2′biphényl-yl-4) méthyl-5 pyrimidine
- n-Butyl-6 dihydroxy-2,4 (carboxy-2′biphényl-yl-4) méthyl-5 pyrimidine
- n-Propyl-6 diméthoxy-2,4 [(tétrazol-yl-5)-2′biphényl-yl-4] méthyl-5 pyrimidine
- n-Butyl-6 diméthoxy-2,4 [(tétrazol-yl-5)-2′biphényl-yl-4] méthyl-5 pyrimidine
- n-Propyl-6 mercapto-2 hydroxy-4 [(tétrazol-yl-5)-2′biphényl-yl-4] méthyl-5 pyrimidine
- n-Butyl-6 mercapto-2 hydroxy-4 [(tétrazol-yl-5)-2′biphényl-yl-4] méthyl-5 pyrimidine
- n-Propyl-6 méthylthio-2 hydroxy-4 [(tétrazol-yl-5)-2′biphényl-yl-4] méthyl-5 pyrimidine
- n-Butyl-6 méthylthio-2 hydroxy-4 [(tétrazol-yl-5)-2′biphényl-yl-4] méthyl-5 pyrimidine
- n-Propyl-6 méthylthio-2 méthoxy-4 [(tétrazol-yl-5)-2′biphényl-yl-4] méthyl-5 pyrimidine
- n-Butyl-6 méthylthio-2 méthoxy-4 [(tétrazol-yl-5)-2′biphényl-yl-4] méthyl-5 pyrimidine
- n-Propyl-6 méthyl-2 phényl-3 dihydro-3,4 oxo-4 [(tétrazol-yl-5)-2′biphényl-yl-4] méthyl-5 pyrimidine
- n-Butyl-6 méthyl-2 phényl-3 dihydro-3,4 oxo-4 [(tétrazol-yl-5)-2′biphényl-yl-4] méthyl-5 pyrimidine
- n-Propyl-6 méthyl-2 (chloro-2 phényl)-3 dihydro-3,4 oxo-4 [(tétrazol-yl-5)-2′biphényl-yl-4] méthyl-5 pyrimidine
- n-Butyl-6 méthyl-2 (chloro-2 phényl)-3 dihydro-3,4 oxo-4 [(tétrazol-yl-5)-2′biphényl-yl-4] méthyl-5 pyrimidine
- n-Propyl-6 méthyl-2 méthoxyméthyl-4 [(tétrazol-yl-5)-2′biphényl-yl-4] méthyl-5 pyrimidine
- n-Butyl-6 méthyl-2 méthoxyméthyl-4 [(tétrazol-yl-5)-2′biphényl-yl-4] méthyl-5 pyrimidine
- n-Propyl-6 méthyl-2 hydroxyméthyl-4 [(tétrazol-yl-5)-2′biphényl-yl-4] méthyl-5 pyrimidine

- n-Butyl-6 méthyl-2 hydroxyméthyl-4 [(tétrazol-yl-5)-2′biphényl-yl-4] méthyl-5 pyrimidine
- n-Propyl-6 hydroxyméthyl-4 [(tétrazol-yl-5)-2′biphényl-yl-4] méthyl-5 pyrimidine
- n-Butyl-6 hydroxyméthyl-4 [(tétrazol-yl-5)-2′biphényl-yl-4] méthyl-5 pyrimidine
- n-Propyl-6 méthoxyméthyl-4 [(tétrazol-yl-5)-2′biphényl-yl-4] méthyl-5 pyrimidine
- n-Butyl-6 méthoxyméthyl-4 [(tétrazol-yl-5)-2′biphényl-yl-4] méthyl-5 pyrimidine
- n-Propyl-6 méthyl-2 carboxy-4 [(tétrazol-yl-5)-2′biphényl-yl-4] méthyl-5 pyrimidine
- n-Butyl-6 méthyl-2 carboxy-4 [(tétrazol-yl-5)-2′biphényl-yl-4] méthyl-5 pyrimidine
- n-Propyl-6 carboxy-4 [(tétrazol-yl-5)-2′biphényl-yl-4] méthyl-5 pyrimidine
- n-Butyl-6 carboxy-4 [(tétrazol-yl-5)-2′biphényl-yl-4] méthyl-5 pyrimidine
- n-Propyl-6 méthyl-2 carbaldéhyde-4 [(tétrazol-yl-5)-2′biphényl-yl-4] méthyl-5 pyrimidine
- n-Butyl-6 méthyl-2 carbaldéhyde-4 [(tétrazol-yl-5)-2′biphényl-yl-4] méthyl-5 pyrimidine
- n-Propyl-6 carbaldéhyde-4 [(tétrazol-yl-5)-2′biphényl-yl-4] méthyl-5 pyrimidine
- n-Butyl-6 carbaldéhyde-4 [(tétrazol-yl-5)-2′biphényl-yl-4] méthyl-5 pyrimidine

## PHARMACOLOGIE

### I. Principe

L′affinité des produits des exemples pour les récepteurs de l′angiotensine II est évaluée par technique de déplacement d′un radioligand spécifiquement fixé sur les récepteurs surrénaliens de l′angiotensine II, chez le rat.

### II. Mode opératoire

Une aliquote d′un homogénat de surrénales de rat incube en présence d’une concentration unique de [ $^{125}I$ ] - SIAII (Sar$^1$, Tyr$^4$, Ile$^8$ - angiotensine II), antagoniste des récepteurs de l’angiotensine II, et de deux concentrations d’agents compétiteurs ($10^{-5}$ M, $10^{-7}$ M), durant 60 min à 25°C.

La réaction est achevée par ajout de tampon, puis rapide filtration à travers des filtres de papier de verre. La liaison non spécifique est déterminée en présence d’angiotensine II.

### III. Expression des résultats

Les résultats sont exprimés, pour les concentrations testées, en pourcentage de déplacement du radioligand spécifiquement fixé sur les récepteurs surrénaliens de l’angiotensine II.

IV. Résultats

| Produit de l'exemple | % de déplacement du ligand marqué | |
|---|---|---|
| | 1E-7M | 1E-5M |
| Exemple 13 | 64 | 96 |
| Exemple 14 | 38 | 81 |
| Exemple 16 | 31 | 65 |
| Exemple 24 | 62 | 93 |
| Exemple 27 | 20 | 76 |
| Exemple 31 | 51 | 71 |
| Exemple 32 | 67 | 94 |
| Exemple 33 | 62 | 69 |
| Exemple 54 | 65 | 84 |
| Exemple 55 | 60 | 86 |
| Exemple 56 | 59 | 86 |
| Exemple 57 | 69 | 80 |
| Exemple 58 | 53 | 78 |
| Exemple 59 | 57 | 89 |
| Exemple 61 | 63 | 96 |
| Exemple 75 | 0 | 72 |
| Exemple 76 | 37 | 93 |
| Exemple 77 | 4 | 63 |
| Exemple 78 | 41 | 74 |

**TOXICOLOGIE**

Les produits des exemples décrits présentent, après administration par voie orale, une excellente tolérance.

Leur dose létale 50 chez le rat a été évaluée supérieure à 300 mg/kg.

**CONCLUSION**

Les produits des exemples décrits présentent une bonne affinité pour les récepteurs à l'angiotensine II. A ce titre ils pourront être utilisés avec bénéfice dans les diverses pathologies où l'angiotensine II est impliquée, en particulier dans les traitements de l'hypertension artérielle de l'insuffisance cardiaque, à des posologies de 1 à 400 mg par voie orale et 0.01 à 50 mg par voie intraveineuse, en une ou plusieurs prises par jour.

## Revendications

1. Dérivés de pyrimidine caractérisés en ce qu'ils répondent à la formule générale (I) ou (I') :

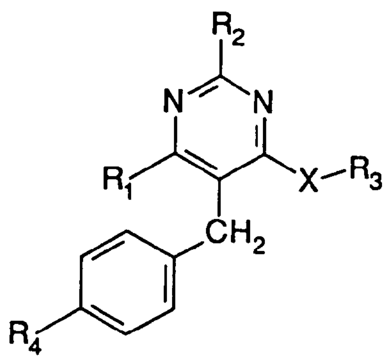

Formule (I)

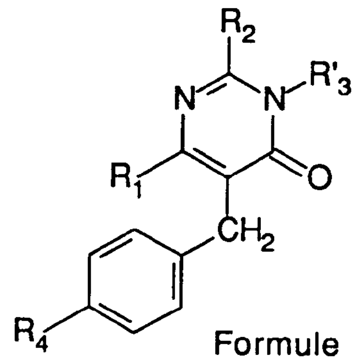

Formule (I')

Dans la formule (I),

$R_1$ est un radical alkyle inférieur de 1 à 6 atomes de carbone ou un radical alkényle inférieur de 2 à 6 atomes de carbone ;

$R_2$ est l'atome d'hydrogène, un atome d'halogène, un radical alkyle inférieur de 1 à 6 atomes de carbone, un radical halogéno alkyle inférieur de 1 à 6 atomes de carbone, un radical cyclo alkyle en $C_3$-$C_7$, un groupement OH, SH ou $NH_2$, un groupement $OR_5$, $SR_5$ ou $NHR_5$, $R_5$ étant un radical alkyle inférieur de 1 à 6 atomes de carbone, un radical halogéno alkyle inférieur de 1 à 6 atomes de carbone ou un radical cycloalkyle en $C_3$-$C_7$, NH $COR_6$, $R_5$ ayant la même signification que $R_5$ mais pouvant être également un noyau aromatique, un méthane biphényle substitué ou non ou un hétérocycle ; $R_2$ peut encore représenter un noyau aromatique ou un hétérocycle ;

X peut représenter une liaison, un atome d'oxygène, de soufre, un radical NH ou un halogène,

$R_3$ sera absent quand X est un halogène ou peut représenter : un atome d'hydrogène, un radical alkyle inférieur de 1 à 6 atomes de carbone, un radical cycloalkyle en $C_3$-$C_7$, ou encore : un groupement - $(CH_2)_n$-CN, un groupement -$(CH_2)_n$-$COOR_7$, un groupement -$(CH_2)_n$-$OR_7$,
un groupement

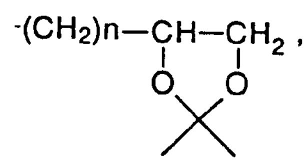

un groupement $^-(CH_2)n$-$O^-COR_7$,
un groupement

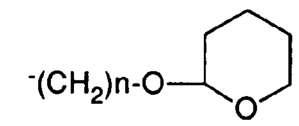 ,

un groupement $-(CH_2)_n-SR_7$, n étant un nombre entier de 0 à 5, $R_7$ étant un atome d'hydrogène ou un radical alkyle inférieur de 1 à 6 atomes de carbone ; $R_3$ peut encore représenter un groupement $-(CH_2)_p-CONR_8 R_9$, $-(CH_2)_p- NR_8R_9$, p étant un nombre entier de 0 à 5, $R_8$ et $R_9$ représentant indépendamment un atome d'hydrogène ou un radical alkyle inférieur de 1 à 6 atomes de carbone ou pouvant former ensemble avec l'atome d'azote auquel ils sont rattachés un hétérocycle choisi parmi la pyrrolidine, la pipéridine, la morpholine, la thiomorpholine, un phtalimide ou une pipérazine qui peut être substituée par un alkyle inférieur, un noyau aromatique ou un hétérocycle ; $R_3$ peut encore représenter un groupement $-(CH_2)_q-NH-(CH_2)_r-COOR_{10}$ ou $-(CH_2)_q-NH-CO-NHR_{11}$ ou $-(CH_2)_q-NH-CS-NH-R_{11}$, q et r étant des nombres entiers de 0 à 5, $R_{10}$ et $R_{11}$ représentant indépendamment un radical alkyle inférieur de 1 à 6 atomes de carbone, $R_{11}$ pouvant en outre être un noyau aromatique ou un hétérocycle ou encore un groupement $-(CH_2)_n-CO-OR_7$, n et $R_7$ étant définis comme ci-dessus ; $R_3$ peut encore représenter le groupement $SO_3H$ ou un de ses esters ou un de ses amides; $R_3$ peut encore représenter un groupement amino acide

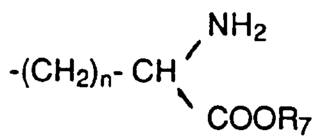

ou un de ses amides

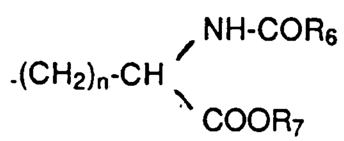 ,

n, $R_6$ et $R_7$ étant définis comme ci-dessus ; $R_3$ peut enfin représenter un radical $-(CH_2)_n-$ noyau aromatique ou $-(CH_2)_n$-hétérocycle, n étant défini comme ci-dessus ;

$R_4$ peut représenter un groupement nitro, amino, -COO $R_{12}$, $R_{12}$ étant un atome d'hydrogène ou un radical alkyle inférieur de 1 à 6 atomes de carbone ou un benzyle ; $R_4$ peut également représenter les radicaux suivants :

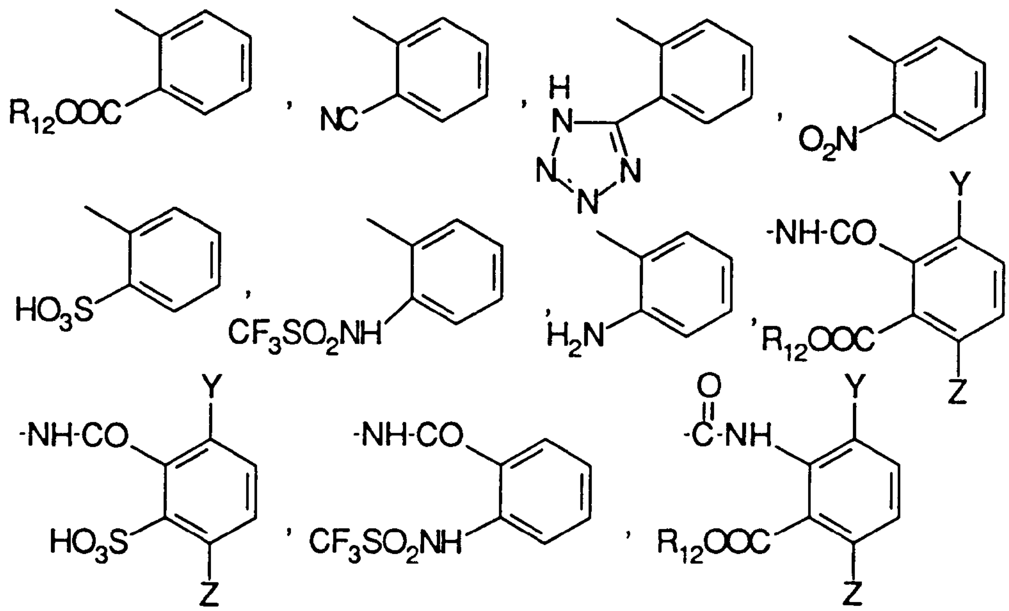

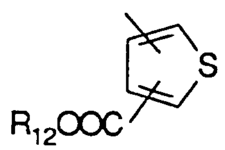
,
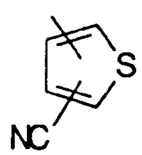
,
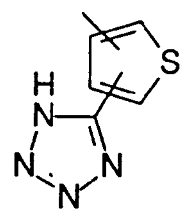

dans lesquels $R_{12}$ a la même signification que ci-dessus et Y et Z peuvent représenter indépendamment un atome d'hydrogène, un radical alkyle inférieur, un atome d'halogène, un radical alkoxy inférieur ou un radical trifluorométhyle ;

dans la formule (I'), $R_1$, $R_2$ et $R_4$ ont la même signification que dans la formule (I), $R'_3$ a la même signification que $R_3$ à la différence que contrairement à ce dernier, il ne pourra représenter un groupement $SO_3H$ ou un de ses esters ou un de ses amides et que de plus dans le cas de $R'_3$ le nombre q ne pourra être inférieur à 2, le nombre p égal à 0 et le nombre n égal à 0 sauf dans le cas d'un groupement $-(CH_2)_n-COOR_7$ ou d'un noyau aromatique ou d'un hétérocycle ;

Les dérivés précités pouvant se présenter sous la forme de sels d'addition en particulier de sels d'addition pharmaceutiquement acceptables.

2. Dérivés selon la revendication 1, caractérisés en ce que dans les formules générales (I) et (I') :

$R_1$ est un radical alkyle inférieur de 1 à 6 atomes de carbone, de préférence n-propyle ou n-butyle ;

$R_2$ est l'atome d'hydrogène, un atome d'halogène, un radical alkyle inférieur de 1 à 6 atomes de carbone, de préférence méthyle ou isopropyle ; un groupement OH, SH ou $NH_2$, un groupement $OR_5$, $SR_5$, $R_5$ étant un radical alkyle inférieur de 1 à 6 atomes de carbone, de préférence méthyle ; un noyau aromatique ;

X représente une liaison, un atome d'oxygène, un atome de soufre, un radical NH ou un halogène ;

$R_3$ sera absent quand X est un halogène, ou peut représenter : un atome d'hydrogène, un radical alkyle inférieur de 1 à 6 atomes de carbone, de préférence méthyle ou isopropyle,

un groupement $-(CH_2)_n-COOR_7$, un groupement $-(CH_2)_n-OR_7$, un groupement $-(CH_2)_n$ $O-COR_7$, un groupement $-(CH_2)_n-SR_7$, un groupement

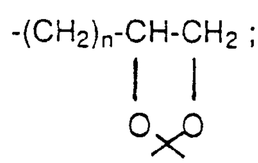

n étant un nombre entier de 0 à 5, de préférence de 0 à 3, $R_7$ étant un atome d'hydrogène ou un radical alkyle inférieur de 1 à 6 atomes de carbone, de préférence méthyle ou éthyle ;

un groupement $-(CH_2)_p-CONR_8R_9$, $-(CH_2)_p-NR_8R_9$; p étant un nombre entier de 0 à 5, de préférence de 0 à 2, $R_8$ et $R_9$, identiques ou différents, représentant un atome d'hydrogène, un radical alkyle inférieur de 1 à 6 atomes de carbone, de préférence méthyle, ou pouvant former ensemble avec l'atome d'azote auquel ils sont reliés un hétérocycle choisi parmi la pyrrolidine, la pipéridine, la morpholine, la thiomorpholine, un phtalamide ou une pipérazine qui peut être substituée par un noyau aromatique ;

un radical $-(CH_2)_n$-noyau aromatique, n étant défini comme ci-dessus ;

$R_4$ représente un groupement choisi parmi les radicaux suivants :

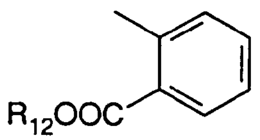 , 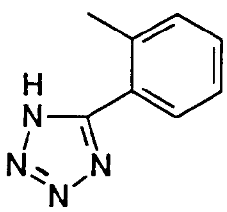 , 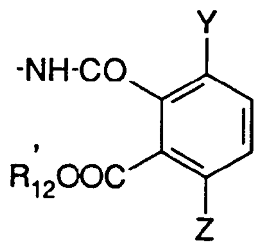 , 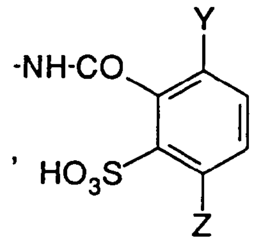 ;

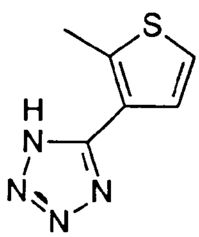

$R_{12}$ étant un atome d'hydrogène ou un radical alkyle inférieur de 1 à 6 atomes de carbone, Y et Z représentant indépendamment un atome d'hydrogène, un radical alkyle inférieur ou un atome d'halogène.

3. Dérivés de pyrimidines caractérisés en ce qu'ils répondent à la formule générale (I') :

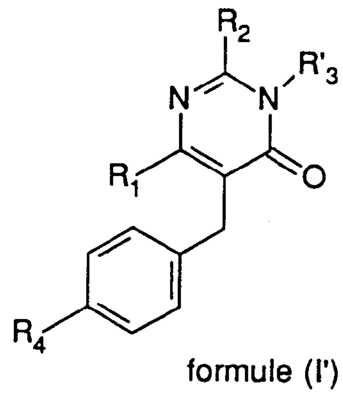

formule (I')

dans laquelle :

$R_1$ est un radical alkyle inférieur de 1 à 6 atomes de carbone ou un radical alkényle inférieur de 2 à 6 atomes de carbone ;

$R_2$ est l'atome d'hydrogène, un atome d'halogène, un radical alkyle inférieur de 1 à 6 atomes de carbone, un radical halogéno alkyle inférieur de 1 à 6 atomes de carbone, un groupement $OR_5$, $SR_8$ ou $NHR_5$, $R_8$ étant un radical alkyle inférieur de 1 à 6 atomes de carbone ou un radical cycloalkyle en $C_3$-$C_7$, $NHCOR_6$, $R_8$ ayant la même signification que $R_5$ mais pouvant être également un noyau aromatique, un méthane biphényle substitué ou non ou un hétérocycle ; $R_2$ peut encore représenter un noyau aromatique ou un hétérocycle ;

$R'_3$ peut représenter un radical alkyle inférieur de 1 à 6 atomes de carbone, un radical cycloalkyle en $C_3$-$C_7$, un groupement $(CH_2)_n-CN$, un groupement $-(CH_2)_n-COOR_7$, un groupement $-(CH_2)_n-OR_7$, un

groupement

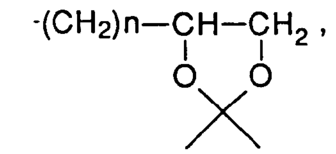

un groupement $-(CH_2)n-O^-COR_7$, un groupement

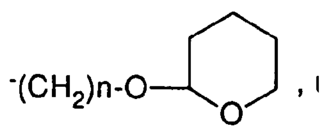

un groupement $-(CH_2)n-SR_7$, n pouvant être un nombre entier de 0 à 5, $R_7$ étant un atome d'hydrogène ou un radical alkyle inférieur de 1 à 6 atome de carbone ; $R'_3$ peut encore représenter un groupement $(CH_2)_p-CONR_8R_9$, $-(CH_2)_p\ NR_8R_9$, p étant un nombre entier de 1 à 5, $R_8$ et $R_9$ représentant indépendamment un atome d'hydrogène ou un radical alkyle inférieur de 1 à 6 atomes de carbone ou pouvant former avec l'atome d'azote auquel ils sont rattachés un hétérocycle choisi parmi la pyrrolidine, la pipéridine, la morpholine, la thiomorpholine, un phtalimide ou une pipérazine qui peut être substituée par un alkyle inférieur, un noyau aromatique ou un hétérocycle ; $R'_3$ peut encore représenter un groupement $-(CH_2)_q-NH-(CH_2)_r-COOR_{10}$ ou $-(CH_2)_q-NH\ CONH\ R_{11}$ ou $-\ (CH_2)_q-\ NH-CSNH\ R_{11}$, q étant un nombre entier de 2 à 5, r un nombre entier de 0 à 5, $R_{10}$ et $R_{11}$ représentant indépendamment un radical alkyle inférieur de 1 à 6 atomes de carbone, $R_{11}$ pouvant être en outre un noyau aromatique ou un hétérocycle ou encore un groupement $-\ (CH_2)_n-COOR_7$, n et $R_7$ étant définis comme ci-dessus ; $R'_3$ peut encore représenter un amino acide

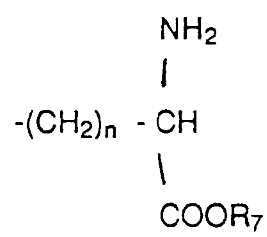

ou un de ses amides

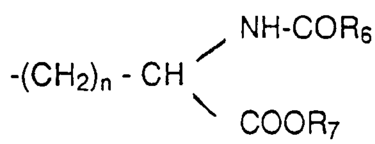

. ,

n, $R_8$ et $R_7$ étant définis comme ci-dessus ; $R'_3$ peut enfin représenter un radical $-(CH_2)_n-$ noyau aromatique ou $(CH_2)_n$-hétérocycle, n étant défini comme ci-dessus ;

$R_4$ peut représenter un groupement nitro, amino, $-COO\ R_{12}$, $R_{12}$ étant un atome d'hydrogène ou un radical alkyle inférieur de 1 à 6 atomes de carbone ou un benzyle ; $R_4$ peut également représenter les radicaux suivants :

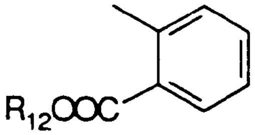 , 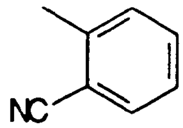 , 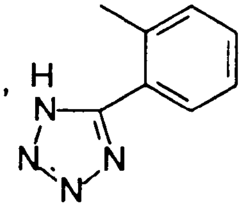 , 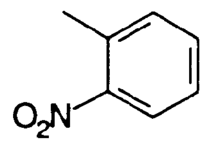

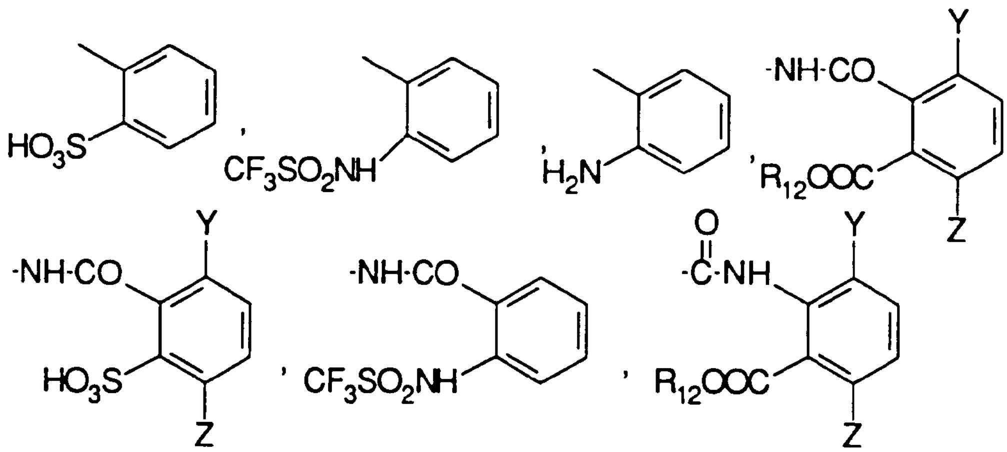

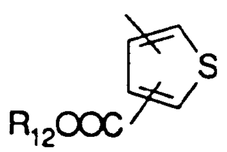 , 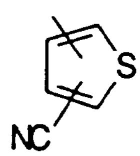 , 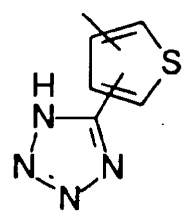

dans lesquels $R_{12}$ a la même signification que ci-dessus et Y et Z peuvent représenter indépendamment un atome d'hydrogène, un radical alkyle inférieur, un atome d'halogène, un radical alkoxy inférieur ou un radical trifluorométhyle, ainsi que leurs sels d'addition, en particulier les sels d'addition pharmaceutiquement acceptables.

**4.** Dérivés de pyrimidine selon la revendication 1 ou 2, caractérisés en ce qu'ils répondent à la formule générale (I'') :

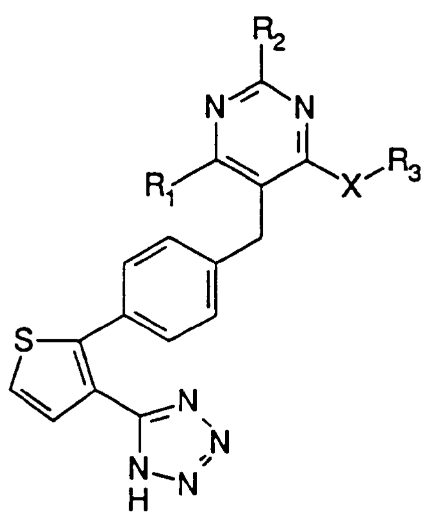

dans laquelle $R_1$, $R_2$, X et $R_3$ sont définis comme ci-dessus, ainsi que leurs sels d'addition, en particulier les sels d'addition pharmaceutiquement acceptables.

5. Dérivés de pyrimidine selon la revendication 1 ou 2, caractérisés en ce qu'ils répondent à la formule générale (I''') :

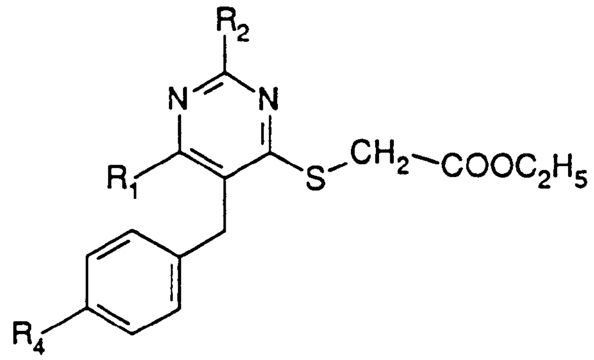

dans laquelle $R_1$, $R_2$ et $R_3$ sont définis comme ci-dessus, ainsi que leurs sels d'addition, en particulier les sels d'addition pharmaceutiquement acceptables.

6. Dérivés selon les revendications 1 à 5, caractérisés en ce que $R_1$ est un groupement choisi parmi n-propyl et n-butyl.

7. Dérivés selon les revendications 1 à 6, caractérisés en ce que $R_2$ est choisi parmi un groupement méthyl ou l'atome d'hydrogène.

8. Dérivés selon les revendications 1 à 6, caractérisés en ce que $R_2$ est un groupement méthyl thio.

9. Dérivés selon les revendications 1, 2, 3 ou 6 à 8, caractérisés en ce que $R_3$ est choisi parmi un groupement éthoxy carbonyl méthyl, un groupement hydroxy-2 éthyl, un groupement méthyl et l'atome d'hydrogène.

10. Dérivés selon les revendications 1, 2, 3, 6 à 8 caractérisés en ce que $R'_3$ est choisi parmi un groupement éthoxy carbonyl méthyl, un groupement hydroxy-2 éthyl et un groupement méthyl.

11. Dérivés selon l'une quelconque des revendications 1,2,3 ou 5 à 10 caractérisés en ce que $R_4$ est choisi parmi un groupement sulfoxy-2 benzoyl amino, un groupement carboxy-2 phényl et un groupement (tétrazol-yl-5)-2 phényl.

12. Dérivés selon l'une quelconque des revendications 1 ou 5 à 9 ou 11 caractérisés en ce qu'ils sont choisis parmi les dérivés de formule :

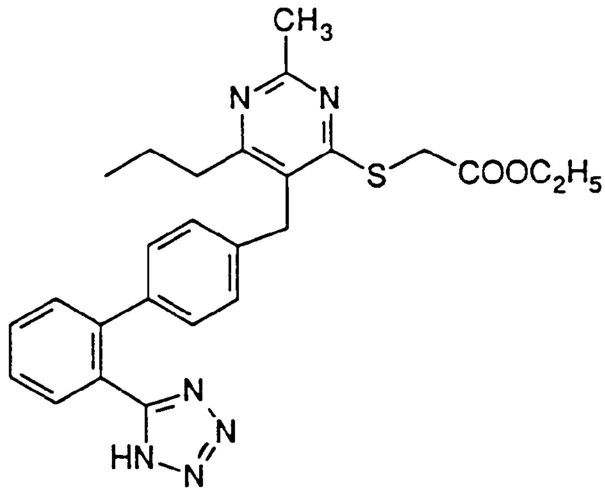

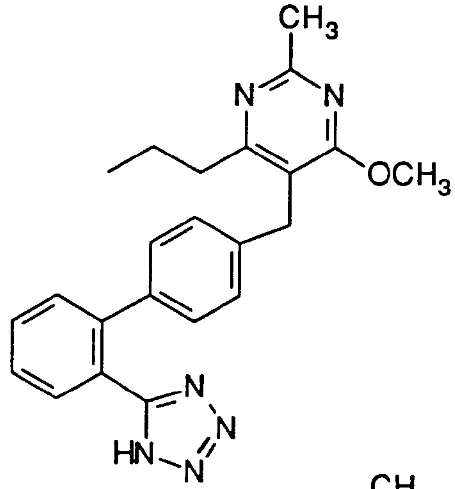

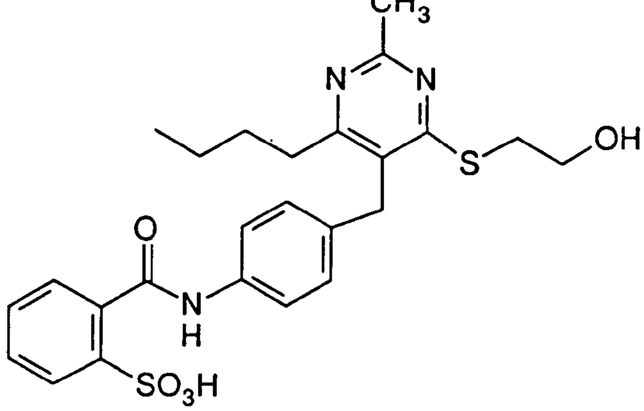

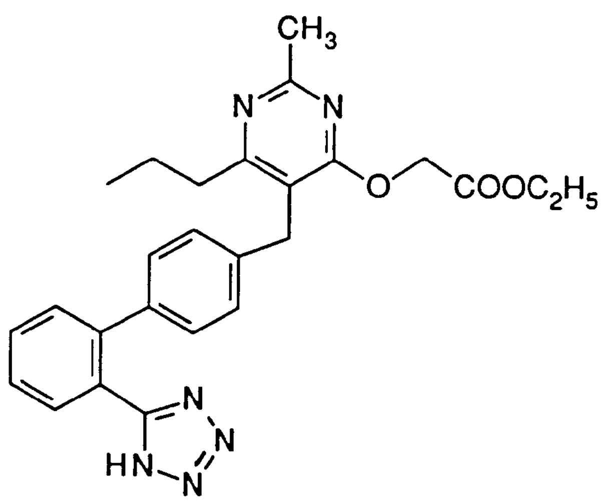

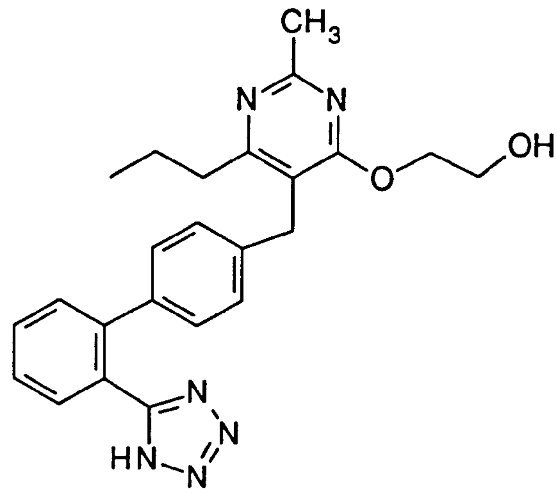

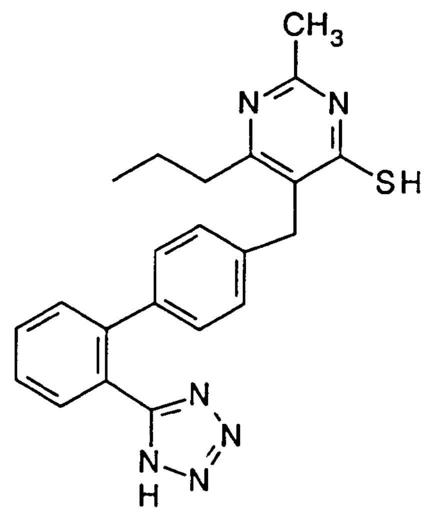

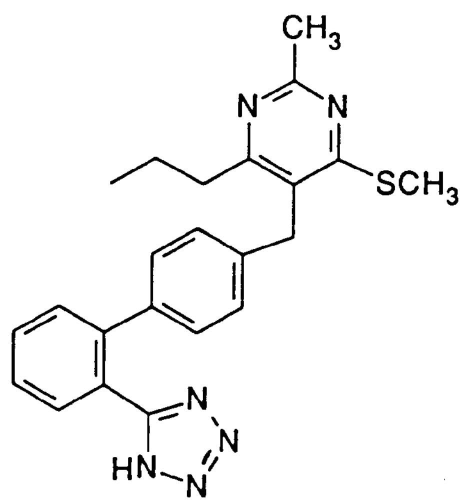

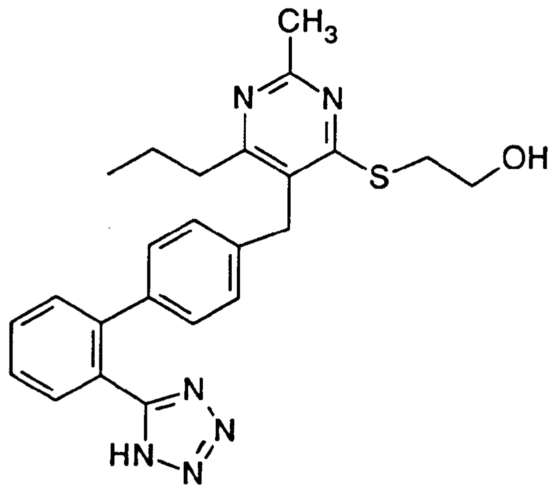

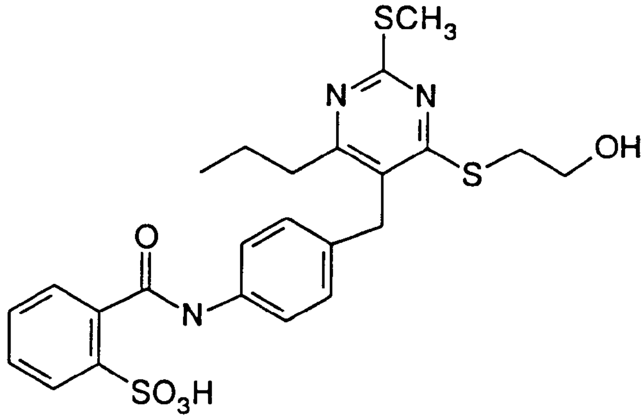

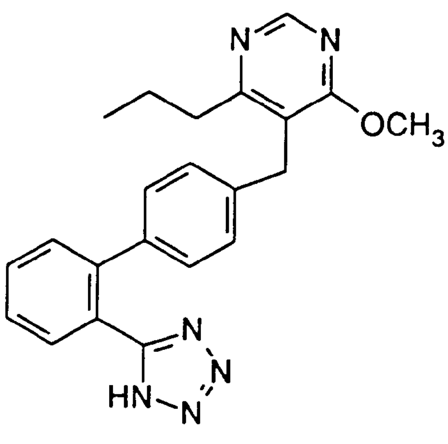

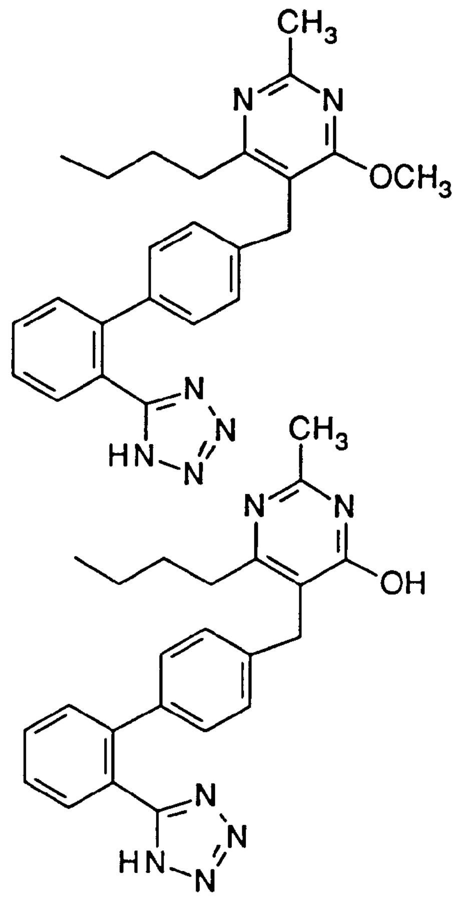

**13.** Dérivés selon l'une quelconque des revendications 1,2,3 ou 6 à 8 ou 10 et 11 caractérisés en ce qu'ils sont choisis parmi les dérivés de structures :

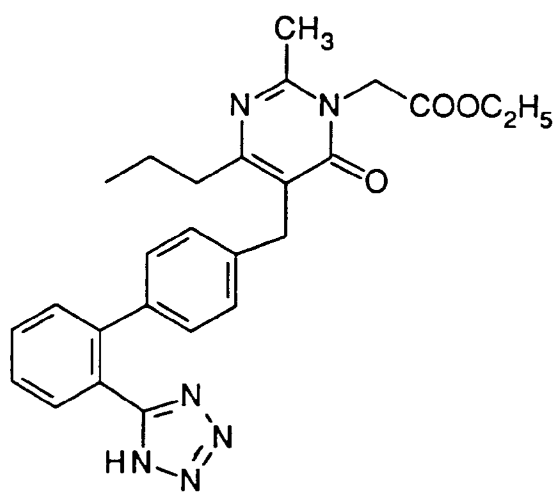

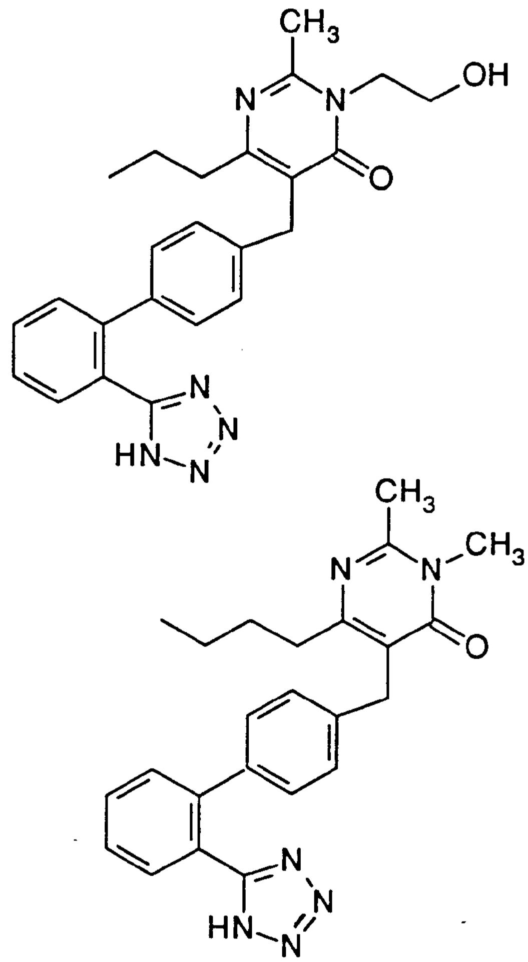

**14.** Procédés de préparation des composés de formule (I) et (I') selon l'une quelconque des revendications 1 à 13 caractérisés en ce que le cycle pyrimidine est préparé par action d'une urée, thiourée, amidine ou d'une guanidine sur des céto esters-1,3, céto nitriles-1,3 ou dicétones-1,3 de formule :

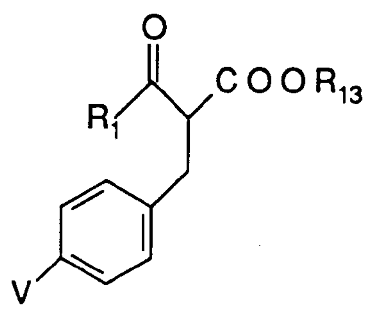

Formule (VI)

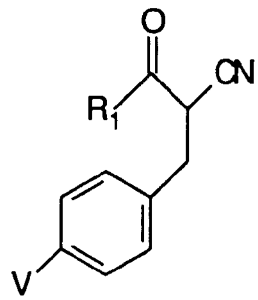

Formule (VII)

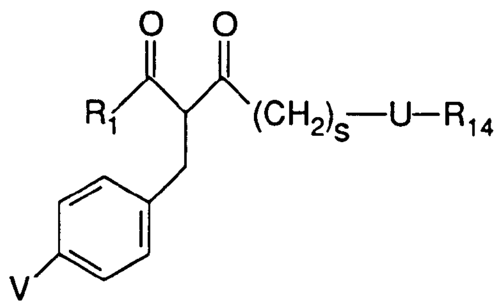

Formule (VIII)

$R_1$ étant défini comme ci-dessus, $R_{13}$ représentant un radical alkyle inférieur de préférence méthyle ou éthyle, s étant un nombre entier de 1 à 5, U pouvent être un atome d'oxygène, de soufre ou un méthylène, $R_{14}$ étant un atome d'hydrogène ou un alkyle inférieur de préférence méthyle ou éthyle, V étant un groupement fonctionnel permettant d'accéder comme il est décrit aux groupements $R_4$ définis comme ci-dessus, dans un alcool en présence d'un alcoolate de sodium ou potassium ou d'une base telle que soude ou potasse à une température pouvant aller de l'ambiante au reflux du solvant.

**15.** Procédés de préparation des composés de formule (I) ou (I′) dans laquelle $R_4$ représente un groupement

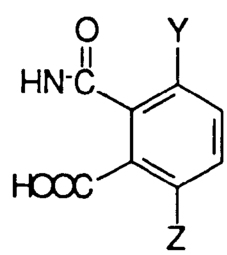

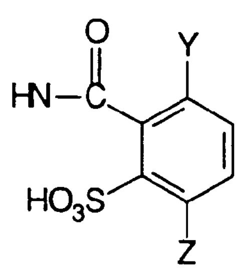

caractérisés en ce qu'on fait réagir un anhydride de formule

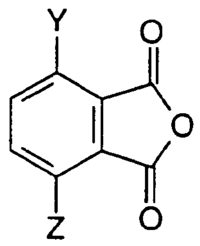

ou de formule

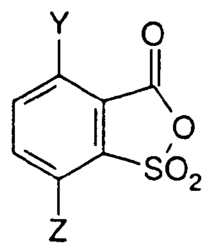

sur un dérivé aminé de formule :

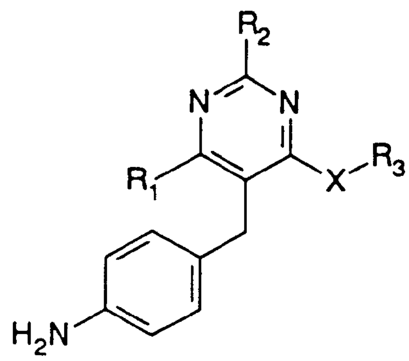

ou

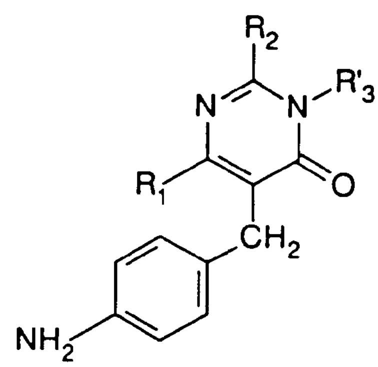

dans laquelle $R_1$, $R_2$, $R_3$ ou $R'_3$, et X sont définis comme ci-dessus et Y et Z représentent un atome d'hydrogène, un radical alkyle inférieur, un atome d'halogène ou un radical alkoxy.

**16.** Procédés de préparation des composés de formule (I) ou (I') dans laquelle $R_4$ représente un groupement

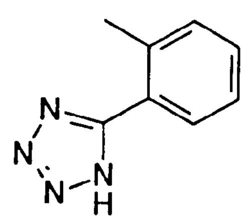

caractérisés en ce qu'on fait réagir un compsé de formule :

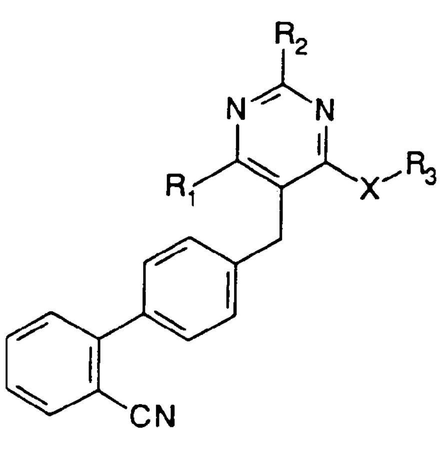

ou

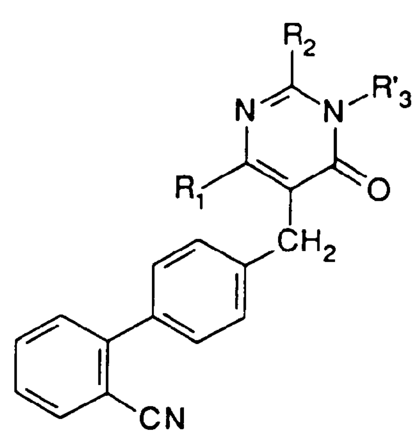

dans laquelle $R_1$, $R_2$, X et $R_3$ ou $R'_3$ sont définis comme ci-dessus avec un azoture de sodium dans le diméthyl formamide en présence d'un sel d'ammonium comme le chlorure d'ammonium à une température comprise entre 100 et 150°C ou par chauffage dans le toluène avec l'azoture de triméthyl étain suivi d'un traitement par le gaz chlorhydrique.

**17.** Composition pharmaceutique, caractérisée en ce qu'elle comprend une quantité pharmaceutiquement efficace d'au moins un composé de formule (I) ou (I') tel que défini à l'une quelconque des revendications 1 à 13, ou un de ses sels d'addition pharmaceutiquement acceptable, éventuellement incorporé dans un excipient, véhicule ou support pharmaceutiquement acceptable.

**18.** Composition pharmaceutique à activité antagoniste des récepteurs à l'angiotensine II permettant de traiter favorablement les maladies cardiovasculaires notamment l'hypertension, l'insuffisance cardiaque, caractérisée en ce qu'elle renferme une quantité pharmaceutiquement efficace d'au moins un composé de formule (I) ou (I') tel que défini dans l'une quelconque des revendications 1 à 13, ou un de ses sels d'addition pharmaceutiquement acceptable, éventuellement incorporé dans une excipient, véhicule ou support pharmaceutiquement acceptable.

**19.** Procédé de préparation d'une composition pharmaceutique, caractérisé en ce qu'on incorpore une quantité pharmaceutiquement efficace d'au moins un composé de formule (I) ou (I') telle que définie dans l'une quelconque des revendications 1 à 13, ou un de ses sels d'addition pharmaceutiquement acceptable, dans un excipient, véhicule ou support pharmaceutiquement acceptable.

**20.** Procédé selon la revendication 19, caractérisé en ce que la composition pharmaceutique est formulée sous forme de gélules, de comprimés dosés de 1 à 400 mg ou sous forme de préparations injectables dosées de 0,01 à 50 mg.

Office européen des brevets

# RAPPORT DE RECHERCHE EUROPEENNE

Numero de la demande

EP 91 40 1773

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 376 141 (BAYER)<br>* Pages 1,7 *<br>--- | 1,17 | C 07 D 239/30<br>C 07 D 239/34<br>C 07 D 239/36<br>C 07 D 239/38<br>C 07 D 403/10<br>A 61 K 31/505 |
| A | JOURNAL OF PHARMACEUTICAL SCIENCE, vol. 59, no. 11, novembre 1970, pages 1637-1645, US; C. IL HONG et al: "Potential anticancer agents VI: 5-substituted pyrimidine-6-carboxaldehydes"<br>* Page 1637; page 1642 (table VII, no. 3); page 1643 *<br>----- | 1,17 | |
| | | | **DOMAINES TECHNIQUES RECHERCHES (Int. Cl.5)** |
| | | | C 07 D 239/00<br>C 07 D 403/10 |

Le présent rapport a été établi pour toutes les revendications

| Lieu de la recherche | Date d'achèvement de la recherche | Examinateur |
|---|---|---|
| LA HAYE | 12-08-1991 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)